(19) Europäisches Patentamt
European Patent Office
Office européen des brevets

(11) EP 3 974 535 A1

(12) EUROPEAN PATENT APPLICATION

(43) Date of publication:
**30.03.2022 Bulletin 2022/13**

(21) Application number: **20198501.7**

(22) Date of filing: **25.09.2020**

(51) International Patent Classification (IPC):
**C12N 15/62** *(2006.01)* **C07K 14/705** *(2006.01)*
**C07K 14/47** *(2006.01)*

(52) Cooperative Patent Classification (CPC):
**C07K 14/70596; C07K 14/4702; C07K 14/705;**
C07K 2319/03; C07K 2319/50

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Universität Leipzig
04109 Leipzig (DE)**

(72) Inventors:
• **LANGENHAN, Tobias
04416 Markkleeberg (DE)**
• **SCHOLZ, Nicole
04179 Leipzig (DE)**

(74) Representative: **Lederer & Keller Patentanwälte
Partnerschaft mbB
Unsöldstraße 2
80538 München (DE)**

(54) **CHIMERIC SENSOR PROTEIN AND METHODS OF USE THEREOF**

(57)     The present invention provides polypeptides and nucleic acid molecules that are useful in identifying modulators of adhesion GPCRs or PC1/PC1-like proteins.

**Figure 6**

SEQUENCE LISTING

<110>   Universitaet Leipzig

<120>   Chimeric Sensor Protein and Methods of Use thereof

<130>   PEP03882UNI

<160>   63

<170>   PatentIn version 3.5

<210>   1
<211>   329
<212>   PRT
<213>   Homo sapiens

<400>   1

Tyr Thr Phe Val Leu Met Pro Ile Asn Ala Ser Asn Ala Leu Thr Leu
1               5                   10                  15

Ala His Gln Leu Arg Val Tyr Thr Ala Glu Ala Ala Ser Phe Ser Asp
                20                  25                  30

Met Met Asp Val Val Tyr Val Ala Gln Met Ile Gln Lys Phe Leu Gly
            35                  40                  45

Tyr Val Asp Gln Ile Lys Glu Leu Val Glu Val Met Val Asp Met Ala
        50                  55                  60

Ser Asn Leu Met Leu Val Asp Glu His Leu Leu Trp Leu Ala Gln Arg
65                  70                  75                  80

Glu Asp Lys Ala Cys Ser Arg Ile Val Gly Ala Leu Glu Arg Ile Gly
                85                  90                  95

Gly Ala Ala Leu Ser Pro His Ala Gln His Ile Ser Val Asn Ala Arg
                100                 105                 110

Asn Val Ala Leu Glu Ala Tyr Leu Ile Lys Pro His Ser Tyr Val Gly
        115                 120                 125

Leu Thr Cys Thr Ala Phe Gln Arg Arg Glu Gly Gly Val Pro Gly Thr
    130                 135                 140

Arg Pro Gly Ser Pro Gly Gln Asn Pro Pro Pro Glu Pro Glu Pro Pro
145                 150                 155                 160

Ala Asp Gln Gln Leu Arg Phe Arg Cys Thr Thr Gly Arg Pro Asn Val
                165                 170                 175

```
Ser Leu Ser Ser Phe His Ile Lys Asn Ser Val Ala Leu Ala Ser Ile
            180                 185                 190

Gln Leu Pro Pro Ser Leu Phe Ser Ser Leu Pro Ala Ala Leu Ala Pro
            195                 200                 205

Pro Val Pro Pro Asp Cys Thr Leu Gln Leu Leu Val Phe Arg Asn Gly
            210                 215                 220

Arg Leu Phe His Ser His Ser Asn Thr Ser Arg Pro Gly Ala Ala Gly
225                 230                 235                 240

Pro Gly Lys Arg Arg Gly Val Ala Thr Pro Val Ile Phe Ala Gly Thr
                245                 250                 255

Ser Gly Cys Gly Val Gly Asn Leu Thr Glu Pro Val Ala Val Ser Leu
            260                 265                 270

Arg His Trp Ala Glu Gly Ala Glu Pro Val Ala Ala Trp Trp Ser Gln
            275                 280                 285

Glu Gly Pro Gly Glu Ala Gly Gly Trp Thr Ser Glu Gly Cys Gln Leu
            290                 295                 300

Arg Ser Ser Gln Pro Asn Val Ser Ala Leu His Cys Gln His Leu Gly
305                 310                 315                 320

Asn Val Ala Val Leu Met Glu Leu Ser
                325
```

```
<210>   2
<211>   334
<212>   PRT
<213>   Homo sapiens

<400>   2
```

```
Ala Asn Asp Val Thr Arg Val Leu Tyr Met Phe Asn Gln Met Pro Leu
1               5                   10                  15

Asn Leu Thr Asn Ala Val Ala Thr Ala Arg Gln Leu Leu Ala Tyr Thr
            20                  25                  30

Val Glu Ala Ala Asn Phe Ser Asp Lys Met Asp Val Ile Phe Val Ala
            35                  40                  45

Glu Met Ile Glu Lys Phe Gly Arg Phe Thr Lys Glu Glu Lys Ser Lys
        50                  55                  60
```

```
Glu Leu Gly Asp Val Met Val Asp Ile Ala Ser Asn Ile Met Leu Ala
65          70              75                  80

Asp Glu Arg Val Leu Trp Leu Ala Gln Arg Glu Ala Lys Ala Cys Ser
            85              90              95

Arg Ile Val Gln Cys Leu Gln Arg Ile Ala Thr Tyr Arg Leu Ala Gly
            100             105             110

Gly Ala His Val Tyr Ser Thr Tyr Ser Pro Asn Ile Ala Leu Glu Ala
        115             120             125

Tyr Val Ile Lys Ser Thr Gly Phe Thr Gly Met Thr Cys Thr Val Phe
        130             135             140

Gln Lys Val Ala Ala Ser Asp Arg Thr Gly Leu Ser Asp Tyr Gly Arg
145             150             155             160

Arg Asp Pro Glu Gly Asn Leu Asp Lys Gln Leu Ser Phe Lys Cys Asn
            165             170             175

Val Ser Asn Thr Phe Ser Ser Leu Ala Leu Lys Asn Thr Ile Val Glu
            180             185             190

Ala Ser Ile Gln Leu Pro Pro Ser Leu Phe Ser Pro Lys Gln Lys Arg
        195             200             205

Glu Leu Arg Pro Thr Asp Asp Ser Leu Tyr Lys Leu Gln Leu Ile Ala
    210             215             220

Phe Arg Asn Gly Lys Leu Phe Pro Ala Thr Gly Asn Ser Thr Asn Leu
225             230             235             240

Ala Asp Asp Gly Lys Arg Arg Thr Val Val Thr Pro Val Ile Leu Thr
            245             250             255

Lys Ile Asp Gly Val Asn Val Asp Thr His His Ile Pro Val Asn Val
        260             265             270

Thr Leu Arg Arg Ile Ala His Gly Ala Asp Ala Val Ala Ala Arg Trp
        275             280             285

Asp Phe Asp Leu Leu Asn Gly Gln Gly Gly Trp Lys Ser Asp Gly Cys
    290             295             300

His Ile Leu Tyr Ser Asp Glu Asn Ile Thr Thr Ile Gln Cys Tyr Ser
305             310             315             320
```

22

```
Leu Ser Asn Tyr Ala Val Leu Met Asp Leu Thr Gly Ser Glu
                325             330
```

<210> 3
<211> 303
<212> PRT
<213> Homo sapiens

<400> 3

```
Ile Asp Tyr Arg Asn Ile Gln Met Met Thr Arg Glu His Leu Ala Lys
1                5                10                15

Ala Gln Arg Gly Leu Pro Gly Glu Gly Val Ser Glu Val Ile Gln Thr
                20                25                30

Leu Val Glu Ile Ser Gln Asp Gly Thr Ser Tyr Ser Gly Asp Leu Leu
                35                40                45

Ser Thr Ile Asp Val Leu Arg Asn Met Thr Glu Ile Phe Arg Arg Ala
        50                55                60

Tyr Tyr Ser Pro Thr Pro Gly Asp Val Gln Asn Phe Val Gln Ile Leu
65                70                75                80

Ser Asn Leu Leu Ala Glu Glu Asn Arg Asp Lys Trp Glu Glu Ala Gln
                85                90                95

Leu Ala Gly Pro Asn Ala Lys Glu Leu Phe Arg Leu Val Glu Asp Phe
                100               105               110

Val Asp Val Ile Gly Phe Arg Met Lys Asp Leu Arg Asp Ala Tyr Gln
        115               120               125

Val Thr Asp Asn Leu Val Leu Ser Ile His Lys Leu Pro Ala Ser Gly
        130               135               140

Ala Thr Asp Ile Ser Phe Pro Met Lys Gly Trp Arg Ala Thr Gly Asp
145               150               155               160

Trp Ala Lys Val Pro Glu Asp Arg Val Thr Val Ser Lys Ser Val Phe
                165               170               175

Ser Thr Gly Leu Thr Glu Ala Asp Glu Ala Ser Val Phe Val Val Gly
                180               185               190

Thr Val Leu Tyr Arg Asn Leu Gly Ser Phe Leu Ala Leu Gln Arg Asn
                195               200               205
```

```
Thr Thr Val Leu Asn Ser Lys Val Ile Ser Val Thr Val Lys Pro Pro
    210                 215             220

Pro Arg Ser Leu Arg Thr Pro Leu Glu Ile Glu Phe Ala His Met Tyr
    225             230             235                 240

Asn Gly Thr Thr Asn Gln Thr Cys Ile Leu Trp Asp Glu Thr Asp Val
                245             250                 255

Pro Ser Ser Ser Ala Pro Pro Gln Leu Gly Pro Trp Ser Trp Arg Gly
                260             265             270

Cys Arg Thr Val Pro Leu Asp Ala Leu Arg Thr Arg Cys Leu Cys Asp
        275             280             285

Arg Leu Ser Thr Phe Ala Ile Leu Ala Gln Leu Ser Ala Asp Ala
    290             295             300
```

```
<210>  4
<211>  335
<212>  PRT
<213>  Homo sapiens

<400>  4
```

```
His Glu Tyr Arg Tyr Leu Tyr Leu Ser Leu Arg Glu His Leu Ala Lys
1               5                   10                  15

Gly Gln Arg Met Leu Ala Gly Glu Gly Met Ser Gln Val Val Arg Ser
            20              25                  30

Leu Gln Glu Leu Leu Ala Arg Arg Thr Tyr Tyr Ser Gly Asp Leu Leu
        35              40              45

Phe Ser Val Asp Ile Leu Arg Asn Val Thr Asp Thr Phe Lys Arg Ala
    50              55              60

Thr Tyr Val Pro Ser Ala Asp Asp Val Gln Arg Phe Phe Gln Val Val
65              70              75                  80

Ser Phe Met Val Asp Ala Glu Asn Lys Glu Lys Trp Asp Asp Ala Gln
                85              90                  95

Gln Val Ser Pro Gly Ser Val His Leu Leu Arg Val Val Glu Asp Phe
            100             105                 110

Ile His Leu Val Gly Asp Ala Leu Lys Ala Phe Gln Ser Ser Leu Ile
        115             120                 125
```

```
Val Thr Asp Asn Leu Val Ile Ser Ile Gln Arg Glu Pro Val Ser Ala
    130                 135                 140

Val Ser Ser Asp Ile Thr Phe Pro Met Arg Gly Arg Arg Gly Met Lys
    145                 150                 155                 160

Asp Trp Val Arg His Ser Glu Asp Arg Leu Phe Leu Pro Lys Glu Val
                165                 170                 175

Leu Ser Leu Ser Ser Pro Gly Lys Pro Ala Thr Ser Gly Ala Ala Gly
            180                 185                 190

Ser Pro Gly Arg Gly Arg Gly Pro Gly Thr Val Pro Pro Gly Pro Gly
        195                 200                 205

His Ser His Gln Arg Leu Leu Pro Ala Asp Pro Asp Glu Ser Ser Tyr
    210                 215                 220

Phe Val Ile Gly Ala Val Leu Tyr Arg Thr Leu Gly Leu Ile Leu Pro
225                 230                 235                 240

Pro Pro Arg Pro Pro Leu Ala Val Thr Ser Arg Val Met Thr Val Thr
            245                 250                 255

Val Arg Pro Pro Thr Gln Pro Pro Ala Glu Pro Leu Ile Thr Val Glu
        260                 265                 270

Leu Ser Tyr Ile Ile Asn Gly Thr Thr Asp Pro His Cys Ala Ser Trp
        275                 280                 285

Asp Tyr Ser Arg Ala Asp Ala Ser Ser Gly Asp Trp Asp Thr Glu Asn
    290                 295                 300

Cys Gln Thr Leu Glu Thr Gln Ala Ala His Thr Arg Cys Gln Cys Gln
305                 310                 315                 320

His Leu Ser Thr Phe Ala Val Leu Ala Gln Pro Pro Lys Asp Leu
                325                 330                 335
```

```
<210>  5
<211>  299
<212>  PRT
<213>  Homo sapiens

<400>  5
```

```
Asn Glu Tyr Arg His Leu Gln His Ser Ile Lys Glu His Leu Ala Lys
1               5                   10                  15
```

25

```
Gly Gln Arg Met Leu Ala Gly Asp Gly Met Ser Gln Val Thr Lys Thr
            20                  25                  30

Leu Leu Asp Leu Thr Gln Arg Lys Asn Phe Tyr Ala Gly Asp Leu Leu
            35                  40                  45

Met Ser Val Glu Ile Leu Arg Asn Val Thr Asp Thr Phe Lys Arg Ala
            50                  55                  60

Ser Tyr Ile Pro Ala Ser Asp Gly Val Gln Asn Phe Phe Gln Ile Val
65                  70                  75                  80

Ser Asn Leu Leu Asp Glu Glu Asn Lys Glu Lys Trp Glu Asp Ala Gln
            85                  90                  95

Gln Ile Tyr Pro Gly Ser Ile Glu Leu Met Gln Val Ile Glu Asp Phe
            100                 105                 110

Ile His Ile Val Gly Met Gly Met Met Asp Phe Gln Asn Ser Tyr Leu
            115                 120                 125

Met Thr Gly Asn Val Val Ala Ser Ile Gln Lys Leu Pro Ala Ala Ser
    130                 135                 140

Val Leu Thr Asp Ile Asn Phe Pro Met Lys Gly Arg Lys Gly Met Val
145                 150                 155                 160

Asp Trp Ala Arg Asn Ser Glu Asp Arg Val Val Ile Pro Lys Ser Ile
            165                 170                 175

Phe Thr Pro Val Ser Ser Lys Glu Leu Asp Glu Ser Ser Val Phe Val
            180                 185                 190

Leu Gly Ala Val Leu Tyr Lys Asn Leu Asp Leu Ile Leu Pro Thr Leu
            195                 200                 205

Arg Asn Tyr Thr Val Ile Asn Ser Lys Ile Ile Val Val Thr Ile Arg
    210                 215                 220

Pro Glu Pro Lys Thr Thr Asp Ser Phe Leu Glu Ile Glu Leu Ala His
225                 230                 235                 240

Leu Ala Asn Gly Thr Leu Asn Pro Tyr Cys Val Leu Trp Asp Asp Ser
            245                 250                 255

Lys Thr Asn Glu Ser Leu Gly Thr Trp Ser Thr Gln Gly Cys Lys Thr
            260                 265                 270
```

26

```
Val Leu Thr Asp Ala Ser His Thr Lys Cys Leu Cys Asp Arg Leu Ser
        275                 280             285

Thr Phe Ala Ile Leu Ala Gln Gln Pro Arg Glu
    290                 295
```

```
<210>  6
<211>  352
<212>  PRT
<213>  Homo sapiens

<400>  6
```

```
Val Asp Leu Arg Ala Met Asn Glu Lys Leu Ser Arg Asn Glu Thr Gln
1                   5               10                  15

Val Asp Gly Ala Arg Ala Leu Gln Leu Val Arg Ala Leu Arg Ser Ala
            20                  25                  30

Thr Gln His Thr Gly Thr Leu Phe Gly Asn Asp Val Arg Thr Ala Tyr
            35                  40                  45

Gln Leu Leu Gly His Val Leu Gln His Glu Ser Trp Gln Gln Gly Phe
        50                  55                  60

Asp Leu Ala Ala Thr Gln Asp Ala Asp Phe His Glu Asp Val Ile His
65                  70                  75                  80

Ser Gly Ser Ala Leu Leu Ala Pro Ala Thr Arg Ala Ala Trp Glu Gln
            85                  90                  95

Ile Gln Arg Ser Glu Gly Gly Thr Ala Gln Leu Leu Arg Arg Leu Glu
            100                 105                 110

Gly Tyr Phe Ser Asn Val Ala Arg Asn Val Arg Arg Thr Tyr Leu Arg
        115                 120                 125

Pro Phe Val Ile Val Thr Ala Asn Met Ile Leu Ala Val Asp Ile Phe
    130                 135                 140

Asp Lys Phe Asn Phe Thr Gly Ala Arg Val Pro Arg Phe Asp Thr Ile
145                 150                 155                 160

His Glu Glu Phe Pro Arg Glu Leu Glu Ser Ser Val Ser Phe Pro Ala
            165                 170                 175

Asp Phe Phe Arg Pro Pro Glu Glu Lys Glu Gly Pro Leu Leu Arg Pro
        180                 185                 190
```

```
Ala Gly Arg Arg Thr Thr Pro Gln Thr Thr Arg Pro Gly Pro Gly Thr
        195                 200                 205

Glu Arg Glu Ala Pro Ile Ser Arg Arg Arg Arg His Pro Asp Asp Ala
        210                 215                 220

Gly Gln Phe Ala Val Ala Leu Val Ile Ile Tyr Arg Thr Leu Gly Gln
225                 230                 235                 240

Leu Leu Pro Glu Arg Tyr Asp Pro Asp Arg Arg Ser Leu Arg Leu Pro
                245                 250                 255

His Arg Pro Ile Ile Asn Thr Pro Met Val Ser Thr Leu Val Tyr Ser
                260                 265                 270

Glu Gly Ala Pro Leu Pro Arg Pro Leu Glu Arg Pro Val Leu Val Glu
                275                 280                 285

Phe Ala Leu Leu Glu Val Glu Glu Arg Thr Lys Pro Val Cys Val Phe
        290                 295                 300

Trp Asn His Ser Leu Ala Val Gly Gly Thr Gly Gly Trp Ser Ala Arg
305                 310                 315                 320

Gly Cys Glu Leu Leu Ser Arg Asn Arg Thr His Val Ala Cys Gln Cys
                325                 330                 335

Ser His Thr Ala Ser Phe Ala Val Leu Met Asp Ile Ser Arg Arg Glu
        340                 345                 350


<210>    7
<211>    339
<212>    PRT
<213>    Homo sapiens

<400>    7

Ser Glu Leu Lys Gly Phe Ala Glu Arg Leu Gln Arg Asn Glu Ser Gly
1                5                  10                  15

Leu Asp Ser Gly Arg Ser Gln Gln Leu Ala Leu Leu Leu Arg Asn Ala
        20                  25                  30

Thr Gln His Thr Ala Gly Tyr Phe Gly Ser Asp Val Lys Val Ala Tyr
        35                  40                  45

Gln Leu Ala Thr Arg Leu Leu Ala His Glu Ser Thr Gln Arg Gly Phe
        50                  55                  60
```

```
Gly Leu Ser Ala Thr Gln Asp Val His Phe Thr Glu Asn Leu Leu Arg
65              70              75                          80

Val Gly Ser Ala Leu Leu Asp Thr Ala Asn Lys Arg His Trp Glu Leu
                85              90                  95

Ile Gln Gln Thr Glu Gly Gly Thr Ala Trp Leu Leu Gln His Tyr Glu
                100             105             110

Ala Tyr Ala Ser Ala Leu Ala Gln Asn Met Arg His Thr Tyr Leu Ser
        115             120             125

Pro Phe Thr Ile Val Thr Pro Asn Ile Val Ile Ser Val Val Arg Leu
    130             135             140

Asp Lys Gly Asn Phe Ala Gly Ala Lys Leu Pro Arg Tyr Glu Ala Leu
145             150             155                         160

Arg Gly Glu Gln Pro Pro Asp Leu Glu Thr Thr Val Ile Leu Pro Glu
            165             170             175

Ser Val Phe Arg Glu Thr Pro Pro Val Val Arg Pro Ala Gly Pro Gly
            180             185             190

Glu Ala Gln Glu Pro Glu Glu Leu Ala Arg Arg Gln Arg Arg His Pro
        195             200             205

Glu Leu Ser Gln Gly Glu Ala Val Ala Ser Val Ile Ile Tyr Arg Thr
        210             215             220

Leu Ala Gly Leu Leu Pro His Asn Tyr Asp Pro Asp Lys Arg Ser Leu
225             230             235                         240

Arg Val Pro Lys Arg Pro Ile Ile Asn Thr Pro Val Val Ser Ile Ser
            245             250             255

Val His Asp Asp Glu Glu Leu Leu Pro Arg Ala Leu Asp Lys Pro Val
            260             265             270

Thr Val Gln Phe Arg Leu Leu Glu Thr Glu Glu Arg Thr Lys Pro Ile
        275             280             285

Cys Val Phe Trp Asn His Ser Ile Leu Val Ser Gly Thr Gly Gly Trp
    290             295             300

Ser Ala Arg Gly Cys Glu Val Val Phe Arg Asn Glu Ser His Val Ser
```

305 310 315 320

Cys Gln Cys Asn His Met Thr Ser Phe Ala Val Leu Met Asp Val Ser
325 330 335

Arg Arg Glu

<210> 8
<211> 347
<212> PRT
<213> Homo sapiens

<400> 8

Arg Glu Leu Ser Leu Leu Leu Asp Gly Leu Glu Leu Asn Lys Thr Ala
1 5 10 15

Leu Asp Thr Met Glu Ala Lys Lys Leu Ala Gln Arg Leu Arg Glu Val
20 25 30

Thr Gly His Thr Asp His Tyr Phe Ser Gln Asp Val Arg Val Thr Ala
35 40 45

Arg Leu Leu Ala His Leu Leu Ala Phe Glu Ser His Gln Gln Gly Phe
50 55 60

Gly Leu Thr Ala Thr Gln Asp Ala His Phe Asn Glu Asn Leu Leu Trp
65 70 75 80

Ala Gly Ser Ala Leu Leu Ala Pro Glu Thr Gly Asp Leu Trp Ala Ala
85 90 95

Leu Gly Gln Arg Ala Pro Gly Gly Ser Pro Gly Ser Ala Gly Leu Val
100 105 110

Arg His Leu Glu Glu Tyr Ala Ala Thr Leu Ala Arg Asn Met Glu Leu
115 120 125

Thr Tyr Leu Asn Pro Met Gly Leu Val Thr Pro Asn Ile Met Leu Ser
130 135 140

Ile Asp Arg Met Glu His Pro Ser Ser Pro Arg Gly Ala Arg Arg Tyr
145 150 155 160

Pro Arg Tyr His Ser Asn Leu Phe Arg Gly Gln Asp Ala Trp Asp Pro
165 170 175

His Thr His Val Leu Leu Pro Ser Gln Ser Pro Arg Pro Ser Pro Ser

30

```
                    180                      185                         190

        Glu Val Leu Pro Thr Ser Ser Ser Ile Glu Asn Ser Thr Thr Ser Ser
                195                 200                 205

        Val Val Pro Pro Pro Ala Pro Pro Glu Pro Glu Pro Gly Ile Ser Ile
            210                 215                 220

        Ile Ile Leu Leu Val Tyr Arg Thr Leu Gly Gly Leu Leu Pro Ala Gln
        225                 230                 235                 240

        Phe Gln Ala Glu Arg Arg Gly Ala Arg Leu Pro Gln Asn Pro Val Met
                        245                 250                 255

        Asn Ser Pro Val Val Ser Val Ala Val Phe His Gly Arg Asn Phe Leu
                    260                 265                 270

        Arg Gly Ile Leu Glu Ser Pro Ile Ser Leu Glu Phe Arg Leu Leu Gln
                275                 280                 285

        Thr Ala Asn Arg Ser Lys Ala Ile Cys Val Gln Trp Asp Pro Pro Gly
            290                 295                 300

        Leu Ala Glu Gln His Gly Val Trp Thr Ala Arg Asp Cys Glu Leu Val
        305                 310                 315                 320

        His Arg Asn Gly Ser His Ala Arg Cys Arg Cys Ser Arg Thr Gly Thr
                        325                 330                 335

        Phe Gly Val Leu Met Asp Ala Ser Pro Arg Glu
                    340                 345
```

```
        <210>   9
        <211>   299
        <212>   PRT
        <213>   Homo sapiens

        <400>   9
```

```
        Leu Pro Ser Leu Phe Met Thr Ser Thr Ala Ser Pro Val Met Pro Thr
        1               5               10                  15

        Asp Ala Tyr His Pro Ile Ile Thr Asn Leu Thr Glu Glu Arg Lys Thr
                    20                  25                  30

        Phe Gln Ser Pro Gly Val Ile Leu Ser Tyr Leu Gln Asn Val Ser Leu
                35                  40                  45

        Ser Leu Pro Ser Lys Ser Leu Ser Glu Gln Thr Ala Leu Asn Leu Thr
```

|  | 50 |  |  |  | 55 |  |  |  | 60 |  |
|---|---|---|---|---|---|---|---|---|---|---|

Lys Thr Phe Leu Lys Ala Val Gly Glu Ile Leu Leu Leu Pro Gly Trp
65                         70                75                  80

Ile Ala Leu Ser Glu Asp Ser Ala Val Val Leu Ser Leu Ile Asp Thr
              85                  90                  95

Ile Asp Thr Val Met Gly His Val Ser Ser Asn Leu His Gly Ser Thr
           100               105            110

Pro Gln Val Thr Val Glu Gly Ser Ser Ala Met Ala Glu Phe Ser Val
           115               120            125

Ala Lys Ile Leu Pro Lys Thr Val Asn Ser Ser His Tyr Arg Phe Pro
130                      135              140

Ala His Gly Gln Ser Phe Ile Gln Ile Pro His Glu Ala Phe His Arg
145                150              155            160

His Ala Trp Ser Thr Val Val Gly Leu Leu Tyr His Ser Met His Tyr
           165               170           175

Tyr Leu Asn Asn Ile Trp Pro Ala His Thr Lys Ile Ala Glu Ala Met
           180               185           190

His His Gln Asp Cys Leu Leu Phe Ala Thr Ser His Leu Ile Ser Leu
           195               200           205

Glu Val Ser Pro Pro Pro Thr Leu Ser Gln Asn Leu Ser Gly Ser Pro
           210               215           220

Leu Ile Thr Val His Leu Lys His Arg Leu Thr Arg Lys Gln His Ser
225                      230              235           240

Glu Ala Thr Asn Ser Ser Asn Arg Val Phe Val Tyr Cys Ala Phe Leu
              245              250           255

Asp Phe Ser Ser Gly Glu Gly Val Trp Ser Asn His Gly Cys Ala Leu
           260               265           270

Thr Arg Gly Asn Leu Thr Tyr Ser Val Cys Arg Cys Thr His Leu Thr
           275               280           285

Asn Phe Ala Ile Leu Met Gln Val Val Pro Leu
           290               295

<210> 10
<211> 271
<212> PRT
<213> Homo sapiens

<400> 10

```
Ile Ser Arg Val Asn Ala Leu Ala Asn Asp Ile Val Leu Leu Pro Asp
1               5                   10                  15

Pro Leu Ser Glu Val His Gly Ala Leu Ser Pro Ala Glu Ala Ser Ser
            20                  25                  30

Phe Leu Gly Leu Leu Glu His Val Leu Ala Met Glu Met Ala Pro Leu
            35                  40                  45

Gly Pro Ala Ala Leu Leu Ala Val Val Arg Phe Leu Lys Arg Val Val
        50                  55                  60

Ala Leu Gly Ala Gly Asp Pro Glu Leu Leu Leu Thr Gly Pro Trp Glu
65                  70                  75                  80

Gln Leu Ser Gln Gly Val Val Ser Val Ala Ser Leu Val Leu Glu Glu
                85                  90                  95

Gln Val Ala Asp Thr Trp Leu Ser Leu Arg Glu Val Ile Gly Gly Pro
                100                 105                 110

Met Ala Leu Val Ala Ser Val Gln Arg Leu Ala Pro Leu Leu Ser Thr
            115                 120                 125

Ser Met Thr Ser Glu Arg Pro Arg Met Arg Ile Gln His Arg His Ala
        130                 135                 140

Gly Leu Ser Gly Val Thr Val Ile His Ser Trp Phe Thr Ser Arg Val
145                 150                 155                 160

Phe Gln His Thr Leu Glu Gly Pro Asp Leu Glu Pro Gln Ala Pro Ala
                165                 170                 175

Ser Ser Glu Glu Ala Asn Arg Val Gln Arg Phe Leu Ser Thr Gln Val
                180                 185                 190

Gly Ser Ala Ile Ile Ser Ser Glu Val Trp Asp Val Thr Gly Glu Val
                195                 200                 205

Asn Val Ala Met Thr Phe His Leu Gln His Arg Ala Gln Ser Pro Leu
            210                 215                 220
```

```
Phe Pro Pro His Pro Pro Ser Pro Tyr Thr Gly Gly Ala Trp Ala Thr
225             230             235             240


Thr Gly Cys Ser Val Ala Ala Leu Tyr Leu Asp Ser Thr Ala Cys Phe
            245             250             255


Cys Asn His Ser Thr Ser Phe Ala Ile Leu Leu Gln Ile Tyr Glu
            260             265             270
```

```
<210>  11
<211>  282
<212>  PRT
<213>  Homo sapiens

<400>  11
```

```
Gln Arg Val Leu Phe Lys Cys Lys Glu Asp Val Ile Pro Asp Asn Lys
1               5               10              15


Gln Ile Gln Gln Cys Gln Glu Gly Thr Ala Val Lys Pro Ala Tyr Val
            20              25              30


Ser Phe Cys Ala Gln Ile Asn Asn Ile Phe Ser Val Leu Asp Lys Val
        35              40              45


Cys Glu Asn Lys Thr Thr Val Val Ser Leu Lys Asn Thr Thr Glu Ser
    50              55              60


Phe Val Pro Val Leu Lys Gln Ile Ser Thr Trp Thr Lys Phe Thr Lys
65              70              75              80


Glu Glu Thr Ser Ser Leu Ala Thr Val Phe Leu Glu Ser Val Glu Ser
            85              90              95


Met Thr Leu Ala Ser Phe Trp Lys Pro Ser Ala Asn Ile Thr Pro Ala
        100             105             110


Val Arg Thr Glu Tyr Leu Asp Ile Glu Ser Lys Val Ile Asn Lys Glu
        115             120             125


Cys Ser Glu Glu Asn Val Thr Leu Asp Leu Val Ala Lys Gly Asp Lys
    130             135             140


Met Lys Ile Gly Cys Ser Thr Ile Glu Glu Ser Glu Ser Thr Glu Thr
145             150             155             160


Thr Gly Val Ala Phe Val Ser Phe Val Gly Met Glu Ser Val Leu Asn
            165             170             175
```

```
Glu Arg Phe Phe Lys Asp His Gln Ala Pro Leu Thr Thr Ser Glu Ile
            180             185                 190

Lys Leu Lys Met Asn Ser Arg Val Val Gly Gly Ile Met Thr Gly Glu
            195             200                 205

Lys Lys Asp Gly Phe Ser Asp Pro Ile Ile Tyr Thr Leu Glu Asn Ile
210             215                 220

Gln Pro Lys Gln Lys Phe Glu Arg Pro Ile Cys Val Ser Trp Ser Thr
225             230                 235                 240

Asp Val Lys Gly Gly Arg Trp Thr Ser Phe Gly Cys Val Ile Leu Glu
                245                 250                 255

Ala Ser Glu Thr Tyr Thr Ile Cys Ser Cys Asn Gln Met Ala Asn Leu
            260                 265                 270

Ala Val Ile Met Ala Ser Gly Glu Leu Thr
            275                 280
```

```
<210>  12
<211>  310
<212>  PRT
<213>  Homo sapiens

<400>  12
```

```
His Gln Cys Asp Ser Ser Thr Val Cys Phe Asn Thr Val Gly Ser Tyr
1               5                   10                  15

Ser Cys Arg Cys Arg Pro Gly Trp Lys Pro Arg His Gly Ile Pro Asn
            20                  25                  30

Asn Gln Lys Asp Thr Val Cys Glu Asp Met Thr Phe Ser Thr Trp Thr
            35                  40                  45

Pro Pro Pro Gly Val His Ser Gln Thr Leu Ser Arg Phe Phe Asp Lys
        50                  55                  60

Val Gln Asp Leu Gly Arg Asp Tyr Lys Pro Gly Leu Ala Asn Asn Thr
65                  70                  75                  80

Ile Gln Ser Ile Leu Gln Ala Leu Asp Glu Leu Leu Glu Ala Pro Gly
                85                  90                  95

Asp Leu Glu Thr Leu Pro Arg Leu Gln Gln His Cys Val Ala Ser His
            100                 105                 110
```

Leu Leu Asp Gly Leu Glu Asp Val Leu Arg Gly Leu Ser Lys Asn Leu
115                 120                 125

Ser Asn Gly Leu Leu Asn Phe Ser Tyr Pro Ala Gly Thr Glu Leu Ser
130                 135                 140

Leu Glu Val Gln Lys Gln Val Asp Arg Ser Val Thr Leu Arg Gln Asn
145                 150                 155                 160

Gln Ala Val Met Gln Leu Asp Trp Asn Gln Ala Gln Lys Ser Gly Asp
                165                 170                 175

Pro Gly Pro Ser Val Val Gly Leu Val Ser Ile Pro Gly Met Gly Lys
                180                 185                 190

Leu Leu Ala Glu Ala Pro Leu Val Leu Glu Pro Glu Lys Gln Met Leu
                195                 200                 205

Leu His Glu Thr His Gln Gly Leu Leu Gln Asp Gly Ser Pro Ile Leu
210                 215                 220

Leu Ser Asp Val Ile Ser Ala Phe Leu Ser Asn Asn Asp Thr Gln Asn
225                 230                 235                 240

Leu Ser Ser Pro Val Thr Phe Thr Phe Ser His Arg Ser Val Ile Pro
                245                 250                 255

Arg Gln Lys Val Leu Cys Val Phe Trp Glu His Gly Gln Asn Gly Cys
                260                 265                 270

Gly His Trp Ala Thr Thr Gly Cys Ser Thr Ile Gly Thr Arg Asp Thr
                275                 280                 285

Ser Thr Ile Cys Arg Cys Thr His Leu Ser Ser Phe Ala Val Leu Met
290                 295                 300

Ala His Tyr Asp Val Gln
305                 310

<210> 13
<211> 236
<212> PRT
<213> Homo sapiens

<400> 13

Asn Thr Cys Gln Asp Thr Thr Ser Ser Lys Thr Thr Glu Gly Arg Lys
1               5                   10                  15

```
Glu Leu Gln Lys Ile Val Asp Lys Phe Glu Ser Leu Leu Thr Asn Gln
            20              25              30

Thr Leu Trp Arg Thr Glu Gly Arg Gln Glu Ile Ser Ser Thr Ala Thr
            35              40              45

Thr Ile Leu Arg Asp Val Glu Ser Lys Val Leu Glu Thr Ala Leu Lys
            50              55              60

Asp Pro Glu Gln Lys Val Leu Lys Ile Gln Asn Asp Ser Val Ala Ile
65              70              75              80

Glu Thr Gln Ala Ile Thr Asp Asn Cys Ser Glu Glu Arg Lys Thr Phe
            85              90              95

Asn Leu Asn Val Gln Met Asn Ser Met Asp Ile Arg Cys Ser Asp Ile
            100             105             110

Ile Gln Gly Asp Thr Gln Gly Pro Ser Ala Ile Ala Phe Ile Ser Tyr
            115             120             125

Ser Ser Leu Gly Asn Ile Ile Asn Ala Thr Phe Phe Glu Glu Met Asp
    130             135             140

Lys Lys Asp Gln Val Tyr Leu Asn Ser Gln Val Val Ser Ala Ala Ile
145             150             155             160

Gly Pro Lys Arg Asn Val Ser Leu Ser Lys Ser Val Thr Leu Thr Phe
            165             170             175

Gln His Val Lys Met Thr Pro Ser Thr Lys Lys Val Phe Cys Val Tyr
            180             185             190

Trp Lys Ser Thr Gly Gln Gly Ser Gln Trp Ser Arg Asp Gly Cys Phe
    195             200             205

Leu Ile His Val Asn Lys Ser His Thr Met Cys Asn Cys Ser His Leu
    210             215             220

Ser Ser Phe Ala Val Leu Met Ala Leu Thr Ser Gln
225             230             235
```

```
<210>   14
<211>   186
<212>   PRT
<213>   Homo sapiens

<400>   14
```

```
Met Gly Ser Arg Phe Leu Leu Val Leu Leu Ser Gly Ala Ser Cys Pro
1               5                   10                  15

Pro Cys Pro Lys Tyr Ala Ser Cys His Asn Ser Thr His Cys Thr Cys
            20                  25                  30

Glu Asp Gly Phe Arg Ala Arg Ser Gly Arg Thr Tyr Phe His Asp Ser
            35                  40                  45

Ser Glu Lys Cys Glu Asp Ile Asn Glu Cys Glu Thr Gly Leu Ala Lys
        50                  55                  60

Cys Lys Tyr Lys Ala Tyr Cys Arg Asn Lys Val Gly Gly Tyr Ile Cys
65                  70                  75                  80

Ser Cys Leu Val Lys Tyr Thr Leu Phe Asn Phe Leu Ala Gly Ile Ile
                85                  90                  95

Asp Tyr Asp His Pro Asp Cys Tyr Glu Asn Asn Ser Gln Gly Thr Thr
            100                 105                 110

Gln Ser Asn Val Asp Ile Trp Val Ser Gly Val Lys Pro Gly Phe Gly
            115                 120                 125

Lys Gln Leu Pro Gly Asp Lys Arg Thr Lys His Ile Cys Val Tyr Trp
        130                 135                 140

Glu Gly Ser Glu Gly Gly Trp Ser Thr Glu Gly Cys Ser His Val His
145                 150                 155                 160

Ser Asn Gly Ser Tyr Thr Lys Cys Lys Cys Phe His Leu Ser Ser Phe
                165                 170                 175

Ala Val Leu Val Ala Leu Ala Pro Lys Glu
                180                 185
```

```
<210>  15
<211>  327
<212>  PRT
<213>  Homo sapiens

<400>  15
```

```
Ser Ser Gly Gln His Gln Cys Asp Ser Ser Thr Val Cys Phe Asn Thr
1               5                   10                  15

Val Gly Ser Tyr Ser Cys Arg Cys Arg Pro Gly Trp Lys Pro Arg His
            20                  25                  30
```

Gly Ile Pro Asn Asn Gln Lys Asp Thr Val Cys Glu Asp Met Thr Phe
   35       40       45

Ser Thr Trp Thr Pro Pro Pro Gly Val His Ser Gln Thr Leu Ser Arg
   50       55       60

Phe Phe Asp Lys Val Gln Asp Leu Gly Arg Asp Ser Lys Thr Ser Ser
65       70       75       80

Ala Glu Val Thr Ile Gln Asn Val Ile Lys Leu Val Asp Glu Leu Met
      85       90       95

Glu Ala Pro Gly Asp Val Glu Ala Leu Ala Pro Pro Val Arg His Leu
      100      105      110

Ile Ala Thr Gln Leu Leu Ser Asn Leu Glu Asp Ile Met Arg Ile Leu
      115      120      125

Ala Lys Ser Leu Pro Lys Gly Pro Phe Thr Tyr Ile Ser Pro Ser Asn
   130      135      140

Thr Glu Leu Thr Leu Met Ile Gln Glu Arg Gly Asp Lys Asn Val Thr
145      150      155      160

Met Gly Gln Ser Ser Ala Arg Met Lys Leu Asn Trp Ala Val Ala Ala
      165      170      175

Gly Ala Glu Asp Pro Gly Pro Ala Val Ala Gly Ile Leu Ser Ile Gln
      180      185      190

Asn Met Thr Thr Leu Leu Ala Asn Ala Ser Leu Asn Leu His Ser Lys
      195      200      205

Lys Gln Ala Glu Leu Glu Glu Ile Tyr Glu Ser Ser Ile Arg Gly Val
   210      215      220

Gln Leu Arg Arg Leu Ser Ala Val Asn Ser Ile Phe Leu Ser His Asn
225      230      235      240

Asn Thr Lys Glu Leu Asn Ser Pro Ile Leu Phe Ala Phe Ser His Leu
      245      250      255

Glu Ser Ser Asp Gly Glu Ala Gly Arg Asp Pro Pro Ala Lys Asp Val
      260      265      270

Met Pro Gly Pro Arg Gln Glu Leu Leu Cys Ala Phe Trp Lys Ser Asp
   275      280      285

```
Ser Asp Arg Gly Gly His Trp Ala Thr Glu Gly Cys Gln Val Leu Gly
    290             295             300

Ser Lys Asn Gly Ser Thr Thr Cys Gln Cys Ser His Leu Ser Ser Phe
    305             310             315             320

Ala Ile Leu Met Ala His Tyr
                325


<210>  16
<211>  274
<212>  PRT
<213>  Homo sapiens

<400>  16

Ser Leu Leu Glu Glu Leu Asn Lys Asn Phe Ser Met Ile Val Gly Asn
1               5               10              15

Ala Thr Glu Ala Ala Val Ser Ser Phe Val Gln Asn Leu Ser Val Ile
            20              25              30

Ile Arg Gln Asn Pro Ser Thr Thr Val Gly Asn Leu Ala Ser Val Val
        35              40              45

Ser Ile Leu Ser Asn Ile Ser Ser Leu Ser Leu Ala Ser His Phe Arg
    50              55              60

Val Ser Asn Ser Thr Met Glu Asp Val Ile Ser Ile Ala Asp Asn Ile
65              70              75              80

Leu Asn Ser Ala Ser Val Thr Asn Trp Thr Val Leu Leu Arg Glu Glu
            85              90              95

Lys Tyr Ala Ser Ser Arg Leu Leu Glu Thr Leu Glu Asn Ile Ser Thr
            100             105             110

Leu Val Pro Pro Thr Ala Leu Pro Leu Asn Phe Ser Arg Lys Phe Ile
        115             120             125

Asp Trp Lys Gly Ile Pro Val Asn Lys Ser Gln Leu Lys Arg Gly Tyr
    130             135             140

Ser Tyr Gln Ile Lys Met Cys Pro Gln Asn Thr Ser Ile Pro Ile Arg
145             150             155             160

Gly Arg Val Leu Ile Gly Ser Asp Gln Phe Gln Arg Ser Leu Pro Glu
                165             170             175
```

40

Thr Ile Ile Ser Met Ala Ser Leu Thr Leu Gly Asn Ile Leu Pro Val
            180                 185                 190

Ser Lys Asn Gly Asn Ala Gln Val Asn Gly Pro Val Ile Ser Thr Val
            195                 200                 205

Ile Gln Asn Tyr Ser Ile Asn Glu Val Phe Leu Phe Phe Ser Lys Ile
            210                 215                 220

Glu Ser Asn Leu Ser Gln Pro His Cys Val Phe Trp Asp Phe Ser His
225                 230                 235                 240

Leu Gln Trp Asn Asp Ala Gly Cys His Leu Val Asn Glu Thr Gln Asp
                245                 250                 255

Ile Val Thr Cys Gln Cys Thr His Leu Thr Ser Phe Ser Ile Leu Met
            260                 265                 270

Ser Pro


<210> 17
<211> 293
<212> PRT
<213> Homo sapiens

<400> 17

Leu Asn Ala Leu Asn Ile Phe Glu Glu Asp Ser Arg Leu Val Gln Pro
1                   5                   10                  15

Phe Glu Asp Asn Ile Lys Ile Ser Val Tyr Thr Gly Lys Ser Glu Thr
            20                  25                  30

Ile Thr Asp Met Leu Leu Gln Lys Cys Pro Thr Asp Leu Ser Cys Val
            35                  40                  45

Ile Arg Asn Ile Gln Gln Ser Pro Trp Ile Pro Gly Asn Ile Ala Val
            50                  55                  60

Ile Val Gln Leu Leu His Asn Ile Ser Thr Ala Ile Trp Thr Gly Val
65                  70                  75                  80

Asp Glu Ala Lys Met Gln Ser Tyr Ser Thr Ile Ala Asn His Ile Leu
                85                  90                  95

Asn Ser Lys Ser Ile Ser Asn Trp Thr Phe Ile Pro Asp Arg Asn Ser
            100                 105                 110

41

```
Ser Tyr Ile Leu Leu His Ser Val Asn Ser Phe Ala Arg Arg Leu Phe
        115                 120                 125

Ile Asp Lys His Pro Val Asp Ile Ser Asp Val Phe Ile His Thr Met
        130                 135                 140

Gly Thr Thr Ile Ser Gly Asp Asn Ile Gly Lys Asn Phe Thr Phe Ser
145                 150                 155                 160

Met Arg Ile Asn Asp Thr Ser Asn Glu Val Thr Gly Arg Val Leu Ile
                165                 170                 175

Ser Arg Asp Glu Leu Arg Lys Val Pro Ser Pro Ser Gln Val Ile Ser
                180                 185                 190

Ile Ala Phe Pro Thr Ile Gly Ala Ile Leu Glu Ala Ser Leu Leu Glu
            195                 200                 205

Asn Val Thr Val Asn Gly Leu Val Leu Ser Ala Ile Leu Pro Lys Glu
        210                 215                 220

Leu Lys Arg Ile Ser Leu Ile Phe Glu Lys Ile Ser Lys Ser Glu Glu
225                 230                 235                 240

Arg Arg Thr Gln Cys Val Gly Trp His Ser Val Glu Asn Arg Trp Asp
                245                 250                 255

Gln Gln Ala Cys Lys Met Ile Gln Glu Asn Ser Gln Gln Ala Val Cys
            260                 265                 270

Lys Cys Arg Pro Ser Lys Leu Phe Thr Ser Phe Ser Ile Leu Met Ser
            275                 280                 285

Pro His Ile Leu Glu
        290


<210>  18
<211>  285
<212>  PRT
<213>  Homo sapiens

<400>  18

Ala Arg Leu Leu Ala Leu Phe Thr Arg Thr Lys Leu Leu Gln Ala Gly
1               5                   10                  15

Gln Gly Ser Pro Ala Glu Glu Val Pro Gln Ile Leu Ala Gln Leu Pro
        20                  25                  30
```

Gly Gln Ala Ala Glu Ala Ser Ser Pro Ser Asp Leu Leu Thr Leu Leu
35                      40              45

Ser Thr Met Lys Tyr Val Ala Lys Val Val Ala Glu Ala Arg Ile Gln
50                      55              60

Leu Asp Arg Arg Ala Leu Lys Asn Leu Leu Ile Ala Thr Asp Lys Val
65              70              75              80

Leu Asp Met Asp Thr Arg Ser Leu Trp Thr Leu Ala Gln Ala Arg Lys
85                      90              95

Pro Trp Ala Gly Ser Thr Leu Leu Leu Ala Val Glu Thr Leu Ala Cys
100             105             110

Ser Leu Cys Pro Gln Asp His Pro Phe Ala Phe Ser Leu Pro Asn Val
115             120             125

Leu Leu Gln Ser Gln Leu Phe Gly Pro Thr Phe Pro Ala Asp Tyr Ser
130             135             140

Ile Ser Phe Pro Thr Arg Pro Pro Leu Gln Ala Gln Ile Pro Arg His
145             150             155             160

Ser Leu Ala Pro Leu Val Arg Asn Gly Thr Glu Ile Ser Ile Thr Ser
165             170             175

Leu Val Leu Arg Lys Leu Asp His Leu Leu Pro Ser Asn Tyr Gly Gln
180             185             190

Gly Leu Gly Asp Ser Leu Tyr Ala Thr Pro Gly Leu Val Leu Val Ile
195             200             205

Ser Ile Met Ala Gly Asp Arg Ala Phe Ser Gln Gly Glu Val Ile Met
210             215             220

Asp Phe Gly Asn Thr Asp Gly Ser Pro His Cys Val Phe Trp Asp His
225             230             235             240

Ser Leu Phe Gln Gly Arg Gly Gly Trp Ser Lys Glu Gly Cys Gln Ala
245             250             255

Gln Val Ala Ser Ala Ser Pro Thr Ala Gln Cys Leu Cys Gln His Leu
260             265             270

Thr Ala Phe Ser Val Leu Met Ser Pro His Thr Val Pro
275             280             285

43

```
<210>   19
<211>   305
<212>   PRT
<213>   Homo sapiens

<400>   19

Leu Ser Val Glu Lys Leu Phe Lys Asp Ser Thr Gly Ala Ser Arg Leu
1               5                   10                  15


Ser Val Ala Ala Pro Ser Ile Pro Leu His Ile Leu Asp Phe Arg Ala
            20                  25                  30


Pro Glu Thr Ile Glu Ser Val Ala Gln Gly Ile Arg Lys Asn Cys Pro
        35                  40                  45


Phe Asp Tyr Ala Cys Ile Thr Asp Met Val Lys Ser Ser Glu Thr Thr
    50                  55                  60


Ser Gly Asn Ile Ala Phe Ile Val Glu Leu Leu Lys Asn Ile Ser Thr
65                  70                  75                  80


Asp Leu Ser Asp Asn Val Thr Arg Glu Lys Met Lys Ser Tyr Ser Glu
                85                  90                  95


Val Ala Asn His Ile Leu Asp Thr Ala Ala Ile Ser Asn Trp Ala Phe
            100                 105                 110


Ile Pro Asn Lys Asn Ala Ser Ser Asp Leu Leu Gln Ser Val Asn Leu
        115                 120                 125


Phe Ala Arg Gln Leu His Ile His Asn Asn Ser Glu Asn Ile Val Asn
    130                 135                 140


Glu Leu Phe Ile Gln Thr Lys Gly Phe His Ile Asn His Asn Thr Ser
145                 150                 155                 160


Glu Lys Ser Leu Asn Phe Ser Met Ser Met Asn Asn Thr Thr Glu Asp
                165                 170                 175


Ile Leu Gly Met Val Gln Ile Pro Arg Gln Glu Leu Arg Lys Leu Trp
            180                 185                 190


Pro Asn Ala Ser Gln Ala Ile Ser Ile Ala Phe Pro Thr Leu Gly Ala
        195                 200                 205


Ile Leu Arg Glu Ala His Leu Gln Asn Val Ser Leu Pro Arg Gln Val
        210                 215                 220
```

```
Asn Gly Leu Val Leu Ser Val Val Leu Pro Glu Arg Leu Gln Glu Ile
225             230             235                 240

Ile Leu Thr Phe Glu Lys Ile Asn Lys Thr Arg Asn Ala Arg Ala Gln
                245             250                 255

Cys Val Gly Trp His Ser Lys Lys Arg Arg Trp Asp Glu Lys Ala Cys
                260             265                 270

Gln Met Met Leu Asp Ile Arg Asn Glu Val Lys Cys Arg Cys Asn Tyr
            275             280                 285

Thr Ser Val Val Met Ser Phe Ser Ile Leu Met Ser Ser Lys Ser Met
            290             295                 300

Thr
305


<210>  20
<211>  277
<212>  PRT
<213>  Homo sapiens

<400>  20

Ala Pro Ile Asn Ser Leu Leu Gln Met Ala Lys Ala Leu Ile Lys Ser
1               5                 10                  15

Pro Ser Gln Asp Glu Met Leu Pro Thr Tyr Leu Lys Asp Leu Ser Ile
            20              25                  30

Ser Ile Asp Lys Ala Glu His Glu Ile Ser Ser Ser Pro Gly Ser Leu
            35              40                  45

Gly Ala Ile Ile Asn Ile Leu Asp Leu Leu Ser Thr Val Pro Thr Gln
        50              55                  60

Val Asn Ser Glu Met Met Thr His Val Leu Ser Thr Val Asn Val Ile
65              70                  75                  80

Leu Gly Lys Pro Val Leu Asn Thr Trp Lys Val Leu Gln Gln Gln Trp
                85              90                  95

Thr Asn Gln Ser Ser Gln Leu Leu His Ser Val Glu Arg Phe Ser Gln
            100             105                 110

Ala Leu Gln Ser Gly Asp Ser Pro Pro Leu Ser Phe Ser Gln Thr Asn
            115             120                 125
```

```
Val Gln Met Ser Ser Met Val Ile Lys Ser Ser His Pro Glu Thr Tyr
    130                 135                 140

Gln Gln Arg Phe Val Phe Pro Tyr Phe Asp Leu Trp Gly Asn Val Val
    145                 150                 155                 160

Ile Asp Lys Ser Tyr Leu Glu Asn Leu Gln Ser Asp Ser Ser Ile Val
                165                 170                 175

Thr Met Ala Phe Pro Thr Leu Gln Ala Ile Leu Ala Gln Asp Ile Gln
                180                 185                 190

Glu Asn Asn Phe Ala Glu Ser Leu Val Met Thr Thr Thr Val Ser His
        195                 200                 205

Asn Thr Thr Met Pro Phe Arg Ile Ser Met Thr Phe Lys Asn Asn Ser
    210                 215                 220

Pro Ser Gly Gly Glu Thr Lys Cys Val Phe Trp Asn Phe Arg Leu Ala
225                 230                 235                 240

Asn Asn Thr Gly Gly Trp Asp Ser Ser Gly Cys Tyr Val Glu Glu Gly
                245                 250                 255

Asp Gly Asp Asn Val Thr Cys Ile Cys Asp His Leu Thr Ser Phe Ser
            260                 265                 270

Ile Leu Met Ser Pro
        275
```

```
<210>   21
<211>   266
<212>   PRT
<213>   Homo sapiens

<400>   21
```

```
Ala Gln Gly Pro Pro Leu Leu Ala Thr Ser Val Thr Ser Trp Trp Ser
1               5                   10                  15

Pro Gln Asn Ile Ser Leu Pro Ser Ala Ala Ser Phe Thr Phe Ser Phe
            20                  25                  30

His Ser Pro Pro His Thr Ala Ala His Asn Ala Ser Val Asp Met Cys
        35                  40                  45

Glu Leu Lys Arg Asp Leu Gln Leu Leu Ser Gln Phe Leu Lys His Pro
        50                  55                  60
```

```
Gln Lys Ala Ser Arg Arg Pro Ser Ala Ala Pro Ala Ser Gln Gln Leu
65              70              75              80

Gln Ser Leu Glu Ser Lys Leu Thr Ser Val Arg Phe Met Gly Asp Met
            85              90              95

Val Ser Phe Glu Glu Asp Arg Ile Asn Ala Thr Val Trp Lys Leu Gln
        100             105             110

Pro Thr Ala Gly Leu Gln Asp Leu His Ile His Ser Arg Gln Glu Glu
        115             120             125

Glu Gln Ser Glu Ile Met Glu Tyr Ser Val Leu Leu Pro Arg Thr Leu
    130             135             140

Phe Gln Arg Thr Lys Gly Arg Ser Gly Glu Ala Glu Lys Arg Leu Leu
145             150             155             160

Leu Val Asp Phe Ser Ser Gln Ala Leu Phe Gln Asp Lys Asn Ser Ser
            165             170             175

Gln Val Leu Gly Glu Lys Val Leu Gly Ile Val Val Gln Asn Thr Lys
        180             185             190

Val Ala Asn Leu Thr Glu Pro Val Val Leu Thr Phe Gln His Gln Leu
        195             200             205

Gln Pro Lys Asn Val Thr Leu Gln Cys Val Phe Trp Val Glu Asp Pro
    210             215             220

Thr Leu Ser Ser Pro Gly His Trp Ser Ser Ala Gly Cys Glu Thr Val
225             230             235             240

Arg Arg Glu Thr Gln Thr Ser Cys Phe Cys Asn His Leu Thr Tyr Phe
            245             250             255

Ala Val Leu Met Val Ser Ser Val Glu Val
            260             265
```

```
<210>  22
<211>  280
<212>  PRT
<213>  Homo sapiens

<400>  22
```

```
Pro Val Lys Ala Ser Phe Ser Ser Pro Thr Val Ser Ala Pro Ala Asn
1               5               10              15
```

```
Val Asn Thr Thr Ser Ala Pro Pro Val Gln Thr Asp Ile Val Asn Thr
            20                  25              30

Ser Ser Ile Ser Asp Leu Glu Asn Gln Val Leu Gln Met Glu Lys Ala
            35                  40                  45

Leu Ser Leu Gly Ser Leu Glu Pro Asn Leu Ala Gly Glu Met Ile Asn
            50                  55                  60

Gln Val Ser Arg Leu Leu His Ser Pro Pro Asp Met Leu Ala Pro Leu
65                  70                  75                  80

Ala Gln Arg Leu Leu Lys Val Val Asp Asp Ile Gly Leu Gln Leu Asn
                85                  90                  95

Phe Ser Asn Thr Thr Ile Ser Leu Thr Ser Pro Ser Leu Ala Leu Ala
            100                 105                 110

Val Ile Arg Val Asn Ala Ser Ser Phe Asn Thr Thr Thr Phe Val Ala
            115                 120                 125

Gln Asp Pro Ala Asn Leu Gln Val Ser Leu Glu Thr Gln Ala Pro Glu
    130                 135                 140

Asn Ser Ile Gly Thr Ile Thr Leu Pro Ser Ser Leu Met Asn Asn Leu
145                 150                 155                 160

Pro Ala His Asp Met Glu Leu Ala Ser Arg Val Gln Phe Asn Phe Phe
            165                 170                 175

Glu Thr Pro Ala Leu Phe Gln Asp Pro Ser Leu Glu Asn Leu Ser Leu
            180                 185                 190

Ile Ser Tyr Val Ile Ser Ser Ser Val Ala Asn Leu Thr Val Arg Asn
            195                 200                 205

Leu Thr Arg Asn Val Thr Val Thr Leu Lys His Ile Asn Pro Ser Gln
    210                 215                 220

Asp Glu Leu Thr Val Arg Cys Val Phe Trp Asp Leu Gly Arg Asn Gly
225                 230                 235                 240

Gly Arg Gly Gly Trp Ser Asp Asn Gly Cys Ser Val Lys Asp Arg Arg
            245                 250                 255

Leu Asn Glu Thr Ile Cys Thr Cys Ser His Leu Thr Ser Phe Gly Val
```

<pre>
              260                 265                 270


Leu Leu Asp Leu Ser Arg Thr Ser
          275                 280


<210>  23
<211>  258
<212>  PRT
<213>  Homo sapiens

<400>  23

Met Ala Thr Pro Arg Gly Leu Gly Ala Leu Leu Leu Leu Leu Leu
1                   5                   10                  15


Pro Thr Ser Gly Gln Glu Lys Pro Thr Glu Gly Pro Arg Asn Thr Cys
          20                  25                  30


Leu Gly Ser Asn Asn Met Tyr Asp Ile Phe Asn Leu Asn Asp Lys Ala
          35                  40                  45


Leu Cys Phe Thr Lys Cys Arg Gln Ser Gly Ser Asp Ser Cys Asn Val
      50                  55                  60


Glu Asn Leu Gln Arg Tyr Trp Leu Asn Tyr Glu Ala His Leu Met Lys
65                  70                  75                      80


Glu Gly Leu Thr Gln Lys Val Asn Thr Pro Phe Leu Lys Ala Leu Val
              85                  90                  95


Gln Asn Leu Ser Thr Asn Thr Ala Glu Asp Phe Tyr Phe Ser Leu Glu
              100                 105                 110


Pro Ser Gln Val Pro Arg Gln Val Met Lys Asp Glu Asp Lys Pro Pro
          115                 120                 125


Asp Arg Val Arg Leu Pro Lys Ser Leu Phe Arg Ser Leu Pro Gly Asn
      130                 135                 140


Arg Ser Val Val Arg Leu Ala Val Thr Ile Leu Asp Ile Gly Pro Gly
145                 150                 155                     160


Thr Leu Phe Lys Gly Pro Arg Leu Gly Leu Gly Asp Gly Ser Gly Val
              165                 170                 175


Leu Asn Asn Arg Leu Val Gly Leu Ser Val Gly Gln Met His Val Thr
              180                 185                 190


Lys Leu Ala Glu Pro Leu Glu Ile Val Phe Ser His Gln Arg Pro Pro
</pre>

```
                      195                        200                        205


        Pro Asn Met Thr Leu Thr Cys Val Phe Trp Asp Val Thr Lys Gly Thr
            210                     215                     220


        Thr Gly Asp Trp Ser Ser Glu Gly Cys Ser Thr Glu Val Arg Pro Glu
        225                     230                     235                     240


        Gly Thr Val Cys Cys Cys Asp His Leu Thr Phe Phe Ala Leu Leu Leu
                        245                     250                     255


        Arg Pro



        <210>   24
        <211>   298
        <212>   PRT
        <213>   Homo sapiens

        <400>   24

        Leu Gln Glu Leu Pro Asp Lys Ile Val Asp Leu Ala Asn Ile Thr Ile
        1                   5                   10                      15


        Ser Asp Glu Asn Ala Glu Asp Val Ala Glu His Ile Leu Asn Leu Ile
                        20                      25                      30


        Asn Glu Ser Pro Ala Leu Gly Lys Glu Glu Thr Lys Ile Ile Val Ser
                        35                      40                      45


        Lys Ile Ser Asp Ile Ser Gln Cys Asp Glu Ile Ser Met Asn Leu Thr
                50                      55                      60


        His Val Met Leu Gln Ile Ile Asn Val Val Leu Glu Lys Gln Asn Asn
        65                      70                      75                      80


        Ser Ala Ser Asp Leu His Glu Ile Ser Asn Glu Ile Leu Arg Ile Ile
                        85                      90                      95


        Glu Arg Thr Gly His Lys Met Glu Phe Ser Gly Gln Ile Ala Asn Leu
                        100                     105                     110


        Thr Val Ala Gly Leu Ala Leu Ala Val Leu Arg Gly Asp His Thr Phe
                        115                     120                     125


        Asp Gly Met Ala Phe Ser Ile His Ser Tyr Glu Glu Gly Thr Asp Pro
                130                     135                     140


        Glu Ile Phe Leu Gly Asn Val Pro Val Gly Gly Ile Leu Ala Ser Ile
```

                145                     150                     155                     160


Tyr Leu Pro Lys Ser Leu Thr Glu Arg Ile Pro Leu Ser Asn Leu Gln
            165                 170                 175


Thr Ile Leu Phe Asn Phe Phe Gly Gln Thr Ser Leu Phe Lys Thr Lys
            180                 185                 190


Asn Val Thr Lys Ala Leu Thr Thr Tyr Val Val Ser Ala Ser Ile Ser
            195                 200                 205


Asp Asp Met Phe Ile Gln Asn Leu Ala Asp Pro Val Val Ile Thr Leu
            210                 215                 220


Gln His Ile Gly Gly Asn Gln Asn Tyr Gly Gln Val His Cys Ala Phe
225                 230                 235                 240


Trp Asp Phe Glu Asn Asn Asn Gly Leu Gly Gly Trp Asn Ser Ser Gly
            245                 250                 255


Cys Lys Val Lys Glu Thr Asn Val Asn Tyr Thr Ile Cys Gln Cys Asp
            260                 265                 270


His Leu Thr His Phe Gly Val Leu Met Asp Leu Ser Arg Ser Thr Val
            275                 280                 285


Asp Ser Val Asn Glu Gln Ile Leu Ala Leu
    290                 295


<210> 25
<211> 235
<212> PRT
<213> Homo sapiens

<400> 25

Met Asp His Cys Gly Ala Leu Phe Leu Cys Leu Cys Leu Leu Thr Leu
1               5                   10                  15


Gln Asn Ala Thr Thr Glu Thr Trp Glu Glu Leu Leu Ser Tyr Met Glu
            20                  25                  30


Asn Met Gln Val Ser Arg Gly Arg Ser Ser Val Phe Ser Ser Arg Gln
            35                  40                  45


Leu His Gln Leu Glu Gln Met Leu Leu Asn Thr Ser Phe Pro Gly Tyr
    50                  55                  60


Asn Leu Thr Leu Gln Thr Pro Thr Ile Gln Ser Leu Ala Phe Lys Leu

```
                65                           70                           75                           80


                Ser Cys Asp Phe Ser Gly Leu Ser Leu Thr Ser Ala Thr Leu Lys Arg
                            85                           90                           95


                Val Pro Gln Ala Gly Gly Gln His Ala Arg Gly Gln His Ala Met Gln
                            100                          105                          110


                Phe Pro Ala Glu Leu Thr Arg Asp Ala Cys Lys Thr Arg Pro Arg Glu
                            115                          120                          125


                Leu Arg Leu Ile Cys Ile Tyr Phe Ser Asn Thr His Phe Phe Lys Asp
                            130                          135                          140


                Glu Asn Asn Ser Ser Leu Leu Asn Asn Tyr Val Leu Gly Ala Gln Leu
                145                          150                          155                          160


                Ser His Gly His Val Asn Asn Leu Arg Asp Pro Val Asn Ile Ser Phe
                            165                          170                          175


                Trp His Asn Gln Ser Leu Glu Gly Tyr Thr Leu Thr Cys Val Phe Trp
                            180                          185                          190


                Lys Glu Gly Ala Arg Lys Gln Pro Trp Gly Gly Trp Ser Pro Glu Gly
                            195                          200                          205


                Cys Arg Thr Glu Gln Pro Ser His Ser Gln Val Leu Cys Arg Cys Asn
                            210                          215                          220


                His Leu Thr Tyr Phe Ala Val Leu Met Gln Leu
                225                          230                          235


                <210>   26
                <211>   273
                <212>   PRT
                <213>   Homo sapiens

                <400>   26

                Leu Lys Glu Ala Asn Glu Val Ala Asn Gln Ile Leu Asn Leu Thr Ala
                1                   5                            10                           15


                Asp Gly Gln Asn Leu Thr Ser Ala Asn Ile Thr Asn Ile Val Glu Gln
                            20                           25                           30


                Val Lys Arg Ile Val Asn Lys Glu Glu Asn Ile Asp Ile Thr Leu Gly
                            35                           40                           45


                Ser Thr Leu Met Asn Ile Phe Ser Asn Ile Leu Ser Ser Ser Asp Ser
```

```
                50                      55                      60

        Asp Leu Leu Glu Ser Ser Ser Glu Ala Leu Lys Thr Ile Asp Glu Leu
        65              70              75              80

        Ala Phe Lys Ile Asp Leu Asn Ser Thr Ser His Val Asn Ile Thr Thr
                        85              90              95

        Arg Asn Leu Ala Leu Ser Val Ser Ser Leu Leu Pro Gly Thr Asn Ala
                    100             105             110

        Ile Ser Asn Phe Ser Ile Gly Leu Pro Ser Asn Asn Glu Ser Tyr Phe
                    115             120             125

        Gln Met Asp Phe Glu Ser Gly Gln Val Asp Pro Leu Ala Ser Val Ile
                130             135             140

        Leu Pro Pro Asn Leu Leu Glu Asn Leu Ser Pro Glu Asp Ser Val Leu
        145             150             155             160

        Val Arg Arg Ala Gln Phe Thr Phe Phe Asn Lys Thr Gly Leu Phe Gln
                    165             170             175

        Asp Val Gly Pro Gln Arg Lys Thr Leu Val Ser Tyr Val Met Ala Cys
                    180             185             190

        Ser Ile Gly Asn Ile Thr Ile Gln Asn Leu Lys Asp Pro Val Gln Ile
                    195             200             205

        Lys Ile Lys His Thr Arg Thr Gln Glu Val His His Pro Ile Cys Ala
        210             215             220

        Phe Trp Asp Leu Asn Lys Asn Lys Ser Phe Gly Gly Trp Asn Thr Ser
        225             230             235             240

        Gly Cys Val Ala His Arg Asp Ser Asp Ala Ser Glu Thr Val Cys Leu
                    245             250             255

        Cys Asn His Phe Thr His Phe Gly Val Leu Met Asp Leu Pro Arg Ser
                    260             265             270

        Ala


        <210>   27
        <211>   268
        <212>   PRT
        <213>   Homo sapiens
```

<400> 27

Leu Asn Asn Ile Ser Ser Glu Val Gln Ile Leu Thr Ser Asp Ala Asn
1               5                   10                  15

Lys Leu Thr Ala Glu Asn Ile Thr Ser Ala Thr Arg Val Val Gly Gln
        20                  25                  30

Ile Phe Asn Thr Ser Arg Asn Ala Ser Pro Glu Ala Lys Lys Val Ala
        35                  40                  45

Ile Val Thr Val Ser Gln Leu Leu Asp Ala Ser Glu Asp Ala Phe Gln
        50                  55                  60

Arg Val Ala Ala Thr Ala Asn Asp Asp Ala Leu Thr Thr Leu Ile Glu
65                  70                  75                  80

Gln Met Glu Thr Tyr Ser Leu Ser Leu Gly Asn Gln Ser Val Val Glu
                85                  90                  95

Pro Asn Ile Ala Ile Gln Ser Ala Asn Phe Ser Ser Glu Asn Ala Val
            100                 105                 110

Gly Pro Ser Asn Val Arg Phe Ser Val Gln Lys Gly Ala Ser Ser Ser
        115                 120                 125

Leu Val Ser Ser Ser Thr Phe Ile His Thr Asn Val Asp Gly Leu Asn
        130                 135                 140

Pro Asp Ala Gln Thr Glu Leu Gln Val Leu Leu Asn Met Thr Lys Asn
145                 150                 155                 160

Tyr Thr Lys Thr Cys Gly Phe Val Val Tyr Gln Asn Asp Lys Leu Phe
            165                 170                 175

Gln Ser Lys Thr Phe Thr Ala Lys Ser Asp Phe Ser Gln Lys Ile Ile
            180                 185                 190

Ser Ser Lys Thr Asp Glu Asn Glu Gln Asp Gln Ser Ala Ser Val Asp
            195                 200                 205

Met Val Phe Ser Pro Lys Tyr Asn Gln Lys Glu Phe Gln Leu Tyr Ser
        210                 215                 220

Tyr Ala Cys Val Tyr Trp Asn Leu Ser Ala Lys Asp Trp Asp Thr Tyr
225                 230                 235                 240

```
Gly Cys Gln Lys Asp Lys Gly Thr Asp Gly Phe Leu Arg Cys Arg Cys
            245             250             255

Asn His Thr Thr Asn Phe Ala Val Leu Met Thr Phe
            260             265


<210>   28
<211>   316
<212>   PRT
<213>   Homo sapiens

<400>   28

Pro Trp Val Asn Gln Val Ala Gln Lys Ile Lys Ser Gly Glu Asn Ala
1               5               10              15

Ala Asn Ile Ala Ser Glu Leu Ala Arg His Thr Arg Gly Ser Ile Tyr
            20              25              30

Ala Gly Asp Val Ser Ser Ser Val Lys Leu Met Glu Gln Leu Leu Asp
            35              40              45

Ile Leu Asp Ala Gln Leu Gln Ala Leu Arg Pro Ile Glu Arg Glu Ser
    50              55              60

Ala Gly Lys Asn Tyr Asn Lys Met His Lys Arg Glu Arg Thr Cys Lys
65              70              75              80

Asp Tyr Ile Lys Ala Val Val Glu Thr Val Asp Asn Leu Leu Arg Pro
            85              90              95

Glu Ala Leu Glu Ser Trp Lys Asp Met Asn Ala Thr Glu Gln Val His
            100             105             110

Thr Ala Thr Met Leu Leu Asp Val Leu Glu Glu Gly Ala Phe Leu Leu
            115             120             125

Ala Asp Asn Val Arg Glu Pro Ala Arg Phe Leu Ala Ala Lys Glu Asn
    130             135             140

Val Val Leu Glu Val Thr Val Leu Asn Thr Glu Gly Gln Val Gln Glu
145             150             155             160

Leu Val Phe Pro Gln Glu Glu Tyr Pro Arg Lys Asn Ser Ile Gln Leu
            165             170             175

Ser Ala Lys Thr Ile Lys Gln Asn Ser Arg Asn Gly Val Val Lys Val
            180             185             190
```

```
Val Phe Ile Leu Tyr Asn Asn Leu Gly Leu Phe Leu Ser Thr Glu Asn
        195                 200             205

Ala Thr Val Lys Leu Ala Gly Glu Ala Gly Pro Gly Gly Pro Gly Gly
        210                 215             220

Ala Ser Leu Val Val Asn Ser Gln Val Ile Ala Ala Ser Ile Asn Lys
225                 230             235                 240

Glu Ser Ser Arg Val Phe Leu Met Asp Pro Val Ile Phe Thr Val Ala
                245             250             255

His Leu Glu Asp Lys Asn His Phe Asn Ala Asn Cys Ser Phe Trp Asn
            260             265             270

Tyr Ser Glu Arg Ser Met Leu Gly Tyr Trp Ser Thr Gln Gly Cys Arg
        275             280             285

Leu Val Glu Ser Asn Lys Thr His Thr Thr Cys Ala Cys Ser His Leu
    290             295             300

Thr Asn Phe Ala Val Leu Met Ala His Arg Glu Ile
305             310             315
```

```
<210> 29
<211> 313
<212> PRT
<213> Homo sapiens

<400> 29
```

```
His Trp Val Asn Gln Leu Ala Gln Lys Ile Arg Ser Gly Glu Asn Ala
1               5               10              15

Ala Ser Leu Ala Asn Glu Leu Ala Lys His Thr Lys Gly Pro Val Phe
        20              25              30

Ala Gly Asp Val Ser Ser Ser Val Arg Leu Met Glu Gln Leu Val Asp
        35              40              45

Ile Leu Asp Ala Gln Leu Gln Glu Leu Lys Pro Ser Glu Lys Asp Ser
    50              55              60

Ala Gly Arg Ser Tyr Asn Lys Leu Gln Lys Arg Glu Lys Thr Cys Arg
65              70              75              80

Ala Tyr Leu Lys Ala Ile Val Asp Thr Val Asp Asn Leu Leu Arg Pro
        85              90              95
```

```
Glu Ala Leu Glu Ser Trp Lys His Met Asn Ser Ser Glu Gln Ala His
            100             105             110

Thr Ala Thr Met Leu Leu Asp Thr Leu Glu Glu Gly Ala Phe Val Leu
            115             120             125

Ala Asp Asn Leu Leu Glu Pro Thr Arg Val Ser Met Pro Thr Glu Asn
    130             135             140

Ile Val Leu Glu Val Ala Val Leu Ser Thr Glu Gly Gln Ile Gln Asp
145             150             155             160

Phe Lys Phe Pro Leu Gly Ile Lys Gly Ala Gly Ser Ser Ile Gln Leu
            165             170             175

Ser Ala Asn Thr Val Lys Gln Asn Ser Arg Asn Gly Leu Ala Lys Leu
            180             185             190

Val Phe Ile Ile Tyr Arg Ser Leu Gly Gln Phe Leu Ser Thr Glu Asn
            195             200             205

Ala Thr Ile Lys Leu Gly Ala Asp Phe Ile Gly Arg Asn Ser Thr Ile
    210             215             220

Ala Val Asn Ser His Val Ile Ser Val Ser Ile Asn Lys Glu Ser Ser
225             230             235             240

Arg Val Tyr Leu Thr Asp Pro Val Leu Phe Thr Leu Pro His Ile Asp
            245             250             255

Pro Asp Asn Tyr Phe Asn Ala Asn Cys Ser Phe Trp Asn Tyr Ser Glu
            260             265             270

Arg Thr Met Met Gly Tyr Trp Ser Thr Gln Gly Cys Lys Leu Val Asp
            275             280             285

Thr Asn Lys Thr Arg Thr Thr Cys Ala Cys Ser His Leu Thr Asn Phe
            290             295             300

Ala Ile Leu Met Ala His Arg Glu Ile
305             310
```

```
<210>   30
<211>   327
<212>   PRT
<213>   Homo sapiens

<400>   30
```

```
Pro Trp Val Asn His Ile Thr Gln Lys Leu Lys Ser Gly Glu Thr Ala
1               5               10                  15

Ala Asn Ile Ala Arg Glu Leu Ala Glu Gln Thr Arg Asn His Leu Asn
            20              25                  30

Ala Gly Asp Ile Thr Tyr Ser Val Arg Ala Met Asp Gln Leu Val Gly
            35              40                  45

Leu Leu Asp Val Gln Leu Arg Asn Leu Thr Pro Gly Gly Lys Asp Ser
        50              55                  60

Ala Ala Arg Ser Leu Asn Lys Leu Gln Lys Arg Glu Arg Ser Cys Arg
65              70              75                  80

Ala Tyr Val Gln Ala Met Val Glu Thr Val Asn Asn Leu Leu Gln Pro
                85              90                  95

Gln Ala Leu Asn Ala Trp Arg Asp Leu Thr Thr Ser Asp Gln Leu Arg
            100             105                 110

Ala Ala Thr Met Leu Leu His Thr Val Glu Glu Ser Ala Phe Val Leu
            115             120                 125

Ala Asp Asn Leu Leu Lys Thr Asp Ile Val Arg Glu Asn Thr Asp Asn
            130             135                 140

Ile Lys Leu Glu Val Ala Arg Leu Ser Thr Glu Gly Asn Leu Glu Asp
145             150             155                 160

Leu Lys Phe Pro Glu Asn Met Gly His Gly Ser Thr Ile Gln Leu Ser
                165             170                 175

Ala Asn Thr Leu Lys Gln Asn Gly Arg Asn Gly Glu Ile Arg Val Ala
            180             185                 190

Phe Val Leu Tyr Asn Asn Leu Gly Pro Tyr Leu Ser Thr Glu Asn Ala
            195             200                 205

Ser Met Lys Leu Gly Thr Glu Ala Leu Ser Thr Asn His Ser Val Ile
            210             215                 220

Val Asn Ser Pro Val Ile Thr Ala Ala Ile Asn Lys Glu Phe Ser Asn
225             230             235                 240

Lys Val Tyr Leu Ala Asp Pro Val Val Phe Thr Val Lys His Ile Lys
                245             250                 255
```

```
Gln Ser Glu Glu Asn Phe Asn Pro Asn Cys Ser Phe Trp Ser Tyr Ser
            260                 265                 270

Lys Arg Thr Met Thr Gly Tyr Trp Ser Thr Gln Gly Cys Arg Leu Leu
            275                 280                 285

Thr Thr Asn Lys Thr His Thr Thr Cys Ser Cys Asn His Leu Thr Asn
            290                 295                 300

Phe Ala Val Leu Met Ala His Val Glu Val Lys His Ser Asp Ala Val
305                 310                 315                 320

His Asp Leu Leu Leu Asp Val
                325


<210>  31
<211>  296
<212>  PRT
<213>  Homo sapiens

<400>  31

Ala Asn Ile Asn Lys Thr Leu Thr Lys Ile Arg Ser Ile Lys Glu Pro
1               5               10                  15

Val Ala Leu Leu Gln Glu Val Tyr Arg Asn Ser Val Thr Asp Leu Ser
            20                  25                  30

Pro Thr Asp Ile Ile Thr Tyr Ile Glu Ile Leu Ala Glu Ser Ser Ser
            35                  40                  45

Leu Leu Gly Tyr Lys Asn Asn Thr Ile Ser Ala Lys Asp Thr Leu Ser
            50                  55                  60

Asn Ser Thr Leu Thr Glu Phe Val Lys Thr Val Asn Asn Phe Val Gln
65                  70                  75                  80

Arg Asp Thr Phe Val Val Trp Asp Lys Leu Ser Val Asn His Arg Arg
                85                  90                  95

Thr His Leu Thr Lys Leu Met His Thr Val Glu Gln Ala Thr Leu Arg
            100                 105                 110

Ile Ser Gln Ser Phe Gln Lys Thr Thr Glu Phe Asp Thr Asn Ser Thr
            115                 120                 125

Asp Ile Ala Leu Lys Val Phe Phe Phe Asp Ser Tyr Asn Met Lys His
            130                 135                 140
```

```
Ile His Pro His Met Asn Met Asp Gly Asp Tyr Ile Asn Ile Phe Pro
145                 150                 155                 160

Lys Arg Lys Ala Ala Tyr Asp Ser Asn Gly Asn Val Ala Val Ala Phe
                165                 170                 175

Val Tyr Tyr Lys Ser Ile Gly Pro Leu Leu Ser Ser Ser Asp Asn Phe
            180                 185                 190

Leu Leu Lys Pro Gln Asn Tyr Asp Asn Ser Glu Glu Glu Glu Arg Val
        195                 200                 205

Ile Ser Ser Val Ile Ser Val Ser Met Ser Ser Asn Pro Pro Thr Leu
    210                 215                 220

Tyr Glu Leu Glu Lys Ile Thr Phe Thr Leu Ser His Arg Lys Val Thr
225                 230                 235                 240

Asp Arg Tyr Arg Ser Leu Cys Ala Phe Trp Asn Tyr Ser Pro Asp Thr
            245                 250                 255

Met Asn Gly Ser Trp Ser Ser Glu Gly Cys Glu Leu Thr Tyr Ser Asn
            260                 265                 270

Glu Thr His Thr Ser Cys Arg Cys Asn His Leu Thr His Phe Ala Ile
        275                 280                 285

Leu Met Ser Ser Gly Pro Ser Ile
    290                 295


<210>  32
<211>  278
<212>  PRT
<213>  Homo sapiens

<400>  32

Gln Ala Ile Trp Gly Leu Ala Asp Gln Leu His Gln Pro Val Asn Asp
1               5               10                  15

Asp Ile Leu Asn Arg Val Leu His Thr Ile Ser Met Lys Val Ala Thr
            20                  25                  30

Glu Asn Thr Asp Glu Gln Leu Ser Ala Met Met His Leu Ile Glu Lys
        35                  40                  45

Ile Thr Thr Glu Gly Lys Ile Gln Ala Phe Ser Val Ala Ser Arg Thr
    50                  55                  60
```

```
Leu Phe Tyr Glu Ile Leu Cys Ser Leu Ile Asn Pro Lys Arg Lys Asp
65              70              75              80

Thr Arg Gly Phe Ser His Phe Ala Glu Val Thr Glu Asn Phe Ala Phe
            85              90              95

Ser Leu Leu Thr Asn Val Thr Cys Gly Ser Pro Gly Glu Lys Ser Lys
            100             105             110

Thr Ile Leu Asp Ser Cys Pro Tyr Leu Ser Ile Leu Ala Leu His Trp
            115             120             125

Tyr Pro Gln Gln Ile Asn Gly His Lys Phe Glu Gly Lys Glu Gly Asp
        130             135             140

Tyr Ile Arg Ile Pro Glu Arg Leu Leu Asp Val Gln Asp Ala Glu Ile
145             150             155             160

Met Ala Gly Lys Ser Thr Cys Lys Leu Val Gln Phe Thr Glu Tyr Ser
            165             170             175

Ser Gln Gln Trp Phe Ile Ser Gly Asn Asn Leu Pro Thr Leu Lys Asn
            180             185             190

Lys Val Leu Ser Leu Ser Val Lys Gly Gln Ser Ser Gln Leu Leu Thr
            195             200             205

Asn Asp Asn Glu Val Leu Tyr Arg Ile Tyr Ala Ala Glu Pro Arg Ile
        210             215             220

Ile Pro Gln Thr Ser Leu Cys Leu Leu Trp Asn Gln Ala Ala Ala Ser
225             230             235             240

Trp Leu Ser Asp Ser Gln Phe Cys Lys Val Val Glu Glu Thr Ala Asp
            245             250             255

Tyr Val Glu Cys Ala Cys Ser His Met Ser Val Tyr Ala Val Tyr Ala
            260             265             270

Arg Thr Asp Asn Leu Ser
            275
```

```
<210>    33
<211>    422
<212>    PRT
<213>    Homo sapiens

<400>    33
```

Ala Gln Ile Arg Lys Asn Ile Thr Glu Thr Leu Val Ser Leu Arg Val
1               5               10                  15

His Thr Val Asp Asp Ile Gln Gln Ile Ala Ala Ala Leu Ala Gln Cys
            20              25                  30

Met Gly Pro Ser Arg Glu Leu Val Cys Arg Ser Cys Leu Lys Gln Thr
            35              40                  45

Leu His Lys Leu Glu Ala Met Met Leu Ile Leu Gln Ala Glu Thr Thr
        50              55                  60

Ala Gly Thr Val Thr Pro Thr Ala Ile Gly Asp Ser Ile Leu Asn Ile
65              70                  75                  80

Thr Gly Asp Leu Ile His Leu Ala Ser Ser Asp Val Arg Ala Pro Gln
            85                  90                  95

Pro Ser Glu Leu Gly Ala Glu Ser Pro Ser Arg Met Val Ala Ser Gln
            100                 105                 110

Ala Tyr Asn Leu Thr Ser Ala Leu Met Arg Ile Leu Met Arg Ser Arg
        115                 120                 125

Val Leu Asn Glu Glu Pro Leu Thr Leu Ala Gly Glu Glu Ile Val Ala
    130                 135                 140

Gln Gly Lys Arg Ser Asp Pro Arg Ser Leu Leu Cys Tyr Gly Gly Ala
145                 150                 155                 160

Pro Gly Pro Gly Cys His Phe Ser Ile Pro Glu Ala Phe Ser Gly Ala
            165                 170                 175

Leu Ala Asn Leu Ser Asp Val Val Gln Leu Ile Phe Leu Val Asp Ser
        180                 185                 190

Asn Pro Phe Pro Phe Gly Tyr Ile Ser Asn Tyr Thr Val Ser Thr Lys
        195                 200                 205

Val Ala Ser Met Ala Phe Gln Thr Gln Ala Gly Ala Gln Ile Pro Ile
    210                 215                 220

Glu Arg Leu Ala Ser Glu Arg Ala Ile Thr Val Lys Val Pro Asn Asn
225                 230                 235                 240

Ser Asp Trp Ala Ala Arg Gly His Arg Ser Ser Ala Asn Ser Ala Asn
            245                 250                 255

Ser Val Val Val Gln Pro Gln Ala Ser Val Gly Ala Val Val Thr Leu
              260               265           270

Asp Ser Ser Asn Pro Ala Ala Gly Leu His Leu Gln Leu Asn Tyr Thr
              275               280               285

Leu Leu Asp Gly His Tyr Leu Ser Glu Glu Pro Glu Pro Tyr Leu Ala
              290               295               300

Val Tyr Leu His Ser Glu Pro Arg Pro Asn Glu His Asn Cys Ser Ala
305               310               315               320

Ser Arg Arg Ile Arg Pro Glu Ser Leu Gln Gly Ala Asp His Arg Pro
              325               330               335

Tyr Thr Phe Phe Ile Ser Pro Gly Ser Arg Asp Pro Ala Gly Ser Tyr
              340               345               350

His Leu Asn Leu Ser Ser His Phe Arg Trp Ser Ala Leu Gln Val Ser
              355               360               365

Val Gly Leu Tyr Thr Ser Leu Cys Gln Tyr Phe Ser Glu Glu Asp Met
              370               375               380

Val Trp Arg Thr Glu Gly Leu Leu Pro Leu Glu Glu Thr Ser Pro Arg
385               390               395               400

Gln Ala Val Cys Leu Thr Arg His Leu Thr Ala Phe Gly Ala Ser Leu
              405               410               415

Phe Val Pro Pro Ser His
              420

<210>   34
<211>   430
<212>   PRT
<213>   Homo sapiens

<400>   34

Asn Leu Ser Thr Leu Gln Leu Met Gly Ser Tyr Thr Glu Ile Arg Asn
1               5               10              15

Tyr Ile Thr Val Ile Thr Arg Ile Leu Ser Arg Leu Ser Lys Glu Asp
              20              25              30

Lys Thr Ala Ser Cys Asn Gln Trp Ser Arg Ile Gln Asp Ala Leu Ile
              35              40              45

Ser Ser Val Cys Arg Leu Ala Phe Val Asp Gln Glu Glu Met Ile Gly
        50              55          60

Ser Val Leu Met Leu Arg Asp Leu Val Ser Phe Ser Asn Lys Leu Gly
65              70          75                      80

Phe Met Ser Ala Val Leu Ile Leu Lys Tyr Thr Arg Ala Leu Leu Ala
            85          90                      95

Gln Gly Gln Phe Ser Gly Pro Phe Val Ile Asp Lys Gly Val Arg Leu
            100         105             110

Glu Leu Ile Gly Leu Ile Ser Arg Val Trp Glu Val Ser Glu Gln Glu
        115             120             125

Asn Ser Lys Glu Glu Val Tyr Arg His Glu Glu Gly Ile Thr Val Ile
    130             135             140

Ser Asp Leu Leu Leu Gly Cys Leu Ser Leu Asn His Val Ser Thr Gly
145             150             155             160

Gln Met Glu Phe Arg Thr Leu Leu His Tyr Asn Leu Gln Ser Ser Val
            165             170             175

Gln Ser Leu Gly Ser Val Gln Val His Leu Pro Gly Asp Leu Ala Gly
        180             185             190

His Ser Pro Ala Gly Ala Glu Thr Gln Ser Pro Cys Tyr Ile Ser Gln
        195             200             205

Leu Ile Leu Phe Lys Lys Asn Pro Tyr Pro Gly Ser Gln Ala Pro Gly
    210             215             220

Gln Ile Gly Gly Val Val Gly Leu Asn Leu Tyr Thr Cys Ser Ser Arg
225             230             235             240

Arg Pro Ile Asn Arg Gln Trp Leu Arg Lys Pro Val Met Val Glu Phe
            245             250             255

Gly Glu Glu Asp Gly Leu Asp Asn Arg Arg Asn Lys Thr Thr Phe Val
            260             265             270

Leu Leu Arg Asp Lys Val Asn Leu His Gln Phe Thr Glu Leu Ser Glu
            275             280             285

Asn Pro Gln Glu Ser Leu Gln Ile Glu Ile Glu Phe Ser Lys Pro Val

```
                290                     295                     300

        Thr Arg Ala Phe Pro Val Met Leu Leu Val Arg Phe Ser Glu Lys Pro
        305                 310                 315                 320

        Thr Pro Ser Asp Phe Leu Val Lys Gln Ile Tyr Phe Trp Asp Glu Ser
                        325                 330                 335

        Ile Val Gln Ile Tyr Ile Pro Ala Ala Ser Gln Lys Asp Ala Ser Val
                    340                 345                 350

        Gly Tyr Leu Ser Leu Leu Asp Ala Asp Tyr Asp Arg Lys Pro Pro Asn
                    355                 360                 365

        Arg Tyr Leu Ala Lys Ala Val Asn Tyr Thr Val His Phe Gln Trp Ile
                    370                 375                 380

        Arg Cys Leu Phe Trp Asp Lys Arg Glu Trp Lys Ser Glu Arg Phe Ser
        385                 390                 395                 400

        Pro Gln Pro Gly Thr Ser Pro Glu Lys Val Asn Cys Ser Tyr His Arg
                        405                 410                 415

        Leu Ala Ala Phe Ala Leu Leu Arg Arg Lys Leu Lys Ala Ser
                        420                 425                 430


        <210>   35
        <211>   411
        <212>   PRT
        <213>   Homo sapiens

        <400>   35

        Ser Leu Ser Ile Asp Val Arg Gln Lys Val Arg Glu His Val Leu Gly
        1               5                   10                  15

        Ser Leu Ser Ala Val Thr Thr Gly Leu Glu Asp Val Gln Arg Val Gln
                    20                  25                  30

        Glu Leu Ala Glu Val Leu Arg Glu Val Thr Cys Arg Ser Lys Glu Leu
                    35                  40                  45

        Thr Pro Ser Ala Gln Trp Glu Ala Ser Leu Ala Leu Gln His Ala Ser
                50                  55                  60

        Glu Ala Leu Leu Thr Val Ser Ala Lys Ala Arg Pro Glu Asp Gln Arg
        65                  70                  75                  80

        Arg Gln Ala Ala Thr Arg Asp Leu Phe Gln Ala Val Gly Ser Val Leu
```

<pre>
                    85                      90                      95


        Glu Ala Ser Leu Ser Asn Arg Pro Glu Glu Pro Ala Glu Ala Ser Ser
                    100                 105             110


        Ser Gln Ile Ala Thr Val Leu Arg Leu Leu Arg Val Met Glu His Val
                    115                 120             125


        Gln Thr Thr Leu Leu Leu Gly Lys Leu Pro Gly Gly Leu Pro Ala Met
                    130                 135             140


        Leu Ala Thr Pro Ser Ile Ser Val Tyr Thr Asn Arg Ile Gln Pro Trp
        145                 150                 155             160


        Ser Trp Gln Gly Ser Ser Leu Arg Pro Asp Ala Ala Asp Ser Ala Thr
                    165                 170             175


        Phe Met Leu Pro Ala Ala Ser Ser Leu Ser Ser Leu Glu Gly Gly Gln
                    180                 185             190


        Glu Pro Val Asp Ile Lys Ile Met Ser Phe Pro Lys Ser Pro Phe Pro
                    195                 200             205


        Ala Arg Ser His Phe Asp Val Ser Gly Thr Val Gly Gly Leu Arg Val
                    210                 215             220


        Thr Ser Pro Ser Gly Gln Leu Ile Pro Val Lys Asn Leu Ser Glu Asn
        225                 230                 235             240


        Ile Glu Ile Leu Leu Pro Arg His Ser Gln Arg His Ser Gln Pro Thr
                    245                 250             255


        Val Leu Asn Leu Thr Ser Pro Glu Ala Leu Trp Val Asn Val Thr Ser
                    260                 265             270


        Gly Glu Ala Thr Leu Gly Ile Gln Leu His Trp Arg Pro Asp Ile Ala
                    275                 280             285


        Leu Thr Leu Ser Leu Gly Tyr Gly Tyr His Pro Asn Lys Ser Ser Tyr
                    290                 295             300


        Asp Ala Gln Thr His Leu Val Pro Met Val Ala Pro Asp Glu Leu Pro
        305                 310                 315             320


        Thr Trp Ile Leu Ser Pro Gln Asp Leu Arg Phe Gly Glu Gly Val Tyr
                    325                 330             335
</pre>

```
Tyr Leu Thr Val Val Pro Glu Ser Asp Leu Glu Pro Ala Pro Gly Arg
        340             345             350

Asp Leu Thr Val Gly Ile Thr Thr Phe Leu Ser His Cys Val Phe Trp
        355             360             365

Asp Glu Val Gln Glu Thr Trp Asp Asp Ser Gly Cys Gln Val Gly Pro
        370             375             380

Arg Thr Ser Pro Tyr Gln Thr His Cys Leu Cys Asn His Leu Thr Phe
385             390             395             400

Phe Gly Ser Thr Phe Leu Val Met Ser Asn Ala
            405             410
```

```
<210>   36
<211>   411
<212>   PRT
<213>   Homo sapiens

<400>   36
```

```
Ser Leu Gln Lys Ala Ser Gly Gln Val Ile Asp Glu Ile Ala Gly Asn
1               5               10              15

Phe Ser Arg Ala Val His Gly Leu Gln Ala Leu Asn Lys Leu Gln Glu
        20              25              30

Ala Cys Glu Phe Leu Gln Lys Leu Thr Ala Leu Thr Pro Arg Phe Ser
        35              40              45

Lys Pro Ala Gln Val Asn Leu Ile Asn Ser Leu Ile Tyr Leu Ser Glu
        50              55              60

Glu Leu Leu Arg Ile Pro Phe Gln Asn Asn Asn Ser Leu Gly Phe Lys
65              70              75              80

Val Pro Pro Thr Val Cys Pro Phe His Ser Leu Asn Asn Val Thr Lys
                85              90              95

Ala Gly Glu Gly Ser Trp Leu Glu Ser Lys Arg His Thr Glu Pro Val
            100             105             110

Glu Asp Ile Leu Glu Met Ser Leu Val Glu Phe Gly Asn Ile Gly Glu
            115             120             125

Ala Phe Leu Glu Gln Asn Gln Ser Pro Glu Ser Ser Val Thr Leu Thr
        130             135             140
```

Ser Ala Asn Ala Thr Leu Leu Leu Ser Arg Gln Asn Ile Ser Thr Leu
145                 150                 155                 160

Pro Leu Ser Ser Tyr Thr Leu Gly His Pro Ala Pro Val Arg Leu Gly
                165                 170                 175

Phe Pro Ser Ala Leu Ala Leu Lys Glu Leu Leu Asn Lys His Pro Gly
                180                 185                 190

Val Asn Val Gln Ile Thr Gly Leu Ala Phe Asn Pro Phe Lys Asp Leu
                195                 200                 205

Asp Asn Arg Asn Ile Val Gly Ser Ile Gly Ser Val Leu Leu Ser Ala
    210                 215                 220

Asn Arg Lys Leu Leu Gln Val His Asp Leu Met Glu Asp Ile Glu Ile
225                 230                 235                 240

Met Leu Trp Arg Asn Val Ser Leu Glu Thr His Pro Thr Ser Leu Asn
                245                 250                 255

Met Ser Thr His Gln Leu Thr Ile Thr Val Asn Val Thr Ser Leu Glu
                260                 265                 270

Lys Ser Leu Ile Val Ser Ile Asp Pro Asp Ser Pro Leu Leu Met Thr
                275                 280                 285

Leu Tyr Leu Gly Phe Gln Tyr Gln Pro Asn Cys Thr His Phe His Leu
    290                 295                 300

Asn Ile Thr Leu Pro Lys Asp Lys Val Trp Gln Lys Asp Glu Glu Tyr
305                 310                 315                 320

Thr Trp Val Leu Asn Pro Glu His Leu Gln His Gly Ile Gly Thr Tyr
                325                 330                 335

Tyr Ile Thr Ala Val Leu Ser Glu Arg Gln Glu Gly Ala Gln Gln Thr
                340                 345                 350

Pro Ser Leu Val Ser Val Ile Thr Ala Val Thr Gln Cys Tyr Tyr Trp
                355                 360                 365

Glu Ile His Asn Gln Thr Trp Ser Ser Ala Gly Cys Gln Val Gly Pro
    370                 375                 380

Gln Ser Thr Ile Leu Arg Thr Gln Cys Leu Cys Asn His Leu Thr Phe
385                 390                 395                 400

```
            Phe Ala Ser Asp Phe Phe Val Val Pro Arg Thr
                        405             410


            <210>   37
            <211>   439
            <212>   PRT
            <213>   Homo sapiens


            <400>   37

            Ile Val Ala Ser Val Leu Asn Asn Met Lys Thr Glu Leu Pro Leu Arg
            1               5               10              15


            Asp Asp Arg Val Asn Leu Arg Lys His Leu Ile Asp Gln Ser Phe Leu
                        20              25              30


            Leu Pro Val Ser Thr Leu Val Glu Ile Gly Gln Val Val Met Thr Ile
                        35              40              45


            Thr Lys Leu Thr Gln Lys Pro Ser Glu Phe Thr Trp Asp Ala Gln Lys
                50              55              60


            Arg Ala Thr Met Arg Val Trp Gln Ala Asn Gln Ala Leu Gln Glu Tyr
            65              70              75              80


            Gln Gln Lys Asp Lys Arg Phe Arg Ser Glu Gln Ile Glu Ile Val Ser
                        85              90              95


            Thr Gly Ile Leu Met Ser Leu Ser Asn Ile Leu Lys Met Thr Ser Pro
                        100             105             110


            His Gln Val Val Lys Asp Pro Phe Tyr Val Ile Glu Ser Leu Ser Asp
                        115             120             125


            Thr Ile Leu Ala Asn Lys Val Pro Gly Asn Lys Thr Thr Ser Met Arg
                        130             135             140


            Thr Pro Asn Phe Asn Met Tyr Val Lys Lys Val Glu Lys Trp Gly Ile
            145             150             155             160


            Asn Gln Leu Phe Arg Asn Glu Lys His Cys Arg Asn Cys Phe Tyr Pro
                        165             170             175


            Thr Leu Asn Val Ser Ser Val Pro Gly Leu Ser Ala Asn Gly Pro Ile
                        180             185             190


            Ser Thr Met Phe Cys Asp Phe Thr Asn Asp Leu Phe Pro Trp Leu Asn
                        195             200             205
```

```
Asp Gln Glu Asn Thr Ser Val Glu Val Ser Gly Phe Arg Met Thr Gly
    210                 215                 220

Val Ala Asp Asn Gly Ser Val Leu Glu Ile Thr Pro Asp Val Ala Glu
    225             230                 235                 240

Val Tyr Leu Val Arg Lys Asn Leu Thr Phe Ala Ala Phe Asn Leu Thr
                245                 250                 255

Val Gly Pro Asn Ser Glu Val Asp Gly Ser Leu Lys Lys Thr Thr Gly
                260                 265                 270

Gly Phe Ser Phe Gln Val Asp Ser Thr Val Leu Arg Glu Val Leu Val
        275                 280                 285

His Ile Val Thr Glu Val Met Val Leu Phe Thr Val Leu Val Tyr Thr
    290                 295                 300

Gly Ser Gln Ile Thr Pro Thr Ala Leu Val Ala Thr Phe Leu Val Pro
305                 310                 315                 320

His Asp Ile Pro Pro Phe Ala Ser Gln Ser Ala Leu Phe Asp Pro Ala
                325                 330                 335

Cys Thr Val Lys Lys Ala Arg Val Val Cys Leu Pro Val Ser Leu Leu
                340                 345                 350

Gln Leu Ile Ala Gln His Ser His Ser Pro His Cys Thr Val Ser Ile
        355                 360                 365

Val Leu Gln Ala Pro Arg Phe Val Met Lys Leu Asn Asp Lys Leu Val
    370                 375                 380

Arg Ile Ser Ile Phe Ser Val Gln Cys Leu Asp Met Tyr Gly Ile Gln
385                 390                 395                 400

Ser Glu Trp Arg Glu Gly Tyr Cys Ile Leu Gly Glu Lys Thr Ser Trp
                405                 410                 415

Tyr Glu Val His Cys Ile Cys Lys Asn Val Val Arg Ala Arg Arg Gln
                420                 425                 430

Leu Gly Thr Ile Gly Leu Thr
            435


<210>  38
<211>  26
```

<212> PRT
<213> Caenorhabditis elegans

<400> 38

His Leu Thr His Phe Ala Val Leu Met Asp Val Arg Gly His Asp Leu
1               5               10              15

Asn Glu Ile Asp Gln Thr Leu Leu Thr Leu
            20              25

<210> 39
<211> 26
<212> PRT
<213> Rattus norvegicus

<400> 39

His Leu Thr Asn Phe Ala Val Leu Met Ala His Arg Glu Ile Tyr Gln
1               5               10              15

Gly Arg Ile Asn Glu Leu Leu Leu Ser Val
            20              25

<210> 40
<211> 28
<212> PRT
<213> Drosophila melanogaster

<400> 40

His Leu Thr Asn Phe Ala Ile Leu Met Asp Val Val Asp Glu His Gln
1               5               10              15

His Ser Leu Phe Thr Met Phe Asp Gly Asn Met Arg
            20              25

<210> 41
<211> 24
<212> PRT
<213> Homo sapiens

<400> 41

His Leu Thr Tyr Phe Ala Val Leu Met Gln Leu Ser Pro Ala Leu Val
1               5               10              15

Pro Ala Glu Leu Leu Ala Pro Leu
            20

<210> 42
<211> 24
<212> PRT
<213> Homo sapiens

<400> 42

His Leu Thr Tyr Phe Ala Val Leu Met Val Ser Ser Val Glu Val Asp
1               5                   10                  15

Ala Val His Lys His Tyr Leu Ser
                20

<210> 43
<211> 26
<212> PRT
<213> Homo sapiens

<400> 43

His Leu Thr Asn Phe Ala Ile Leu Met Gln Val Val Pro Leu Glu Leu
1               5                   10                  15

Ala Arg Gly His Gln Val Ala Leu Ser Ser
                20                  25

<210> 44
<211> 19
<212> PRT
<213> Homo sapiens

<400> 44

His Leu Ser Ser Phe Ala Ile Leu Met Ala His Tyr Asp Val Glu Asp
1               5                   10                  15

Trp Lys Leu

<210> 45
<211> 26
<212> PRT
<213> Homo sapiens

<400> 45

His Phe Thr His Phe Gly Val Leu Met Asp Leu Pro Arg Ser Ala Ser
1               5                   10                  15

Gln Leu Asp Ala Arg Asn Thr Lys Val Leu
                20                  25

<210> 46
<211> 26
<212> PRT
<213> Drosophila melanogaster

<400> 46

His Ile Ser Ser Tyr Ala Val Ile Val Asp Val Ile Asp Pro Glu Asp

```
                    1                    5                         10                        15


                    Ile Pro Glu Pro Ser Leu Leu Val Gln Ile
                                    20                  25



                    <210>  47
                    <211>  26
                    <212>  PRT
                    <213>  Homo sapiens

                    <400>  47

                    His Leu Thr Ala Phe Gly Ala Ser Leu Phe Val Pro Pro Ser His Val
                    1                    5                         10                        15


                    Arg Phe Val Phe Pro Glu Pro Thr Ala Asp
                                    20                  25



                    <210>  48
                    <211>  30
                    <212>  PRT
                    <213>  Caenorhabditis elegans

                    <400>  48

                    His Leu Thr Met Phe Ser Val Gly Ala Phe Asn Pro Thr Ile Asp Ala
                    1                    5                         10                        15


                    Asp Phe Ser Tyr Asn Tyr Asn Val Asn Glu Ile Glu Lys Asn
                                    20                  25                  30



                    <210>  49
                    <211>  30
                    <212>  PRT
                    <213>  Homo sapiens

                    <400>  49

                    Arg Leu Ala Ala Phe Ala Leu Leu Arg Arg Lys Leu Lys Ala Ser Phe
                    1                    5                         10                        15


                    Glu Val Ser Asp Ile Ser Lys Leu Gln Ser His Pro Glu Asn
                                    20                  25                  30



                    <210>  50
                    <211>  32
                    <212>  PRT
                    <213>  Homo sapiens

                    <400>  50

                    His Leu Thr Phe Phe Gly Ser Thr Phe Leu Val Met Ser Asn Ala Ile
                    1                    5                         10                        15
```

73

```
Asn Ile His Gln Thr Ala Glu Leu Phe Ala Thr Phe Glu Asp Asn Pro
            20              25              30
```

<210> 51
<211> 32
<212> PRT
<213> Homo sapiens

<400> 51

```
His Leu Thr Phe Phe Ala Ser Asp Phe Phe Val Val Pro Arg Thr Val
1           5               10              15
```

```
Asn Val Glu Asp Thr Ile Lys Leu Phe Leu Arg Val Thr Asn Asn Pro
            20              25              30
```

<210> 52
<211> 69
<212> PRT
<213> Homo sapiens

<400> 52

```
Gln Ser Ala Thr Asp Val Ala Ala Phe Leu Gly Ala Leu Ala Ser Leu
1           5               10              15
```

```
Gly Ser Leu Asn Ile Pro Tyr Lys Ile Glu Ala Val Gln Ser Glu Thr
            20              25              30
```

```
Val Glu Pro Pro Pro Pro Ala Gln Leu His Phe Met Tyr Val Ala Ala
        35              40              45
```

```
Ala Ala Phe Val Leu Leu Phe Phe Val Gly Cys Gly Val Leu Leu Ser
        50              55              60
```

```
Arg Lys Arg Arg Arg
65
```

<210> 53
<211> 64
<212> PRT
<213> Homo sapiens

<400> 53

```
Lys Asn Thr Asp Ala Ala Ala Ala Leu Leu Ala Ser His Ala Ile Gln
1           5               10              15
```

```
Gly Thr Leu Ser Tyr Pro Leu Val Ser Val Val Ser Glu Ser Leu Thr
            20              25              30
```

```
Pro Glu Arg Thr Gln Leu Leu Tyr Leu Leu Ala Val Ala Val Val Ile
        35              40              45
```

Ile Leu Phe Ile Ile Leu Leu Gly Val Ile Met Ala Lys Arg Lys Arg
50                     55                  60

<210>  54
<211>  68
<212>  PRT
<213>  Homo sapiens

<400>  54

Pro Asp Ala Gln Ser Ala Ala Asp Tyr Leu Gly Ala Leu Ser Ala Val
1               5                   10                  15

Glu Arg Leu Asp Phe Pro Tyr Pro Leu Arg Asp Val Arg Gly Glu Pro
            20                  25                  30

Leu Glu Pro Pro Glu Pro Ser Val Pro Leu Leu Pro Leu Leu Val Ala
            35                  40                  45

Gly Ala Val Leu Leu Leu Val Ile Leu Val Leu Gly Val Met Val Ala
    50                  55                  60

Arg Arg Lys Arg
65

<210>  55
<211>  74
<212>  PRT
<213>  Homo sapiens

<400>  55

Trp Asp Pro Gly Leu Leu Leu Arg Phe Leu Ala Ala Met Ala Ala Val
1               5                   10                  15

Gly Ala Leu Glu Pro Leu Leu Pro Gly Pro Leu Leu Ala Val His Pro
            20                  25                  30

His Ala Gly Thr Ala Pro Pro Ala Asn Gln Leu Pro Trp Pro Val Leu
            35                  40                  45

Cys Ser Pro Val Ala Gly Val Ile Leu Leu Ala Leu Gly Ala Leu Leu
    50                  55                  60

Val Leu Gln Leu Ile Arg Arg Arg Arg Arg
65                  70

<210>  56
<211>  70
<212>  PRT

<210> Drosophila melanogaster

<400> 56

Gly Asn Met Arg Leu Ile Lys Ile Asp Thr Ala Ala Lys His Gln Leu
1               5                   10                  15

Arg Asn Asp Phe Gln Ile His Ser Val Arg Gly Ile Lys Asn Pro Gly
            20                  25                  30

Asp Glu Asp Asn Gly Glu Pro Pro Ala Asn Val Lys Tyr Val Ile Thr
        35                  40                  45

Gly Ile Ile Leu Val Ile Ile Ala Leu Ala Phe Phe Gly Met Val Leu
    50                  55                  60

Ser Thr Gln Arg Lys Arg
65                  70

<210> 57
<211> 49
<212> PRT
<213> Drosophila melanogaster

<400> 57

Ile His Ser Val Arg Gly Ile Lys Asn Pro Gly Asp Glu Asp Asn Gly
1               5                   10                  15

Glu Pro Pro Ala Asn Val Lys Tyr Val Ile Thr Gly Ile Ile Leu Val
            20                  25                  30

Ile Ile Ala Leu Ala Phe Phe Gly Met Val Leu Ser Thr Gln Arg Lys
        35                  40                  45

Arg

<210> 58
<211> 45
<212> PRT
<213> Mus musculus

<400> 58

Ile Glu Ala Val Lys Ser Glu Pro Val Glu Pro Pro Leu Pro Ser Gln
1               5                   10                  15

Leu His Leu Met Tyr Val Ala Ala Ala Ala Phe Val Leu Leu Phe Phe
            20                  25                  30

Val Gly Cys Gly Val Leu Leu Ser Arg Lys Arg Arg Arg

<210> 59
<211> 1125
<212> PRT
<213> Artificial Sequence

<220>
<223> Full-length NTF release sensor for dmCIRL used in Example 2

<400> 59

Met Lys Leu Cys Ile Leu Leu Ala Val Val Ala Phe Val Gly Leu Ser
1               5                   10                  15

Leu Gly Glu Thr Gly Phe Pro Lys Ser Leu Ser Val Leu Asn Ser Arg
            20                  25                  30

Cys Ala His Lys Gln Ser Cys Gly Val Leu Ala Ala Thr Ser Met Phe
        35                  40                  45

Gly Asp Pro Cys Pro Gly Thr His Lys Tyr Leu Glu Ala His Tyr Gln
    50                  55                  60

Cys Ile Ser Ala Ala Gln Thr Ser Thr Thr Thr Asn Arg Pro Ser Pro
65                  70                  75                  80

Pro Pro Trp Val Leu Ser Asn Gly Pro Pro Ile Phe Gly Asn Gly Ser
            85                  90                  95

Gly Leu Ile His Pro Pro Gly Val Gly Ala Gly Ala Pro Pro Pro Pro
            100                 105                 110

Arg Leu Pro Thr Leu Pro Gly Val Val Gly Ile Ser Gly Asn Pro Gly
        115                 120                 125

Leu Phe Asn Val Pro Pro Gln His Thr Ala Val Thr His Ser Thr Pro
    130                 135                 140

Ser Ser Ser Thr Thr Ala Val Gly Gly Gly Arg Leu Lys Gly Gly Ala
145                 150                 155                 160

Thr Ser Thr Thr Thr Thr Lys His Pro Ala Gly Arg His Asp Gly Leu
            165                 170                 175

Pro Pro Pro Pro Gln Leu His His His His Asn His His Gly Glu Asp
            180                 185                 190

Thr Ala Ser Pro Thr Lys Pro Ser Ser Lys Leu Pro Ala Gly Gly Asn
    195                 200                 205

```
Ala Thr Ser Pro Ser Asn Thr Arg Ile Leu Thr Gly Val Gly Gly Ser
    210                 215                 220

Gly Thr Asp Asp Gly Thr Leu Leu Thr Thr Lys Ser Ser Pro Asn Arg
225                 230                 235                 240

Pro Pro Gly Thr Ala Ala Ser Gly Ser Val Val Pro Gly Asn Gly Ser
                245                 250                 255

Val Val Arg Thr Ile Asn Asn Ile Asn Leu Asn Ala Ala Gly Met Ser
                260                 265                 270

Gly Gly Asp Asp Glu Ser Lys Leu Phe Cys Gly Pro Thr His Ala Arg
                275                 280                 285

Asn Leu Tyr Trp Asn Met Thr Arg Val Gly Asp Val Asn Val Gln Pro
    290                 295                 300

Cys Pro Gly Gly Ala Ala Gly Ile Ala Lys Trp Arg Cys Val Leu Met
305                 310                 315                 320

Lys Arg Ile Pro Asp Ser Gly Tyr Asp Glu Tyr Asp Asp Asp Ile Ser
                325                 330                 335

Ser Thr Thr Pro Ala Pro Ser Gly Gly Asp Cys Leu His Asn Ser Ser
                340                 345                 350

Ser Cys Glu Pro Pro Val Ser Met Ala His Lys Val Asn Gln Arg Leu
                355                 360                 365

Arg Asn Phe Glu Pro Thr Trp His Pro Ala Thr Pro Asp Leu Thr Gln
    370                 375                 380

Cys Arg Ser Leu Trp Leu Asn Asn Leu Glu Met Arg Val Asn Gln Arg
385                 390                 395                 400

Asp Ser Ser Leu Ile Ser Ile Ala Asn Asp Met Ser Glu Val Thr Ser
                405                 410                 415

Ser Lys Thr Leu Tyr Gly Gly Asp Met Leu Val Thr Thr Lys Ile Ile
                420                 425                 430

Gln Thr Val Ser Glu Lys Met Met His Asp Lys Glu Thr Phe Pro Asp
                435                 440                 445

Gln Arg Gln Arg Glu Ala Met Ile Met Glu Leu Leu His Cys Val Val
```

```
        450                    455                    460


Lys Thr Gly Ser Asn Leu Leu Asp Glu Ser Gln Leu Ser Ser Trp Leu
465             470             475             480


Asp Leu Asn Pro Glu Asp Gln Met Arg Val Ala Thr Ser Leu Leu Thr
            485             490             495


Gly Leu Glu Tyr Asn Ala Phe Leu Leu Ala Asp Thr Ile Ile Arg Glu
            500             505             510


Arg Ser Val Val Gln Lys Val Lys Asn Ile Leu Leu Ser Val Arg Val
            515             520             525


Leu Glu Thr Lys Thr Ile Gln Ser Ser Val Val Phe Pro Asp Ser Asp
            530             535             540


Gln Trp Pro Leu Ser Ser Asp Arg Ile Glu Leu Pro Arg Ala Ala Leu
545             550             555             560


Ile Asp Asn Ser Glu Gly Gly Leu Val Arg Ile Val Phe Ala Ala Phe
                565             570             575


Asp Arg Leu Glu Ser Ile Leu Lys Pro Ser Tyr Asp His Phe Asp Leu
            580             585             590


Lys Ser Ser Arg Ser Tyr Ala Ile Leu Ser Asn Asp Ser Asp Val Asn
            595             600             605


Ala Gly Glu Ile Gln Gln Arg Leu Arg Ile Leu Asn Ser Lys Val Ile
            610             615             620


Ser Ala Ser Leu Gly Lys Gly Arg His Ile Gln Leu Ser Gln Pro Ile
625             630             635             640


Thr Leu Thr Leu Lys His Leu Lys Thr Glu Asn Val Thr Asn Pro Thr
                645             650             655


Cys Val Phe Trp Asn Tyr Ile Asp His Ala Trp Ser Ala Asn Gly Cys
                660             665             670


Ser Leu Glu Ser Thr Asn Arg Thr His Ser Val Cys Ser Cys Asn His
            675             680             685


Leu Thr Asn Phe Ala Ile Leu Met Asp Val Val Asp Glu His Gln His
            690             695             700
```

Ser Leu Phe Thr Met Phe Asp Gly Asn Met Arg Pro Arg Ile Asp Thr
705             710             715             720

Ala Ala Lys His Gln Leu Arg Asn Asp Phe Gln Ile His Ser Val Arg
        725             730             735

Gly Ile Lys Asn Pro Gly Asp Glu Asp Asn Gly Glu Pro Pro Ala Asn
        740             745             750

Val Lys Tyr Val Ile Thr Gly Ile Ile Leu Val Ile Ile Ala Leu Ala
        755             760             765

Phe Phe Gly Met Val Leu Ser Thr Gln Arg Lys Arg Ser Gly Pro Pro
    770             775             780

Lys Leu Lys Ala Leu Thr Ala Arg Gln Gln Glu Val Phe Asp Leu Ile
785             790             795             800

Arg Asp His Ile Ser Gln Thr Gly Met Pro Pro Thr Arg Ala Glu Ile
            805             810             815

Ala Gln Arg Leu Gly Phe Arg Ser Pro Asn Ala Ala Glu Glu His Leu
        820             825             830

Lys Ala Leu Ala Arg Lys Gly Val Ile Glu Ile Val Ser Gly Ala Ser
        835             840             845

Arg Gly Ile Arg Leu Leu Gln Glu Glu Glu Glu Gly Leu Pro Leu Val
    850             855             860

Gly Arg Val Ala Ala Gly Glu Pro Leu Leu Ala Gln Gln His Ile Glu
865             870             875             880

Gly His Tyr Gln Val Asp Pro Ser Leu Phe Lys Pro Asn Ala Asp Phe
            885             890             895

Leu Leu Arg Val Ser Gly Met Ser Met Lys Asp Ile Gly Ile Met Asp
        900             905             910

Gly Asp Leu Leu Ala Val His Lys Thr Gln Asp Val Arg Asn Gly Gln
        915             920             925

Val Val Val Ala Arg Ile Asp Asp Glu Val Thr Val Lys Arg Leu Lys
    930             935             940

Lys Gln Gly Asn Lys Val Glu Leu Leu Pro Glu Asn Ser Glu Phe Lys
945             950             955             960

```
Pro Ile Val Val Asp Leu Arg Gln Gln Ser Phe Thr Ile Glu Gly Leu
              965                 970                 975

Ala Val Gly Val Ile Arg Asn Gly Asp Trp Leu Glu Phe Pro Gly Ile
              980                 985                 990

Arg Arg Pro Ala Gly Ile Pro Gly Asp Leu Ala Pro Pro Thr Asp Val
         995              1000                1005

Ser Leu Gly Asp Glu Leu His Leu Asp Gly Glu Asp Val Ala Met
    1010              1015                1020

Ala His Ala Asp Ala Leu Asp Asp Phe Asp Leu Asp Met Leu Gly
    1025              1030                1035

Asp Gly Asp Ser Pro Gly Pro Gly Phe Thr Pro His Asp Ser Ala
    1040              1045                1050

Pro Tyr Gly Ala Leu Asp Met Ala Asp Phe Glu Phe Glu Gln Met
    1055              1060                1065

Phe Thr Asp Ala Leu Gly Ile Asp Glu Tyr Gly Gly Ala Ser Arg
    1070              1075                1080

Tyr Leu Asp Pro Ala Phe Leu Tyr Lys Val Val Ser Ser Ala Thr
    1085              1090                1095

Met Asp Tyr Lys Asp His Asp Gly Asp Tyr Lys Asp His Asp Ile
    1100              1105                1110

Asp Tyr Lys Asp Asp Asp Asp Lys His Arg Ser Thr
    1115              1120                1125
```

```
<210>   60
<211>   1125
<212>   PRT
<213>   Artificial Sequence

<220>
<223>   Full-length NTF release sensor for dmCIRL (GPS cleavage site
        mutated)

<400>   60
```

```
Met Lys Leu Cys Ile Leu Leu Ala Val Val Ala Phe Val Gly Leu Ser
1               5                   10                  15

Leu Gly Glu Thr Gly Phe Pro Lys Ser Leu Ser Val Leu Asn Ser Arg
              20                  25                  30
```

```
Cys Ala His Lys Gln Ser Cys Gly Val Leu Ala Ala Thr Ser Met Phe
        35                  40                  45

Gly Asp Pro Cys Pro Gly Thr His Lys Tyr Leu Glu Ala His Tyr Gln
        50                  55                  60

Cys Ile Ser Ala Ala Gln Thr Ser Thr Thr Thr Asn Arg Pro Ser Pro
65                  70                  75                  80

Pro Pro Trp Val Leu Ser Asn Gly Pro Pro Ile Phe Gly Asn Gly Ser
            85                  90                  95

Gly Leu Ile His Pro Pro Gly Val Gly Ala Gly Ala Pro Pro Pro Pro
            100                 105                 110

Arg Leu Pro Thr Leu Pro Gly Val Val Gly Ile Ser Gly Asn Pro Gly
        115                 120                 125

Leu Phe Asn Val Pro Pro Gln His Thr Ala Val Thr His Ser Thr Pro
        130                 135                 140

Ser Ser Ser Thr Thr Ala Val Gly Gly Gly Arg Leu Lys Gly Gly Ala
145                 150                 155                 160

Thr Ser Thr Thr Thr Thr Lys His Pro Ala Gly Arg His Asp Gly Leu
                165                 170                 175

Pro Pro Pro Pro Gln Leu His His His His Asn His His Gly Glu Asp
            180                 185                 190

Thr Ala Ser Pro Thr Lys Pro Ser Ser Lys Leu Pro Ala Gly Gly Asn
        195                 200                 205

Ala Thr Ser Pro Ser Asn Thr Arg Ile Leu Thr Gly Val Gly Gly Ser
        210                 215                 220

Gly Thr Asp Asp Gly Thr Leu Leu Thr Thr Lys Ser Ser Pro Asn Arg
225                 230                 235                 240

Pro Pro Gly Thr Ala Ala Ser Gly Ser Val Val Pro Gly Asn Gly Ser
            245                 250                 255

Val Val Arg Thr Ile Asn Asn Ile Asn Leu Asn Ala Ala Gly Met Ser
            260                 265                 270

Gly Gly Asp Asp Glu Ser Lys Leu Phe Cys Gly Pro Thr His Ala Arg
            275                 280                 285
```

82

Asn Leu Tyr Trp Asn Met Thr Arg Val Gly Asp Val Asn Val Gln Pro
290 295 300

Cys Pro Gly Gly Ala Ala Gly Ile Ala Lys Trp Arg Cys Val Leu Met
305 310 315 320

Lys Arg Ile Pro Asp Ser Gly Tyr Asp Glu Tyr Asp Asp Asp Ile Ser
325 330 335

Ser Thr Thr Pro Ala Pro Ser Gly Gly Asp Cys Leu His Asn Ser Ser
340 345 350

Ser Cys Glu Pro Pro Val Ser Met Ala His Lys Val Asn Gln Arg Leu
355 360 365

Arg Asn Phe Glu Pro Thr Trp His Pro Ala Thr Pro Asp Leu Thr Gln
370 375 380

Cys Arg Ser Leu Trp Leu Asn Asn Leu Glu Met Arg Val Asn Gln Arg
385 390 395 400

Asp Ser Ser Leu Ile Ser Ile Ala Asn Asp Met Ser Glu Val Thr Ser
405 410 415

Ser Lys Thr Leu Tyr Gly Gly Asp Met Leu Val Thr Thr Lys Ile Ile
420 425 430

Gln Thr Val Ser Glu Lys Met Met His Asp Lys Glu Thr Phe Pro Asp
435 440 445

Gln Arg Gln Arg Glu Ala Met Ile Met Glu Leu Leu His Cys Val Val
450 455 460

Lys Thr Gly Ser Asn Leu Leu Asp Glu Ser Gln Leu Ser Ser Trp Leu
465 470 475 480

Asp Leu Asn Pro Glu Asp Gln Met Arg Val Ala Thr Ser Leu Leu Thr
485 490 495

Gly Leu Glu Tyr Asn Ala Phe Leu Leu Ala Asp Thr Ile Ile Arg Glu
500 505 510

Arg Ser Val Val Gln Lys Val Lys Asn Ile Leu Leu Ser Val Arg Val
515 520 525

Leu Glu Thr Lys Thr Ile Gln Ser Ser Val Val Phe Pro Asp Ser Asp

```
            530                        535                          540


    Gln Trp Pro Leu Ser Ser Asp Arg Ile Glu Leu Pro Arg Ala Ala Leu
    545             550             555             560


    Ile Asp Asn Ser Glu Gly Gly Leu Val Arg Ile Val Phe Ala Ala Phe
                    565             570             575


    Asp Arg Leu Glu Ser Ile Leu Lys Pro Ser Tyr Asp His Phe Asp Leu
                580             585             590


    Lys Ser Ser Arg Ser Tyr Ala Ile Leu Ser Asn Asp Ser Asp Val Asn
            595             600             605


    Ala Gly Glu Ile Gln Gln Arg Leu Arg Ile Leu Asn Ser Lys Val Ile
        610             615             620


    Ser Ala Ser Leu Gly Lys Gly Arg His Ile Gln Leu Ser Gln Pro Ile
    625             630             635             640


    Thr Leu Thr Leu Lys His Leu Lys Thr Glu Asn Val Thr Asn Pro Thr
                645             650             655


    Cys Val Phe Trp Asn Tyr Ile Asp His Ala Trp Ser Ala Asn Gly Cys
                660             665             670


    Ser Leu Glu Ser Thr Asn Arg Thr His Ser Val Cys Ser Cys Asn Ala
                675             680             685


    Leu Thr Asn Phe Ala Ile Leu Met Asp Val Val Asp Glu His Gln His
                690             695             700


    Ser Leu Phe Thr Met Phe Asp Gly Asn Met Arg Pro Arg Ile Asp Thr
    705             710             715             720


    Ala Ala Lys His Gln Leu Arg Asn Asp Phe Gln Ile His Ser Val Arg
                725             730             735


    Gly Ile Lys Asn Pro Gly Asp Glu Asp Asn Gly Glu Pro Pro Ala Asn
                740             745             750


    Val Lys Tyr Val Ile Thr Gly Ile Ile Leu Val Ile Ile Ala Leu Ala
                755             760             765


    Phe Phe Gly Met Val Leu Ser Thr Gln Arg Lys Arg Ser Gly Pro Pro
                770             775             780
```

Lys Leu Lys Ala Leu Thr Ala Arg Gln Gln Glu Val Phe Asp Leu Ile
785                     790             795             800

Arg Asp His Ile Ser Gln Thr Gly Met Pro Pro Thr Arg Ala Glu Ile
                805             810             815

Ala Gln Arg Leu Gly Phe Arg Ser Pro Asn Ala Ala Glu Glu His Leu
            820             825             830

Lys Ala Leu Ala Arg Lys Gly Val Ile Glu Ile Val Ser Gly Ala Ser
        835             840             845

Arg Gly Ile Arg Leu Leu Gln Glu Glu Glu Glu Gly Leu Pro Leu Val
    850             855             860

Gly Arg Val Ala Ala Gly Glu Pro Leu Leu Ala Gln Gln His Ile Glu
865             870             875             880

Gly His Tyr Gln Val Asp Pro Ser Leu Phe Lys Pro Asn Ala Asp Phe
            885             890             895

Leu Leu Arg Val Ser Gly Met Ser Met Lys Asp Ile Gly Ile Met Asp
            900             905             910

Gly Asp Leu Leu Ala Val His Lys Thr Gln Asp Val Arg Asn Gly Gln
        915             920             925

Val Val Val Ala Arg Ile Asp Asp Glu Val Thr Val Lys Arg Leu Lys
    930             935             940

Lys Gln Gly Asn Lys Val Glu Leu Leu Pro Glu Asn Ser Glu Phe Lys
945             950             955             960

Pro Ile Val Val Asp Leu Arg Gln Gln Ser Phe Thr Ile Glu Gly Leu
            965             970             975

Ala Val Gly Val Ile Arg Asn Gly Asp Trp Leu Glu Phe Pro Gly Ile
            980             985             990

Arg Arg Pro Ala Gly Ile Pro Gly Asp Leu Ala Pro Pro Thr Asp Val
        995             1000            1005

Ser Leu Gly Asp Glu Leu His Leu Asp Gly Glu Asp Val Ala Met
    1010            1015            1020

Ala His Ala Asp Ala Leu Asp Asp Phe Asp Leu Asp Met Leu Gly
    1025            1030            1035

```
Asp Gly  Asp Ser Pro Gly Pro  Gly Phe Thr Pro His  Asp Ser Ala
    1040             1045             1050


Pro Tyr  Gly Ala Leu Asp Met  Ala Asp Phe Glu Phe  Glu Gln Met
    1055             1060             1065


Phe Thr  Asp Ala Leu Gly Ile  Asp Glu Tyr Gly Gly  Ala Ser Arg
    1070             1075             1080


Tyr Leu  Asp Pro Ala Phe Leu  Tyr Lys Val Val Ser  Ser Ala Thr
    1085             1090             1095


Met Asp  Tyr Lys Asp His Asp  Gly Asp Tyr Lys Asp  His Asp Ile
    1100             1105             1110


Asp Tyr  Lys Asp Asp Asp Asp  Lys His Arg Ser Thr
    1115             1120             1125
```

```
<210>  61
<211>  1125
<212>  PRT
<213>  Artificial Sequence

<220>
<223>  Full-length NTF release sensor for dmCIRL (S3 cleavage site
       mutated)

<400>  61
```

```
Met Lys Leu Cys Ile Leu Leu Ala Val Val Ala Phe Val Gly Leu Ser
1               5               10              15


Leu Gly Glu Thr Gly Phe Pro Lys Ser Leu Ser Val Leu Asn Ser Arg
            20              25              30


Cys Ala His Lys Gln Ser Cys Gly Val Leu Ala Ala Thr Ser Met Phe
        35              40              45


Gly Asp Pro Cys Pro Gly Thr His Lys Tyr Leu Glu Ala His Tyr Gln
    50              55              60


Cys Ile Ser Ala Ala Gln Thr Ser Thr Thr Asn Arg Pro Ser Pro
65              70              75              80


Pro Pro Trp Val Leu Ser Asn Gly Pro Pro Ile Phe Gly Asn Gly Ser
            85              90              95


Gly Leu Ile His Pro Pro Gly Val Gly Ala Gly Ala Pro Pro Pro Pro
        100             105             110
```

Arg Leu Pro Thr Leu Pro Gly Val Val Gly Ile Ser Gly Asn Pro Gly
115                     120             125

Leu Phe Asn Val Pro Pro Gln His Thr Ala Val Thr His Ser Thr Pro
130                     135             140

Ser Ser Ser Thr Thr Ala Val Gly Gly Gly Arg Leu Lys Gly Gly Ala
145                 150             155                 160

Thr Ser Thr Thr Thr Thr Lys His Pro Ala Gly Arg His Asp Gly Leu
                165             170             175

Pro Pro Pro Pro Gln Leu His His His His Asn His His Gly Glu Asp
                180             185             190

Thr Ala Ser Pro Thr Lys Pro Ser Ser Lys Leu Pro Ala Gly Gly Asn
            195             200             205

Ala Thr Ser Pro Ser Asn Thr Arg Ile Leu Thr Gly Val Gly Gly Ser
    210             215             220

Gly Thr Asp Asp Gly Thr Leu Leu Thr Thr Lys Ser Ser Pro Asn Arg
225             230             235                 240

Pro Pro Gly Thr Ala Ala Ser Gly Ser Val Val Pro Gly Asn Gly Ser
            245             250             255

Val Val Arg Thr Ile Asn Asn Ile Asn Leu Asn Ala Ala Gly Met Ser
            260             265             270

Gly Gly Asp Asp Glu Ser Lys Leu Phe Cys Gly Pro Thr His Ala Arg
            275             280             285

Asn Leu Tyr Trp Asn Met Thr Arg Val Gly Asp Val Asn Val Gln Pro
    290             295             300

Cys Pro Gly Gly Ala Ala Gly Ile Ala Lys Trp Arg Cys Val Leu Met
305             310             315                 320

Lys Arg Ile Pro Asp Ser Gly Tyr Asp Glu Tyr Asp Asp Asp Ile Ser
            325             330             335

Ser Thr Thr Pro Ala Pro Ser Gly Gly Asp Cys Leu His Asn Ser Ser
        340             345             350

Ser Cys Glu Pro Pro Val Ser Met Ala His Lys Val Asn Gln Arg Leu
    355             360             365

```
Arg Asn Phe Glu Pro Thr Trp His Pro Ala Thr Pro Asp Leu Thr Gln
    370             375             380

Cys Arg Ser Leu Trp Leu Asn Asn Leu Glu Met Arg Val Asn Gln Arg
385             390             395             400

Asp Ser Ser Leu Ile Ser Ile Ala Asn Asp Met Ser Glu Val Thr Ser
            405             410             415

Ser Lys Thr Leu Tyr Gly Gly Asp Met Leu Val Thr Thr Lys Ile Ile
            420             425             430

Gln Thr Val Ser Glu Lys Met Met His Asp Lys Glu Thr Phe Pro Asp
        435             440             445

Gln Arg Gln Arg Glu Ala Met Ile Met Glu Leu Leu His Cys Val Val
    450             455             460

Lys Thr Gly Ser Asn Leu Leu Asp Glu Ser Gln Leu Ser Ser Trp Leu
465             470             475             480

Asp Leu Asn Pro Glu Asp Gln Met Arg Val Ala Thr Ser Leu Leu Thr
            485             490             495

Gly Leu Glu Tyr Asn Ala Phe Leu Leu Ala Asp Thr Ile Ile Arg Glu
            500             505             510

Arg Ser Val Val Gln Lys Val Lys Asn Ile Leu Leu Ser Val Arg Val
        515             520             525

Leu Glu Thr Lys Thr Ile Gln Ser Ser Val Val Phe Pro Asp Ser Asp
    530             535             540

Gln Trp Pro Leu Ser Ser Asp Arg Ile Glu Leu Pro Arg Ala Ala Leu
545             550             555             560

Ile Asp Asn Ser Glu Gly Gly Leu Val Arg Ile Val Phe Ala Ala Phe
            565             570             575

Asp Arg Leu Glu Ser Ile Leu Lys Pro Ser Tyr Asp His Phe Asp Leu
        580             585             590

Lys Ser Ser Arg Ser Tyr Ala Ile Leu Ser Asn Asp Ser Asp Val Asn
    595             600             605

Ala Gly Glu Ile Gln Gln Arg Leu Arg Ile Leu Asn Ser Lys Val Ile
```

|     | 610 |     |     |     |     | 615 |     |     |     |     | 620 |     |     |     |
|-----|-----|-----|-----|-----|-----|-----|-----|-----|-----|-----|-----|-----|-----|-----|

Ser Ala Ser Leu Gly Lys Gly Arg His Ile Gln Leu Ser Gln Pro Ile
625               630              635              640

Thr Leu Thr Leu Lys His Leu Lys Thr Glu Asn Val Thr Asn Pro Thr
               645              650              655

Cys Val Phe Trp Asn Tyr Ile Asp His Ala Trp Ser Ala Asn Gly Cys
               660              665              670

Ser Leu Glu Ser Thr Asn Arg Thr His Ser Val Cys Ser Cys Asn His
               675              680              685

Leu Thr Asn Phe Ala Ile Leu Met Asp Val Val Asp Glu His Gln His
               690              695              700

Ser Leu Phe Thr Met Phe Asp Gly Asn Met Arg Pro Arg Ile Asp Thr
705               710              715              720

Ala Ala Lys His Gln Leu Arg Asn Asp Phe Gln Ile His Ser Val Arg
               725              730              735

Gly Ile Lys Asn Pro Gly Asp Glu Asp Asn Gly Glu Pro Pro Ala Asn
               740              745              750

Val Lys Tyr Val Ile Thr Gly Ile Ile Leu Val Ile Ile Ala Leu Ala
               755              760              765

Phe Phe Gly Met Lys Leu Ser Thr Gln Arg Lys Arg Ser Gly Pro Pro
               770              775              780

Lys Leu Lys Ala Leu Thr Ala Arg Gln Gln Glu Val Phe Asp Leu Ile
785               790              795              800

Arg Asp His Ile Ser Gln Thr Gly Met Pro Pro Thr Arg Ala Glu Ile
               805              810              815

Ala Gln Arg Leu Gly Phe Arg Ser Pro Asn Ala Ala Glu Glu His Leu
               820              825              830

Lys Ala Leu Ala Arg Lys Gly Val Ile Glu Ile Val Ser Gly Ala Ser
               835              840              845

Arg Gly Ile Arg Leu Leu Gln Glu Glu Glu Glu Gly Leu Pro Leu Val
850               855              860

```
Gly Arg Val Ala Ala Gly Glu Pro Leu Leu Ala Gln Gln His Ile Glu
865             870             875                     880

Gly His Tyr Gln Val Asp Pro Ser Leu Phe Lys Pro Asn Ala Asp Phe
            885             890                 895

Leu Leu Arg Val Ser Gly Met Ser Met Lys Asp Ile Gly Ile Met Asp
            900             905                 910

Gly Asp Leu Leu Ala Val His Lys Thr Gln Asp Val Arg Asn Gly Gln
        915             920                 925

Val Val Val Ala Arg Ile Asp Asp Glu Val Thr Val Lys Arg Leu Lys
    930             935                 940

Lys Gln Gly Asn Lys Val Glu Leu Leu Pro Glu Asn Ser Glu Phe Lys
945             950             955                     960

Pro Ile Val Val Asp Leu Arg Gln Gln Ser Phe Thr Ile Glu Gly Leu
            965             970                 975

Ala Val Gly Val Ile Arg Asn Gly Asp Trp Leu Glu Phe Pro Gly Ile
            980             985                 990

Arg Arg Pro Ala Gly Ile Pro Gly  Asp Leu Ala Pro Pro  Thr Asp Val
        995             1000                1005

Ser Leu  Gly Asp Glu Leu His  Leu Asp Gly Glu Asp  Val Ala Met
    1010                1015                1020

Ala His  Ala Asp Ala Leu Asp  Asp Phe Asp Leu Asp  Met Leu Gly
    1025                1030                1035

Asp Gly  Asp Ser Pro Gly Pro  Gly Phe Thr Pro His  Asp Ser Ala
    1040                1045                1050

Pro Tyr  Gly Ala Leu Asp Met  Ala Asp Phe Glu Phe  Glu Gln Met
    1055                1060                1065

Phe Thr  Asp Ala Leu Gly Ile  Asp Glu Tyr Gly Gly  Ala Ser Arg
    1070                1075                1080

Tyr Leu  Asp Pro Ala Phe Leu  Tyr Lys Val Val Ser  Ser Ala Thr
    1085                1090                1095

Met Asp  Tyr Lys Asp His Asp  Gly Asp Tyr Lys Asp  His Asp Ile
    1100                1105                1110
```

90

```
Asp Tyr  Lys Asp Asp Asp Asp  Lys His Arg Ser Thr
    1115                1120                1125


<210>   62
<211>   432
<212>   PRT
<213>   Artificial Sequence

<220>
<223>   NTF release sensor positive control

<400>   62

Met Gln Ser Gln Arg Ser Arg Arg Arg Ser Arg Ala Pro Asn Thr Trp
1               5               10              15


Ile Cys Phe Trp Ile Asn Lys Met His Ala Val Ala Ser Leu Pro Ala
            20              25              30


Ser Leu Pro Leu Leu Leu Leu Thr Leu Ala Phe Ala Asn Leu Pro Asn
        35              40              45


Thr Val Arg Gly Thr Asp Thr Ala Leu Val Ala Ala Ser Cys Thr Ser
    50              55              60


Val Gly Cys Gln Asn Ile Asp Thr Ala Ala Lys His Gln Leu Arg Asn
65              70              75              80


Asp Phe Gln Ile His Ser Val Arg Gly Ile Lys Asn Pro Gly Asp Glu
            85              90              95


Asp Asn Gly Glu Pro Pro Ala Asn Val Lys Tyr Val Ile Thr Gly Ile
            100             105             110


Ile Leu Val Ile Ile Ala Leu Ala Phe Phe Gly Met Val Leu Ser Thr
        115             120             125


Gln Arg Lys Arg Ser Gly Pro Pro Lys Leu Lys Ala Leu Thr Ala Arg
        130             135             140


Gln Gln Glu Val Phe Asp Leu Ile Arg Asp His Ile Ser Gln Thr Gly
145             150             155             160


Met Pro Pro Thr Arg Ala Glu Ile Ala Gln Arg Leu Gly Phe Arg Ser
                165             170             175


Pro Asn Ala Ala Glu Glu His Leu Lys Ala Leu Ala Arg Lys Gly Val
            180             185             190
```

```
Ile Glu Ile Val Ser Gly Ala Ser Arg Gly Ile Arg Leu Leu Gln Glu
        195                 200             205

Glu Glu Glu Gly Leu Pro Leu Val Gly Arg Val Ala Ala Gly Glu Pro
    210                 215                 220

Leu Leu Ala Gln Gln His Ile Glu Gly His Tyr Gln Val Asp Pro Ser
225                 230                 235                 240

Leu Phe Lys Pro Asn Ala Asp Phe Leu Leu Arg Val Ser Gly Met Ser
                245                 250                 255

Met Lys Asp Ile Gly Ile Met Asp Gly Asp Leu Leu Ala Val His Lys
                260                 265                 270

Thr Gln Asp Val Arg Asn Gly Gln Val Val Val Ala Arg Ile Asp Asp
        275                 280                 285

Glu Val Thr Val Lys Arg Leu Lys Lys Gln Gly Asn Lys Val Glu Leu
    290                 295                 300

Leu Pro Glu Asn Ser Glu Phe Lys Pro Ile Val Val Asp Leu Arg Gln
305                 310                 315                 320

Gln Ser Phe Thr Ile Glu Gly Leu Ala Val Gly Val Ile Arg Asn Gly
                325                 330                 335

Asp Trp Leu Glu Phe Pro Gly Ile Arg Arg Pro Ala Gly Ile Pro Gly
        340                 345                 350

Asp Leu Ala Pro Pro Thr Asp Val Ser Leu Gly Asp Glu Leu His Leu
        355                 360                 365

Asp Gly Glu Asp Val Ala Met Ala His Ala Asp Ala Leu Asp Asp Phe
    370                 375                 380

Asp Leu Asp Met Leu Gly Asp Gly Asp Ser Pro Gly Pro Gly Phe Thr
385                 390                 395                 400

Pro His Asp Ser Ala Pro Tyr Gly Ala Leu Asp Met Ala Asp Phe Glu
                405                 410                 415

Phe Glu Gln Met Phe Thr Asp Ala Leu Gly Ile Asp Glu Tyr Gly Gly
        420                 425                 430
```

```
<210>   63
<211>   1187
<212>   PRT
```

<213>    Artificial Sequence

<220>
<223>    NTF release sensor negative control

<400>    63

Met Gln Ser Gln Arg Ser Arg Arg Arg Ser Arg Ala Pro Asn Thr Trp
1               5                   10                  15

Ile Cys Phe Trp Ile Asn Lys Met His Ala Val Ala Ser Leu Pro Ala
            20                  25                  30

Ser Leu Pro Leu Leu Leu Leu Thr Leu Ala Phe Ala Asn Leu Pro Asn
            35                  40                  45

Thr Val Arg Gly Thr Asp Thr Ala Leu Val Ala Ala Ser Cys Thr Ser
        50                  55                  60

Val Gly Cys Gln Asn Gly Gly Thr Cys Val Thr Gln Leu Asn Gly Lys
65                  70                  75                  80

Thr Tyr Cys Ala Cys Asp Ser His Tyr Val Gly Asp Tyr Cys Glu His
            85                  90                  95

Arg Asn Pro Cys Asn Ser Met Arg Cys Gln Asn Gly Gly Thr Cys Gln
            100                 105                 110

Val Thr Phe Arg Asn Gly His Pro Gly Ile Ser Cys Lys Cys Pro Leu
            115                 120                 125

Gly Phe Asp Glu Ser Leu Cys Glu Ile Ala Val Pro Glu Asp Cys Thr
            130                 135                 140

Glu Ser Ser Cys Leu Asn Gly Gly Ser Cys Ile Asp Gly Ile Asn Gly
145                 150                 155                 160

Tyr Asn Cys Ser Cys Leu Ala Gly Tyr Ser Gly Ala Asn Cys Gln Tyr
            165                 170                 175

Lys Leu Asn Lys Cys Asp Ser Asn Pro Cys Leu Asn Gly Ala Thr Cys
            180                 185                 190

His Glu Gln Asn Asn Glu Tyr Thr Cys His Cys Pro Ser Gly Phe Thr
            195                 200                 205

Gly Lys Gln Cys Ser Glu Tyr Val Asp Trp Cys Gly Gln Ser Pro Cys
        210                 215                 220

```
Glu Asn Gly Ala Thr Cys Ser Gln Met Lys His Gln Phe Ser Cys Lys
225             230             235             240

Cys Ser Ala Gly Trp Thr Gly Lys Leu Cys Asp Val Gln Thr Ile Ser
            245             250             255

Cys Gln Asp Ala Ala Asp Arg Lys Gly Leu Ser Leu Arg Gln Leu Cys
        260             265             270

Asn Asn Gly Thr Cys Lys Asp Tyr Gly Asn Ser His Val Cys Tyr Cys
        275             280             285

Ser Gln Gly Tyr Ala Gly Ser Tyr Cys Gln Lys Glu Ile Asp Glu Cys
        290             295             300

Gln Ser Gln Pro Cys Gln Asn Gly Gly Thr Cys Arg Asp Leu Ile Gly
305             310             315             320

Ala Tyr Glu Cys Gln Cys Arg Gln Gly Phe Gln Gly Gln Asn Cys Glu
            325             330             335

Leu Asn Ile Asp Asp Cys Ala Pro Asn Pro Cys Gln Asn Gly Gly Thr
        340             345             350

Cys His His Arg Val Met Asn Phe Ser Cys Ser Cys Pro Pro Gly Thr
        355             360             365

Met Gly Ile Ile Cys Glu Ile Asn Lys Asp Asp Cys Lys Pro Gly Ala
    370             375             380

Cys His Asn Asn Gly Ser Cys Ile Asp Arg Val Gly Gly Phe Glu Cys
385             390             395             400

Val Cys Gln Pro Gly Phe Val Gly Ala Arg Cys Glu Gly Asp Ile Asn
            405             410             415

Glu Cys Leu Ser Asn Pro Cys Ser Asn Ala Gly Thr Leu Asp Cys Val
        420             425             430

Gln Leu Val Asn Asn Tyr His Cys Asn Cys Arg Pro Gly His Met Gly
        435             440             445

Arg His Cys Glu His Lys Val Asp Phe Cys Ala Gln Ser Pro Cys Gln
        450             455             460

Asn Gly Gly Asn Cys Asn Ile Arg Gln Ser Gly His His Cys Ile Cys
465             470             475             480
```

94

Asn Asn Gly Phe Tyr Gly Lys Asn Cys Glu Leu Ser Gly Gln Asp Cys
        485             490             495

Asp Ser Asn Pro Cys Arg Val Gly Asn Cys Val Val Ala Asp Glu Gly
        500             505             510

Phe Gly Tyr Arg Cys Glu Cys Pro Arg Gly Thr Leu Gly Glu His Cys
        515             520             525

Glu Ile Asp Thr Leu Asp Glu Cys Ser Pro Asn Pro Cys Ala Gln Gly
        530             535             540

Ala Ala Cys Glu Asp Leu Leu Gly Asp Tyr Glu Cys Leu Cys Pro Ser
545             550             555             560

Lys Trp Lys Gly Lys Arg Cys Asp Ile Tyr Asp Ala Asn Tyr Pro Gly
        565             570             575

Trp Asn Gly Gly Ser Gly Ser Gly Asn Asp Arg Tyr Ala Ala Asp Leu
        580             585             590

Glu Gln Gln Arg Ala Met Cys Asp Lys Arg Gly Cys Thr Glu Lys Gln
        595             600             605

Gly Asn Gly Ile Cys Asp Ser Asp Cys Asn Thr Tyr Ala Cys Asn Phe
        610             615             620

Asp Gly Asn Asp Cys Ser Leu Gly Ile Asn Pro Trp Ala Asn Cys Thr
625             630             635             640

Ala Asn Glu Cys Trp Asn Lys Phe Lys Asn Gly Lys Cys Asn Glu Glu
        645             650             655

Cys Asn Asn Ala Ala Cys His Tyr Asp Gly His Asp Cys Glu Arg Lys
        660             665             670

Leu Lys Ser Cys Asp Thr Leu Phe Asp Ala Tyr Cys Gln Lys His Tyr
        675             680             685

Gly Asp Gly Phe Cys Asp Tyr Gly Cys Asn Asn Ala Glu Cys Ser Trp
        690             695             700

Asp Gly Leu Asp Cys Glu Asn Lys Thr Gln Ser Pro Val Leu Ala Glu
705             710             715             720

Gly Ala Met Ser Val Val Met Leu Met Asn Val Glu Ala Phe Arg Glu
        725             730             735

95

```
Ile Gln Ala Gln Phe Leu Arg Asn Met Ser His Met Leu Arg Thr Thr
            740                 745             750

Val Arg Leu Lys Lys Asp Ala Leu Gly His Asp Ile Ile Ile Asn Trp
            755             760             765

Lys Asp Asn Val Arg Val Pro Glu Ile Glu Asp Thr Asp Phe Ala Arg
        770             775             780

Lys Asn Lys Ile Leu Tyr Thr Gln Gln Val His Gln Thr Gly Ile Gln
785             790             795             800

Ile Tyr Leu Glu Ile Asp Asn Arg Lys Cys Thr Glu Cys Phe Thr His
                805             810             815

Ala Val Glu Ala Ala Glu Phe Leu Ala Ala Thr Ala Ala Lys His Gln
            820             825             830

Leu Arg Asn Asp Phe Gln Ile His Ser Val Arg Gly Ile Lys Asn Pro
            835             840             845

Gly Asp Glu Asp Asn Gly Glu Pro Pro Ala Asn Val Lys Tyr Val Ile
    850             855             860

Thr Gly Ile Ile Leu Val Ile Ile Ala Leu Ala Phe Phe Gly Met Val
865             870             875             880

Leu Ser Thr Gln Arg Lys Arg Ser Gly Pro Pro Lys Leu Lys Ala Leu
            885             890             895

Thr Ala Arg Gln Gln Glu Val Phe Asp Leu Ile Arg Asp His Ile Ser
            900             905             910

Gln Thr Gly Met Pro Pro Thr Arg Ala Glu Ile Ala Gln Arg Leu Gly
            915             920             925

Phe Arg Ser Pro Asn Ala Ala Glu Glu His Leu Lys Ala Leu Ala Arg
            930             935             940

Lys Gly Val Ile Glu Ile Val Ser Gly Ala Ser Arg Gly Ile Arg Leu
945             950             955             960

Leu Gln Glu Glu Glu Glu Gly Leu Pro Leu Val Gly Arg Val Ala Ala
                965             970             975

Gly Glu Pro Leu Leu Ala Gln Gln His Ile Glu Gly His Tyr Gln Val
```

```
              980                    985                         990

         Asp Pro Ser Leu Phe Lys Pro Asn  Ala Asp Phe Leu Leu  Arg Val Ser
             995                1000                1005

         Gly Met  Ser Met Lys Asp Ile  Gly Ile Met Asp Gly  Asp Leu Leu
             1010                1015                1020

         Ala Val  His Lys Thr Gln Asp  Val Arg Asn Gly Gln  Val Val Val
             1025                1030                1035

         Ala Arg  Ile Asp Asp Glu Val  Thr Val Lys Arg Leu  Lys Lys Gln
             1040                1045                1050

         Gly Asn  Lys Val Glu Leu Leu  Pro Glu Asn Ser Glu  Phe Lys Pro
             1055                1060                1065

         Ile Val  Val Asp Leu Arg Gln  Gln Ser Phe Thr Ile  Glu Gly Leu
             1070                1075                1080

         Ala Val  Gly Val Ile Arg Asn  Gly Asp Trp Leu Glu  Phe Pro Gly
             1085                1090                1095

         Ile Arg  Arg Pro Ala Gly Ile  Pro Gly Asp Leu Ala  Pro Pro Thr
             1100                1105                1110

         Asp Val  Ser Leu Gly Asp Glu  Leu His Leu Asp Gly  Glu Asp Val
             1115                1120                1125

         Ala Met  Ala His Ala Asp Ala  Leu Asp Asp Phe Asp  Leu Asp Met
             1130                1135                1140

         Leu Gly  Asp Gly Asp Ser Pro  Gly Pro Gly Phe Thr  Pro His Asp
             1145                1150                1155

         Ser Ala  Pro Tyr Gly Ala Leu  Asp Met Ala Asp Phe  Glu Phe Glu
             1160                1165                1170

         Gln Met  Phe Thr Asp Ala Leu  Gly Ile Asp Glu Tyr  Gly Gly
             1175                1180                1185
```

**Description**

The present invention provides chimeric polypeptides that are useful in identifying modulators of adhesion G protein-coupled receptors and Polycystin-1/Polycystin-1-like proteins. The invention further provides screening methods using the novel polypeptides.

BACKGROUND

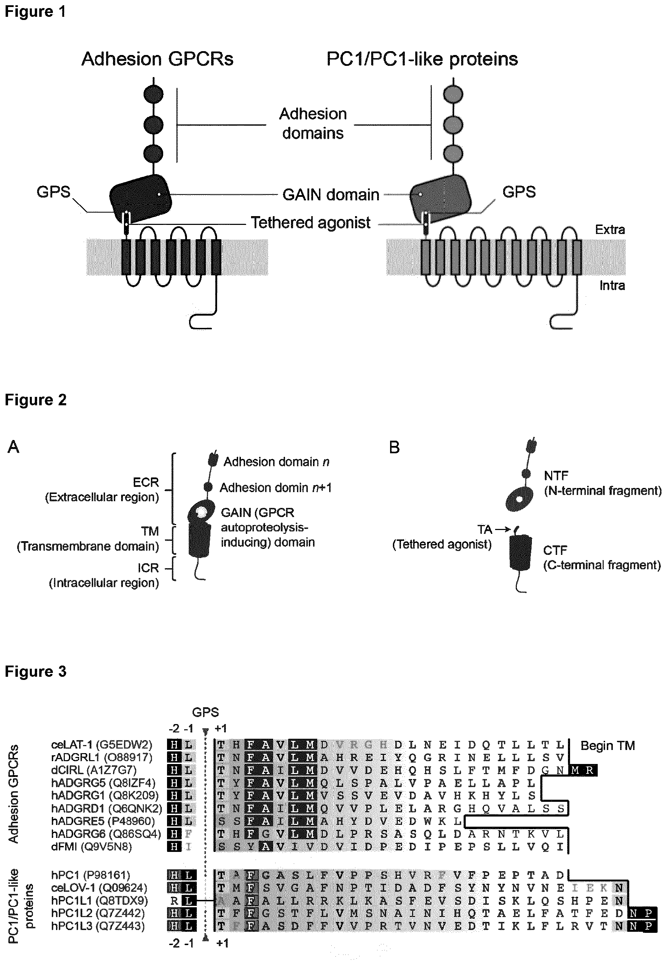

Adhesion G protein-coupled receptors (aGPCRs) and Polycystin-1/Polycystin-1-like (PC1/PC1-like) proteins are involved in physiological cell and organ functions. Genetic aberrations and the resulting functional impairments in these molecules play a role in human diseases. [1-7] aGPCRs and PC1/PC1-like molecules form two classes of cell membrane receptors with 7 and 1/11 transmembrane segments, respectively (Figure 1). They share structural similarities by possessing additional adhesion domains in their extracellular region (ECR), a GPCR autoproteolysis-inducing (GAIN) domain, and an intracellular region (ICR) (Figure 2A).

The physical dissociation of aGPCRs and PC1/PC1-like molecules (Figure 2B) is enabled by the autoproteolytic processing of the receptor proprotein generating a NTF/CTF (N-terminal fragment/C-terminal fragment) heterodimer joined at the GAIN domain[9] (Figures 1 and 2). This process occurs in the endoplasmic reticulum or early Golgi apparatus upon folding of the GAIN domain.[10] The GAIN domain cleaves the receptor precursor at the GPS (GPCR proteolysis site) and stabilizes the NTF-CTF heterodimer through non-covalent interactions[9, 11-15] (Figures 1 and 2 A). If such forces were to overcome, for example, upon engagement of the NTF with ligands, mechanical stimulation or a combination thereof, disjunction of the heterodimer occurs through force transmission to the NTF-CTF complex (Fig. 2B) (reviewed in refs. [3-5, 8, 16]).

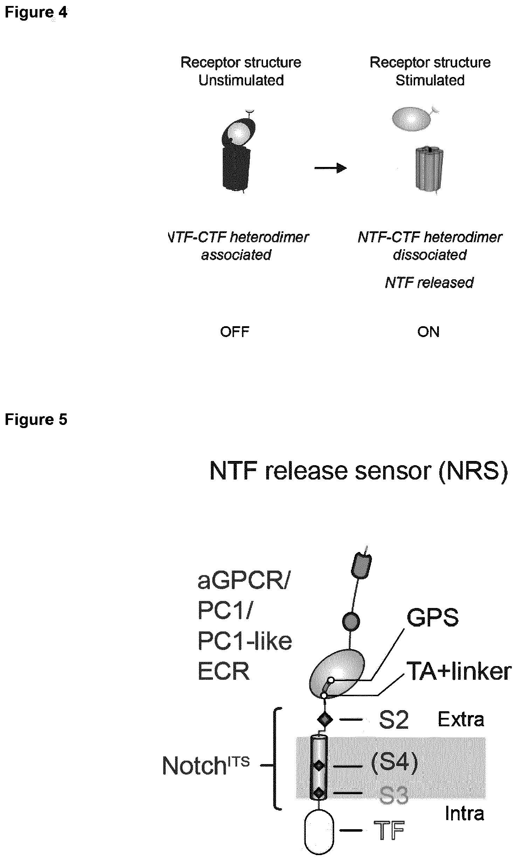

NTF-CTF separation is predicted to expose a short β-sheet motif C-terminal of the GPS[4, 17] This motif has been previously shown to harbour receptor stimulating activity by acting as a tethered agonist (TA)[18, 19]. aGPCR activation results in the stimulation of canonical intracellular second messenger cascades and thereby cell-autonomously governs biochemical and cell biological states (reviewed in ref .[7]) Presence and principal utility of TAs has since been demonstrated for numerous aGPCRs and Polycystin-1 (reviewed in refs. [6, 20, 36]) (Figure 3). Based on these observations, it appears critical to detect GAIN domain proteolysis and NTF-CTF separation as initiating steps for aGPCR and PC1/PC1-like molecule activation (Figure 4), in order to study its occurrence, effects and possible modulation by physiological and artificial means.

To date, the family of adhesion G protein-coupled receptors (aGPCRs) consists of 33 members in humans. Many reports have demonstrated critical functions for members of this family in organogenesis, neurodevelopment, myelination, angiogenesis, and cancer progression. Importantly, mutations in several aGPCRs have been linked to human diseases. Furthermore, overexpression in cancer cells of aGPCRs has been reported. Yet, there are no approved drugs specifically targeting these proteins.

To date, there are no screening methods for identifying molecules that affect GAIN domain proteolysis and/or NTF-CTF separation, in particular in a high-throughput format. There is a need for such methods, in particular for methods to identify inhibitors of GAIN domain proteolysis and/or NTF-CTF separation.

SUMMARY OF THE INVENTION

The inventors surprisingly found that GAIN domain proteolysis and/or NTF-CTF separation can be detected using a chimeric sensor protein comprising the extracellular region of an aGPCR, a Notch transmembrane domain, and an intracellular reporter moiety. The sensor protein of the present invention allows the identification of compounds that modulate the activation of aGPCR molecules or PC1/PC1-like molecules, e.g. by affecting GAIN domain proteolysis and/or NTF-CTF separation.

The present invention therefore relates to the subject matter defined in the following items [1] to [33]:

[1] A polypeptide comprising (i) a first sequence comprising a GPCR autoproteolysis-inducing (GAIN) domain of an adhesion GPCR or of a PC1/PC1-like protein, (ii) a second sequence comprising the transmembrane region of a Notch receptor, and (iii) a third sequence comprising a transcription factor moiety.

[2] The polypeptide of item [1], wherein the first sequence comprises or consists of the N-terminal part of the aGPCR or PC1/PC1-like protein, from the N-terminal amino acid of the aGPCR or PC1/PC1-like protein to the C-terminal end of the GAIN domain of the aGPCR or PC1/PC1-like protein.

[3] The polypeptide of item [1] or [2], wherein the aGPCR is selected from the group of aGPCRs listed in Table 1 hereinbelow.

[4] The polypeptide of any one of the preceding items, wherein the first sequence comprises or consists of the extracellular region of an aGPCR selected from the group of aGPCRs listed in Table 1 hereinbelow.

[5] The polypeptide of item [1] or [2], wherein the PC1/PC1-like protein is selected from the group of PC1/PC1-like proteins listed in Table 2 hereinbelow.

[6] The polypeptide of item [1], [2] or [5], wherein the first sequence comprises or consists of the extracellular region of an PC1/PC1-like protein selected from the group of PC1/PC1-like proteins listed in Table 2 hereinbelow.

[7] The polypeptide of any one of the preceding items, wherein the GAIN domain comprises or consists of an amino acid sequence selected from the group consisting of SEQ ID NOs:1-37.

[8] The polypeptide of any one of the preceding items, wherein the GAIN domain comprises or consists of an amino acid sequence selected from the group consisting of SEQ ID NOs:1-32.

[9] The polypeptide of any one of items [1] to [7], wherein the GAIN domain comprises or consists of an amino acid sequence selected from the group consisting of SEQ ID NOs:33-37.

[10] The polypeptide of any one of the preceding items, wherein the second sequence comprises the S2 cleavage site of a Notch receptor.

[11] The polypeptide of any one of the preceding items, wherein the second sequence comprises the S3 cleavage site and optionally the S4 cleavage site of the Notch receptor.

[12] The polypeptide of any one of the preceding items, wherein the second sequence comprises or consists essentially of a fragment of the Notch receptor comprising the S2 and the S3 cleavage sites, and optionally the S4 cleavage site.

[13] The polypeptide of any one of the preceding items, wherein the second sequence consists essentially of the iuxtamembrane segment and the transmembrane region of the Notch receptor.

[14] The polypeptide of any one of the preceding items, wherein the second sequence comprises or consists essentially of (i) an amino acid sequence selected from the group consisting of SEQ ID NOs:52-56, (ii) an amino acid sequence having a sequence identity of at least 90% to any one of SEQ ID NOs:52-56, (iii) an amino acid sequence which differs from any one of SEQ ID NOs:52-56 in less than 10, or less than 7, or less than 5, or less than 3 amino acids, e.g. in one or two amino acids, or (iv) a fragment of (i), (ii) or (iii) comprising the S2 and the S3 cleavage sites.

[15] The polypeptide of any one of the preceding items, wherein the transcription factor moiety is selected from the group consisting of LexA from *E. coli,* GAL4 from *S. cerevisiae,* QF, QF2 and QF2^W derived from *N. crassa.*

[16] The polypeptide of any one of the preceding items, wherein the transcription factor moiety, upon separation from the second sequence, is capable of activating transcription of a reporter gene.

[17] The polypeptide of any one of the preceding items, wherein release of an N-terminal portion of the first sequence induces cleavage of the second sequence at the one or more proteolytic cleavage sites, thereby releasing the transcription factor moiety.

[18] The polypeptide of any one of the preceding claims, which is a sensor protein suitable for detecting the release of its N-terminal fragment and/or proteolytic cleavage within the GAIN domain.

[19] The polypeptide of item [1], having or consisting essentially of the amino acid sequence as shown in SEQ ID NO:59.

[20] The polypeptide of any one of the preceding items, which is embedded in a cell membrane.

[21] The polypeptide of any one of the preceding items, which is integrated in the membrane of a cell, wherein the first sequence extends to the extracellular space, the transmembrane region is located within the membrane, and the transcription factor moiety extends to the intracellular space.

[22] A nucleic acid encoding the polypeptide of any one of the preceding items.

[23] A plasmid or vector comprising the nucleic acid of item [22].

[24] A cell comprising the polypeptide of any one of items [1] to [21], the nucleic acid of item [22], or the plasmid or vector of item [23].

[25] The cell of item [24], further comprising a nucleic acid capable of binding to the transcription factor moiety, operably linked to a nucleic acid encoding a reporter.

[26] A non-human transgenic animal comprising the polypeptide of any one of items [1] to [21], the nucleic acid of item [22], the plasmid or vector of item [23], or the cell of item [24] or [25].

[27] A non-human transgenic animal expressing the polypeptide of any one of items [1] to [21].

[28] A screening method comprising the following steps

(a) contacting a test compound with the cell of item [24] or [25], or administering a test compound to the transgenic animal of item [26] or [27] and
(b) determining the level of the reporter or of a detectable signal caused or generated by the reporter.

[29] A method for identifying modulators of an aGPCR or of a PC1/PC1-like protein comprising the following steps

(a) contacting a test compound with the cell of item [24] or [25], or administering a test compound to the transgenic animal of item [26] or [27] and
(b) determining the level of the reporter or of a detectable signal caused or generated by the reporter.

[30] The method of item [28] or [29], wherein the modulator is an inhibitor.

[31] The method of any one of items [28] to [30], wherein the cell or the transgenic animal comprises (i) a nucleic acid sequence which is capable of binding to the transcription factor moiety of the polypeptide upon release of the transcription factor moiety from the polypeptide, and (ii) a nucleic acid sequence encoding a reporter; wherein expression of the reporter is induced when the nucleic acid sequence which is capable of binding to the transcription factor moiety binds to the transcription factor moiety.

[32] The method of any one of items [28] to [31], further comprising the steps of comparing the level determined in step (b) with a control level, and selecting the test compound if the level determined in step (b) is lower than the control level.

[33] The use of the polypeptide of any one of items [1] to [21], the nucleic acid of item [22], the plasmid or vector of item [23], the cell of item [24] or [25], or of the transgenic of item [26] or [27] for identifying a modulator of an adhesion GPCR or of a PC1/PC1-like protein, preferably an inhibitor of an adhesion GPCR or of a PC1/PC1-like protein.

DESCRIPTION OF THE FIGURES

**Figure 1.** Structural similarities between adhesion GPCRs and PC1/PC1-like proteins.

**Figure 2.** (A) Adhesion GPCRs and PC1/PC1-like proteins are composed of a large extracellular region (ECR), heptahelical transmembrane (7TM) domain, and an intracellular region (ICR). (B) The GAIN domain is autoproteolytically active and generates an N- (NTF) and C-terminal fragment (CTF) from each adhesion GPCR/PC1/PC1-like preprotein. The two fragments form a non-covalently stabilised heterodimer through non-covalent interactions.

**Figure 3.** Amino acid alignment of the protein region at the GPS of adhesion GPCRs and PC1/PC1-like proteins highlighting the high evolutionary conservation of cleavage site and tethered agonist segment of both protein classes (SEQ ID NOs:38-51).

**Figure 4.** The dissociation model proposes that aGPCR activation involves separation of the NTF/CTF heterodimer and NTF release.

**Figure 5.** Molecular design of a polypeptide of the invention, also referred to herein as NTF Release Sensor (NRS) protein. ECR, extracellular region; GPS, GPCR proteolysis site; ITS, iuxta- and transmembrane segment; TA, tethered agonist; TF, transcription factor subunit.

**Figure 6.** Functionality of the NRS is based on the regulated intramembrane proteolysis of Notch, and couples NTF release to the intracellular release and intranuclear activity of heterologous transcription factors (TF).

**Figure 7.**

(A) NRS signaling is repressable by fusion with heterologous extracellular domains such as Ig domains of CD4, and can be NRS re-activated by extracellular TEVp expression. CD4-NRS-LexA vs. NECN-LexA: 37.7x; n=3, p = < 0.0001; CD4-3TEVs-NRS-LexA vs. + secTEVp: 13.3x, n=3, p = 0.0008; CD4-3TEVs-NRS-LexA vs. + intraTEVp: 1.2x, n=3, p = 0.0335; CD4-6TEVs-NRS-LexA vs. + secTEVp: 28.6x, n=3, p = 0.0004; CD4-6TEVs-NRS-LexA vs. + intraTEVp: 5.6x, n=3, p = 0.0005. Unpaired t-test, two-tailed.

(B) Representative S2 cell reporter assay of different CD4-NRS-LexA versions. Only NECN-LexA and CD4-6TEVs-NRS-LexA activated by extracellular secTEVp results in proteolytic processing NRS and reporter gene activation. Scale bar = 100 $\mu$m.

**Figure 8.**

(A) Alignment of the ITS region of *Drosophila* Notch (SEQ ID NO:57) and mouse Notch (SEQ ID NO:58), positions of S2, S3 and S4 sites are indicated[24]. Predicted transmembrane helix boxed in light grey, S3 site Valine residue mutated in NRS constructs in dark grey.

(B) $\gamma$-secretase substrate recognition by a critical S3 site mutation abrogates NRS responses. Representative luciferase activity assay. $N^{ECN}$-LexA vs. $N^{ECN/S3}$-LexA: 7.9x, n=4, p=0.0009; CD4-6TEVs-NRS-LexA vs. + secTEVp: 31.0x, n=4, p=0.0005; CD4-6TEVs-NRS$^{S3}$-LexA vs. + secTEVp: 1.6x, n=4, p=0.0091. Unpaired t-test, two-tailed.

(C) Pharmacological inhibition of y-secretase activity suppresses NRS responses. Representative luciferase activity assay showing reporter activity without DAPT vs. with 10 M DAPT: Control: 1.1x, n=3, p=0.8298; $N^{EGF}$-LexA: 5.8x, n=3, p=0.0022; $N^{ECN}$-LexA: 43.7x, n=3, p=0.0018; CD4-6TEVs-NRS-LexA: 3.6x, n=3, p=0.0053; CD4-6TEVs-NRS-LexA + secTEVp: 23.4x, n=3, p=0.0039; CD4-6TEVs-NRS-LexA + intraTEVp: 7.0x, n=3, p=0.0493. Unpaired t-test, two-tailed.

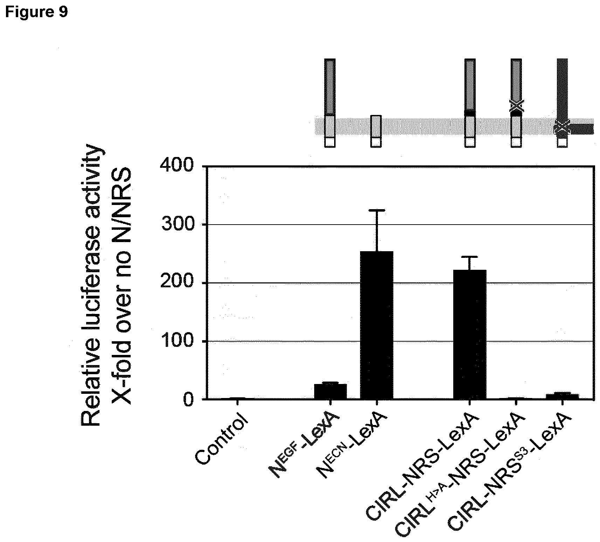

**Figure 9.** CIRL-NRS-LexA shows constitutive activity through NTF/CTF dissociation. Representative luciferase activity assay which is quenched by preclusion of either GAIN domain autoproteolysis or S3 proteolysis through respective cleavage site mutations. $N^{EGF}$-LexA vs. $N^{ECN}$-LexA: 254.6x, n=4, p=0.0006; Cirl-NRS-LexA vs. Cirl$^{H>A}$-NRS-LexA: 148.8x, n=4, p=<0.0001; Cirl-NRS-LexA vs. Cirl-NRS$^{S3}$-LexA: 23.7x, n=4, p=<0.0001.

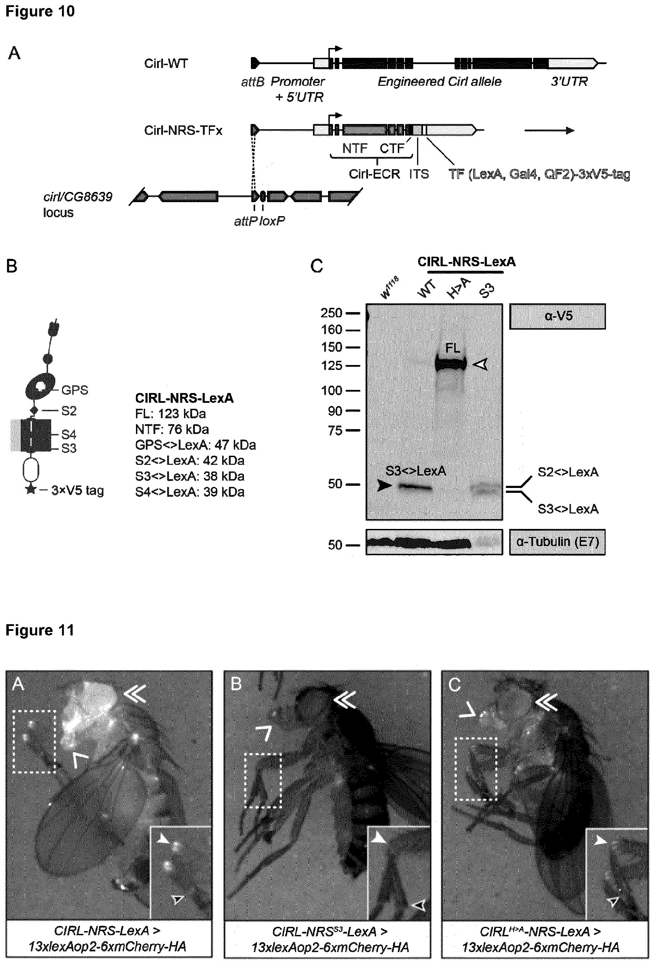

**Figure 10.** NRS working principle tested *in vivo* using *Drosophila melanogaster.* Biochemical confirmation. (A) Layout of genomically engineered *cirl* alleles in *Drosophila melanogaster* that encode various *cirl-NTF-TFx* transgenes. (B) Layout of the gene product encoded by the *cirl-NRS-LexA* allele, and molecular weights of posttranslational cleavage fragments of the gene product resulting from GAIN domain, S2, S3 and S4 proteolyses. GPS, GPCR proteolysis site; FL, full-length protein; NTF, N-terminal fragment. (C) Western blot of protein harvested from animals carrying genomically integrated *cirl-NRS-LexA* sensor variants. The C-terminal sensor protein portion of the wildtype (WT) sensor (black arrowhead) derives from the GPS>S2>S3 cascade. In contrast, when GAIN domain cleavage is blocked by the H>A GPS mutation, the upward band shift corresponds to the full-length sensor protein (white arrowhead). Abrogating sensor proteolysis at the S3 site of the Notch ITS fragment results in a double band indicating incomplete S3 cleavage.

**Figure 11.** NRS working principle tested *in vivo* using *Drosophila melanogaster* Anatomical confirmation through expression analysis of the CIRL-NRS-LexA sensor through induction of a transcriptionally activated mCherry chromophore. (A) CIRL-NRS-LexA expression was observed in proboscis (chevron), eyes (double chevron) and leg joints (boxe and inset). (B) This is abrogated upon mutation the S3 site within the Notch[ITS] component of the sensor protein. (C) mCherry expression is also largely suppressed in flies carrying a sensor variant lacking GAIN domain proteolysis through mutation of a -2 His to Ala, a classical mutation disrupting adhesion GPCR and PC1/PC1-like protein GAIN domain proteolysis. White arrowheads, femorotibial joint; black arrowhead, tibiotarsal joint.

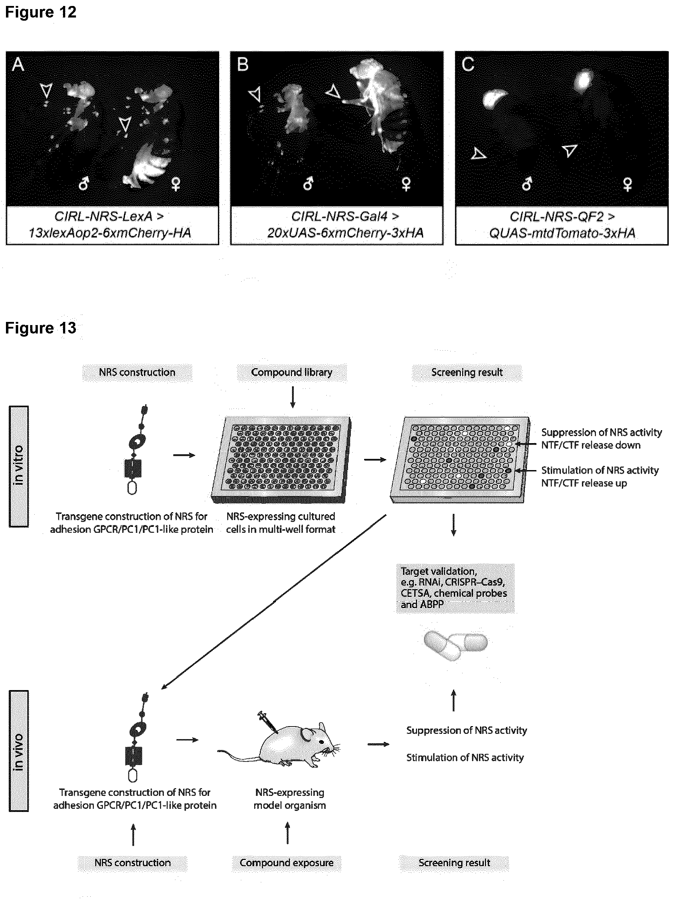

**Figure 12.** Expression of genomically integrated *cirl-NRS-TFx* sensors with different TF cassettes shows comparable expression patterns in adult animals with strong activity in the eye, proboscis and leg joints (arrowheads) of (A) *cirl-NTF-LexA* and (B) *cirl-NTF-Gal4*. (C) *cirl-NTF-QF2* displayed pronounced activity only in the eye. Leg joint expression was detectable at fainter levels (arrowheads).

**Figure 13.** Schematic workflow of the NRS-methodology for *in-vitro* high throughput screening and/or compound testing in transgenic animal models.

DETAILED DESCRIPTION

The present invention relates to a polypeptide comprising (i) a first sequence comprising a GAIN domain of an adhesion GPCR or of a PC1/PC1-like protein (ii) a second sequence comprising the transmembrane region of a Notch receptor, and (iii) a third sequence comprising a transcription activator.

The polypeptide of the invention is a chimeric transmembrane protein comprising a single-pass transmembrane domain.

*First sequence*

The term "adhesion GPCR" or "aGPCR" refers to a G-protein coupled receptor protein of class B subfamily 2[37] characterized by a 7 transmembrane domain and an extracellular region (ECR) with a GAIN domain. It does not necessarily need to but can additionally contain one or more adhesion domains within its ECR.

The term "adhesion domain" refers to autonomously folding protein subunits that permit interactions with ligands contained within the extracellular matrix, mounted on the same (cis) or neighbouring (trans) cell surfaces. Adhesion domains mediate but are not limited to one or more of the following functions: adhesive cell anchoring, formation of cell junctions, cell migration, cell polarisation, tissue polarisation, interaction with fixed or soluble ligands, transmission of mechanical signals onto expressing cells.

The terms "GPCR autoproteolysis-inducing domain" and "GAIN domain", as used herein, refer to a region within the ECR of an aGPCR or PC1/PC1-like protein composed of an N-terminal subdomain A and a C-terminal subdomain B (see refs. 9, 38 and 39). Subdomain A of the GAIN domain is composed of 3 to 6 $\alpha$-heiices. Subdomain B of the GAIN domain is composed of 13 $\beta$-strands and can additionally contain several small $\alpha$-helices. Subdomain B typically forms a twisted $\beta$-sandwich structure. Based on primary protein sequences these properties can be predicted by publicly available software tools such as Phyre2 (http://www.sbg.bio.ic.ac.uk/~phyre2), Psipred (http://bioinf.cs.ucl.ac.uk/psipred/) and DALI (http://ekhidna2.biocenter.helsinki.fi/dali/). Primary sequence identity between orthologous GAIN domains can be as low as 10 %, but the Root Mean Square Deviation (RMSD) of atomic positions of the folded GAIN domain is < 5 Å.

For example, the GAIN domain of rat CL1 (UniProtKB - 088917 / AGRL1_RAT) extends from Thr533 - Ile849 of the amino acid sequence of the receptor. Using structure-based searches such as DALI and BLAST one can determine for other aGPCRs or PC1/PC1-like proteins which amino acids constitute the GAIN domain. For example, the GAIN domains of several human aGPCRs are shown in SEQ ID NOs:1-32 (see also Table 1 below), and the GAIN domains of several human PC1/PC1-like proteins are shown in SEQ ID NOs:33-37 (see also Table 2 below). The GAIN domain may or may not induce autoproteolysis in the polypeptide of the invention. The term "GAIN" domain includes domains which do not exert autoproteolytic activity, but are classified as GAIN domains on the basis of alignments and/or sequence similarity to other proteins known to have a GAIN domain. Some adhesion GPCRs and PC1 molecules contain autoproteolytic domains in addition to the GAIN domain, some are processed by other endogenous proteases. All three modes of cleavage (through the GAIN domain, through non-GAIN domains of the receptors, through endogenous proteases) can be detected using the polypeptide of the invention and are encompassed by the present invention.

The aGPCRs in the sense of the present invention include, but are not limited to, human aGPCRs and non-human orthologs thereof (e.g. from mammalians such as mouse, rat, *Macaca mulatta* or *Macaca fascicularis,* other vertebrates such as *Xenopus tropicalis* and *Danio rerio,* and invertebrates such as *Drosophila melanogaster* or *Caenorhabditis elegans*). Preferably, the aGPCR is a human aGPCR. More preferably, the aGPCR is selected from the aGPCRs listed in Table 1.

Table 1. Human aGPCRs.

| Name | Synonym (s) | Human nucleotide RefSeq | Human SwissProt | GAIN domain sequence(SEQ ID NO) |
|---|---|---|---|---|
| ADGRA2 | GPR124 | NM_032777 | Q96PE1 | 1 |
| ADGRA3 | GPR125 | NM_145290 | Q8IWK6 | 2 |
| ADGRB1 | BAI1 | NM_001702 | 014514 | 3 |
| ADGRB2 | BAI2 | NM_001294335 | 060241 | 4 |
| ADGRB3 | BAI3 | NM_001704 | O60242 | 5 |
| ADGRC1 | CELSR1 | NM_014246 | Q9NYQ6 | 6 |
| ADGRC2 | CELSR2 | NM_001408 | Q9HCU4 | 7 |
| ADGRC3 | CELSR3 | NM_001407 | Q9NYQ7 | 8 |
| ADGRD1 | GPR133 | NM_198827 | Q6QNK2 | 9 |
| ADGRD2 | GPR144 | | Q7Z7M1 | 10 |
| ADGRE1 | EMR1 | NM_001974; NM_001256252; NM_001256253; NM_001256254; NM_001256255 | Q14246 | 11 |
| ADGRE2 | EMR2; CD312 | NM_013447 | Q9UHX3 | 12 |
| ADGRE3 | EMR3 | NM_032571 | Q9BY15 | 13 |
| ADGRE4P | EMR4; GPR127 | NR_024075 | Q86SQ3 | 14 |
| ADGRE5 | CD97 | NM_078481; NM_001025160; NM_001784 | P48960 | 15 |
| ADGRF1 | GPR110 | NM_153840 | Q5T601 | 16 |
| ADGRF2 | GPR111 | NM_153839 | Q8IZF7 | 17 |
| ADGRF3 | GPR113 | NM_153835 | Q8IZF5 | 18 |
| ADGRF4 | GPR115 | NM_153838 | Q8IZF3 | 19 |
| ADGRF5 | GPR116 | NM_015234 | Q8IZF2 | 20 |
| ADGRG1 | GPR56 | NM_005682 | Q9Y653 | 21 |
| ADGRG2 | GPR64 | NM_001079858; NM_001079859; NM_001079860; NM_005756; NM_001184833; NM_001184834; NM_001184835; NM_001184836 | Q8IZP9 | 22 |
| ADGRG3 | GPR97 | NM_170776 | Q86Y34 | 23 |
| ADGRG4 | GPR112 | NM_153834 | Q8IZF6 | 24 |
| ADGRG5 | GPR114 | NM_153837 | Q8IZF4 | 25 |
| ADGRG6 | GPR126 | NM_020455; NM_001032394; NM_001032395; NM_198569 | Q86SQ4 | 26 |
| ADGRG7 | GPR128 | NM_032787 | Q96K78 | 27 |
| ADGRL1 | LPHN1; CIRL1 | NM_014921; NM_001008701 | 094910 | 28 |
| ADGRL2 | LPHN2; CIRL2 | NM_012302 | 095490 | 29 |
| ADGRL3 | LPHN3; CIRL3 | NM_015236 | Q9HAR2 | 30 |

(continued)

| Name | Synonym (s) | Human nucleotide RefSeq | Human SwissProt | GAIN domain sequence (SEQ ID NO) |
|---|---|---|---|---|
| ADGRL4 | ELTD1 | NM_022159 | Q9HBW9 | 31 |
| ADGRV1 | VLGR1; GPR98 | NM_032119 | Q8WXG9 | 32 |

The term "PC1/PC1-like protein" as used herein refers to polypeptides comprising a 1 or 11 transmembrane domain and an extracellular region with a GAIN domain, PKD domains and adhesion domains. Preferably the PC1/PC1-like protein in accordance with this invention is a human PC1/PC1-like protein. In another preferred embodiment the PC1/PC1-like protein is selected from the group consisting of human Polycystin-1 (hPC1; also known as hPKD1), hPC1L1 (also known as hPKDL1), hPC1L2 (also known as hPKDL2), hPC1L3 (also known as hPKDL3), hPKDREJ and non-human orthologs thereof (e.g. from mammalians such as mouse, rat, *Macaca mulatta* or *Macaca fascicularis*).

Table 2. Human PC1/PC1-like proteins.

| Name | Synonym (s) | Human nucleotide RefSeq | Human SwissProt | GAIN domain sequence (SEQ ID NO) |
|---|---|---|---|---|
| PC1 | PKD1 | NM_000296.3; NM_001009944.2 | P98161 | 33 |
| PC1L1 | PKDL1 | NM_138295.4 | Q8TDX9 | 34 |
| PC1L2 | PKDL2 | NM_001076780.1; NM_001278423.1; NM_001278425.1; NM_052892.3 | Q7Z442 | 35 |
| PC1L3 | PKDL3 | NM_181536.1 | Q7Z443 | 36 |
| PKDREJ |  | NM_006071 | Q9NTG1 | 37 |

The full length of the polypeptide of the invention may range from about 200 to about 10,000 amino acids, preferably from about 500 to about 8,000 amino acids, or from about 1,000 to about 6,000 amino acids.

The first sequence of the polypeptide of the invention typically has a length from about 150 to about 7,000 amino acids, preferably from about 200 to about 6,000 amino acids, or from about 300 to about 5,000 amino acids, or from 400 to 4,000 amino acids. The first sequence may comprise at least two adhesion domains, preferably at least three or at least four or at least five adhesion domains.

In another embodiment, the first sequence comprises or consists of the N-terminal amino acid sequence of an aGPCR extending from the N-terminus of the aGPCR to the C-terminus of the GAIN domain. In another embodiment the first sequence comprises or consists essentially of the complete ECR of the aGPCR, i.e. the first sequence of the polypeptide ends before the start of the TM domain of the aGPCR. In yet other embodiments the first sequence comprises or consists essentially of the N-terminal amino acid sequence of a PC1/PC1-like protein from the N-terminus to the C-terminus of the GAIN domain. In another embodiment the first sequence comprises or consists essentially of the complete ECR of the PC1/PC1-like protein, i.e. the first sequence of the polypeptide ends before the start of the TM domain of the PC1/PC1-like protein.

In a specific embodiment, the first sequence comprises or consists essentially of (a) an amino acid sequence selected from the group consisting of SEQ ID NOs:1-37, (b) an amino acid which has a sequence identity of at least 90% to any one of SEQ ID NOs:1-37, or (c) an amino acid sequence which differs from an amino acid sequence as shown in any one of SEQ ID NOs:1-37 in less than 50, or less than 25, or less than 10, or less than 5, or less than 3 amino acids.

In another embodiment, the first sequence comprises or consists essentially of (a) an amino acid sequence selected from the group consisting of SEQ ID NOs:1-32, (b) an amino acid which has a sequence identity of at least 90% to any one of SEQ ID NOs:1-32, or (c) an amino acid sequence which differs from an amino acid sequence as shown in any one of SEQ ID NOs:1-32 in less than 50, or less than 25, or less than 10, or less than 5, or less than 3 amino acids.

In yet another embodiment, the first sequence comprises or consists essentially of (a) an amino acid sequence selected from the group consisting of SEQ ID NOs:33-37, (b) an amino acid which has a sequence identity of at least 90% to any one of SEQ ID NOs:33-37, or (c) an amino acid sequence which differs from an amino acid sequence as shown in any one of SEQ ID NOs:33-37 in less than 50, or less than 25, or less than 10, or less than 5, or less than 3 amino acids.

The difference in amino acid sequence may be a substitution, deletion and/or insertion relative to the reference sequence. The comparison of sequences and determination of percent identity between two amino acid sequences can be accomplished using any suitable program, e.g. the program "BLAST 2 SEQUENCES (blastp)" (Tatusova et al. (1999) FEMS Microbiol. Lett. 174, 247-250) with the following parameters: Matrix BLOSUM62; Open gap 11 and extension gap 1 penalties; gap x_dropoff50; expect 10.0 word size 3; Filter: none.

In another preferred embodiment the first sequence comprises one of the amino acid sequences shown in Figure 3 (SEQ ID NOs:38-51).

In other embodiments, the first sequence comprises or consists of the GAIN domain of one of the following aGPCRs:

mGPR133, rGPR128, rGPR126, rGPR116, mGPR110, rGPR64, mCELSR3, rCELS, mGPR125, mGPR124, rCD97, rEMR1, mEMR4, mGPR97, rGPR114, rETL1, mGPR115, mGPR111, mVLGR1, rGPR113, rBAI1, rBAI2, and rCL1. These GAIN domain sequences are disclosed in Supplementary Table 3 of Arac et al. EMBO J 31, 1364-1378 (2012), which sequences are incorporated herein by reference.

In yet other embodiments, the first sequence comprises or consists of the GAIN domain of one of the following aGPCRs:, TT89292280, TT89298346, DM48958429, TT89296699, CE115532418, MB16753744x7, MB167524088, MB238058440, TA195996857, DD66815909, TA196016767, CE35396740, TA196004662, and DM19527793. These GAIN domain sequences are disclosed in Supplementary Table 2 of Arac et al. EMBO J 31, 1364-1378 (2012), which sequences are incorporated herein by reference.

*Second sequence*

The second sequence comprises the transmembrane region of a Notch receptor. The Notch receptor can be a human Notch receptor. In one embodiment the Notch receptor is selected from the group consisting of Notch1, Notch2, Notch3, and Notch4. The amino acid sequence of human Notch1 can be found in UniProtKB - P46531. The amino acid sequence of human Notch2 is described in UniProtKB - Q04721. The amino acid sequence of human Notch3 is described in UniProtKB - Q9UM47. The amino acid sequence of human Notch4 is described in UniProtKB - Q99466. In other embodiments the Notch receptor is a mammalian orthologue of one of the human Notch proteins mention above.

The length of the second sequence may range from about 40 amino acids to about 100 amino acids, preferably from about 45 to about 75 amino acids, or from about 50 to about 60 amino acids.

The second sequence typically includes the S2 cleavage site which is located in the extracellular part of the Notch protein, adjacent to the TM domain. The S2 cleavage site can be cleaved by the matrix metalloprotease of the Kuzbanian/ADAM (a disintegrin and metalloprotease domain) family. The short segment from the amino acids constituting the S2 cleavage site of a Notch receptor to the C-terminal end of the extracellular region of the Notch receptor is referred to as iuxtamembrane segment herein. For the avoidance of doubt, the iuxtamembrane segment includes the S2 cleavage site, but it does not include amino acids of the transmembrane region of the Notch receptor. Thus, the second sequence typically comprises or consists essentially of the iuxtamembrane seqment and the transmembrane region of a Notch receptor. For example, the iuxtamembrane segment of human Notch 1 includes amino acids 25-42 of SEQ ID NO:52. The structure and function of Notch receptors is reviewed in ref. [24].

The second sequence usually further comprises the S3 cleavage site, which is located within the TM portion of Notch. In yet another embodiment the second sequence further comprises the S4 cleavage site, which is located within the TM portion of Notch.

In a preferred embodiment the second sequence comprises or consists essentially of (i) an amino acid sequence selected from the group consisting of SEQ ID NOs:52-56, (ii) an amino acid sequence having a sequence identity of at least 90% to any one of SEQ ID NOs:52-56, (iii) an amino acid sequence which differs from any one of SEQ ID NOs:52-56 in less than 10, or less than 7, or less than 5, or less than 3 amino acids, e.g. in one or two amino acids, or (iv) a fragment of (i), (ii) or (iii) comprising the S2 and the S3 cleavage sites. The fragment may have an N-terminal truncation of any one of SEQ ID NOs:52-56, by 1-25 amino acids, by 1-24 amino acids, by 1-23 amino acids, by 1-22 amino acids, by 1-21 amino acids, or by 1-20 amino acids. The fragment may have a C-terminal truncation of any one of SEQ ID NOs:52-56, by 1, 2, 3, 4 or 5 amino acids. The embodiments concerning the N-terminal truncation and the C-terminal truncation can be combined.

*Third sequence*

The polypeptide of the invention further comprises, at the C-terminal end, a transcription factor moiety.

The length of the third sequence may range from about 50 amino acids to about 1,500 amino acids, or from about 100 to about 1,000 amino acids.

Upon release from the second sequence, e.g. by proteolytic cleavage, the transcription factor moiety can bind to a DNA target sequence, thereby inducing transcription of a reporter gene. The type of the reporter gene is not particularly limited, provided that it encodes an expression product that can be detected, or which provides a detectable signal. Suitable transcription factors include, but are not limited to, LexA from *Escherichia coli*[21], GAL4 from *Saccharomyces cerevisiae* [22], *and* QF, QF2 and QF2ʷ derived from *Neurospora crassa* [23], for which corresponding transcription factor DNA binding motifs for the construction of reporter transgenes are known.

Optionally, there can be a spacer between the first sequence and the second sequence, and/or between the second and the third sequence. The spacer does not affect the functionality of the chimeric polypeptide. The length of the spacer may range from about 1 to about 10 amino acids. The sequence of the spacer may be heterologous to the adjacent sequences. Preferably, no spacer is present between the first sequence and the second sequence. In another embodiment, no spacer is present between the second and the third sequence. In yet another embodiment, no spacer is present between the first sequence and the second sequence, nor between the second and the third sequence. In a specific embodiment, no spacer is present between the first sequence and the second sequence, and a spacer is present between the second and the third sequence.

In other optional embodiments, the polypeptide of the invention comprises at its N-terminal end an amino acid sequence that is heterologous to the aGPCR sequence or PC1/PC1-like sequence that is present in the first sequence. Examples of such heterologous sequences include tag sequences, signal peptides and the like.

In other optional embodiments, the polypeptide of the invention comprises at its C-terminal end an amino acid sequence that is heterologous to the transcription factor sequence of that is present in the third sequence. Examples of such heterologous sequences include tag sequences and the like.

*Nucleic Acids*

The present invention further relates to a nucleic acid encoding the polypeptide of the invention. The nucleic acid sequences encoding various aGPCRs and Notch proteins are known. Similarly, the nucleic acid sequences encoding various transcription factor moieties are known. Based on the teaching of the present application the skilled person is able to design and prepare suitable nucleic acid constructs encoding the polypeptide described herein.

The invention further pertains to a plasmid or vector comprising a nucleic acid of the invention. The vector is preferably an expression vector. Typical expression vectors contain promoters that direct the synthesis of large amounts of mRNA corresponding to the inserted nucleic acid in the plasmid-bearing cells. They may also include an origin of replication sequence allowing for their autonomous replication within the host organism, and sequences that increase the efficiency with which the synthesized mRNA is translated. Stable long-term vectors may be maintained as freely replicating entities by using regulatory elements of, for example, viruses (e.g., the OriP sequences from the Epstein Barr Virus genome). Cell lines may also be produced that have integrated the vector into the genomic DNA, and in this manner the gene product is produced on a continuous basis.

*Cells*

The present invention further relates to a cell comprising the polypeptide of the present invention. The invention further pertains to a cell comprising the nucleic acid of the present invention. The invention further pertains to a cell comprising the plasmid or vector of the present invention. The invention further pertains to a cell expressing the polypeptide of the present invention. The cell of the invention is obtainable by a process comprising the steps of introducing a nucleic acid encoding the polypeptide of the invention into a host cell, and culturing the cells so obtained under conditions that allow expression of the polypeptide.

Any cell can be chosen as long as the polypeptide as expressed is functional. In one embodiment, the expression of the polypeptide is stable, optionally inducible. Alternatively, the expression of the polypeptide is transient. Preferably, the cell is a eukaryotic cell. More preferably, the cell is an insect or a mammalian cell. Even more preferably, the mammalian cell is a human cell. Examples of mammalian cells are HEK293, HEK293T, MDCK, CHO, COS, NIH3T3, Swiss3T3, BHK, and A549. Even more preferably, the cell is a mammalian cell such as HEK293.

Depending on the type of expression system chosen, the skilled person may possibly adapt the culture conditions to obtain a most favorable expression level of the polypeptide. In the case of an inducible expression system, the skilled person may also possibly optimize an inducing condition. The time period of induction of the expression and the temperature during induction of the expression could also possibly be optimized.

Typically, the cells to be provided are obtained by introducing the nucleic acid encoding a polypeptide of the invention into host cells, e.g. mammalian host cells. Typically, the host cell is transfected with a suitable plasmid or nucleic acid, or transduced with a suitable vector, e.g. a viral vector.

The cell of the invention preferably includes a heterologous nucleic acid sequence which is capable of binding to the transcription factor moiety comprised in the third sequence. Suitable transcription factor binding DNA sequences include, but are not limited to, QUAS (for binding to transcription activators of the Q system, such as QF or QF2), UAS (for binding to transcription activators such as Gal4) and LexAop (for binding to transcription activators such as LexA).

It is further preferred that the cell of the invention comprises a heterologous nucleic acid sequence encoding a reporter. Suitable reporters include, e.g., fluorescent proteins; enzymes that catalyze a reaction that generates a detectable signal as a product, and the like.

Suitable fluorescent proteins are described in Shaner et al. (2005) Nat. Methods 2:905-909) and Matz et al. (1999) Nature Biotechnol. 17:969-973, the content of which is incorporated herein.

Suitable enzymes include, but are not limited to, horse radish peroxidase (HRP), alkaline phosphatase (AP), beta-galactosidase (GAL), glucose-6-phosphate dehydrogenase, beta-N-acetylglucosaminidase, Xanthine Oxidase, firefly luciferase, glucose oxidase (GO), and the like.

Preferred reporters are firefly luciferase, green fluorescent protein and red fluorescent proteins such as mCherry. The nucleic acid sequences encoding reporters are known, as well as suitable promoters to be operably linked to the nucleic acid encoding the reporter.

Such cells are obtained by additionally introducing a nucleic acid encoding the reporter into the cell.

*Transgenic Animals*

The invention further pertains to a non-human transgenic animal comprising the polypeptide of the invention, the nucleic acid of the invention, the plasmid or vector of the invention, or the cell of the invention. The invention further relates to a non-human transgenic animal expressing the polypeptide of the present invention.

The transgenic non-human animal may be a transgenic non-human vertebrate animal, preferably a mammal, preferably a rodent, such as a mouse. Suitable animals are available, or easily generated, using conventional methods, in a variety of genera, including rodents (e.g., rats), rabbits, guinea pigs, dogs, goats, sheep, cows, horses, pigs, llamas, camels or the like. Preferably, the non-human transgenic animal is a transgenic mouse.

The invention further provides a transgenic gamete, including a transgenic ovum or sperm cell, a transgenic embryo, and any other type of transgenic cell or cluster of cells, whether haploid, diploid, or of higher zygosity comprising a nucleic acid of the present invention.

As used herein, the term "embryo" includes a fertilized ovum or egg (i.e., a zygote) as well as later multicellular developmental stages of the organism.

Also included herein are progeny of the transgenic animal that preferably comprises a nucleic acid of the invention.

The transgenic animal may be sterile although, preferably, it is fertile. The present invention further includes a cell line derived from a transgenic embryo or other transgenic cell of the invention, which comprises the nucleic acid of the invention and/or expresses the polypeptide of the invention. Methods of isolating such cells and propagating them are known to those of skill in the art.

Techniques for the generation of non-human transgenic animals are generally known to the skilled person (see, e.g. Pinkert, Transgenic Animal Technology: A Laboratory Handbook, ISBN-13: 978-0124104907). The transgenic non-human animals of the invention can be produced by introducing recombinant nucleic acids into the germline of the non-human animal. Embryonal target cells at various developmental stages are used to introduce the nucleic acids or vectors of the invention. Different methods are used depending on the stage of development of the embryonal target cell(s). Such methods include, but are not limited to, microinjection of zygotes, viral integration, and transformation of embryonic stem cells.

*Methods and Uses*

The invention further relates to methods for identifying compound that are capable of modulating aGPCR activity. The method may be a screening method. The method typically comprises the following steps

(a) contacting a test compound with a cell expressing the polypeptide of the present invention, or administering the test compound to a transgenic animal comprising such a cell, wherein said cell comprises (i) a nucleic acid sequence which is capable of binding to the transcription factor moiety of the polypeptide upon release of the transcription factor moiety from the polypeptide, and (ii) a nucleic acid sequence encoding a reporter; wherein expression of the reporter is induced when the nucleic acid sequence which is capable of binding to the transcription factor moiety binds to the transcription factor moiety; and

(b) determining the level of the reporter or of a detectable signal caused by the reporter.

"Determining the level" can be a quantitative determination or a qualitative determination (e.g. determining whether or not a reporter or signal can be detected).

The method may further comprise one or more of the following steps

- Prior to step (a), providing a cell as described above.
- After step (a), culturing the cell in the presence of the test compound for at least 1 minute, or at least 1 hour, or at least 24 hours; e.g. for a time period from 1 minute to 1 week, or from 10 minutes to 24 hours, or from 1 hour to 12 hours.
- Contacting the cell with a ligand that is capable of binding to the extracellular region of the polypeptide of the invention; the ligand may be added to the cells prior to the test compound, or simultaneously with the test compound; the ligand may be a ligand activating the aGPCR (or PC1/PC1-like protein) or a ligand inhibiting the aGPCR (or PC1/PC1-like protein). In one embodiment the polypeptide is constitutively active, and no ligand is added;
- Comparing the level determined in step (b) with a control level, e.g. with the level in the absence of the test compound. When a ligand of the polypeptide is present when contacting the test compound with the cell the control level is usually determined in the presence of the ligand and in the absence of the test compound. ; When no ligand of the polypeptide is present when contacting the test compound with the cell (e.g. if the polypeptide has constitutive activity) the control level is usually determined in the absence of a ligand of the polypeptide and in the absence of the test compound.
- Selecting the test compound if the level determined in step (b) is higher than the control level (when screening for activating test compounds); or selecting the test compound if the level determined in step (b) is lower than the control level (when screening for inhibiting compounds). Screening for inhibitors is preferred.

The above embodiments described for a cellular assay can be applied to methods using non-human transgenic animals *mutatis mutandis.*

The invention further pertains to the use of the polypeptide of the invention, of the nucleic acid of the invention, of the plasmid or vector of the invention, or of the cell of the invention, or of the transgenic animal of the invention for identifying a modulator, preferably an inhibitor, of an adhesion GPCR or of a PC1/PC1-like protein.

Modulators of an adhesion GPCR or of a PC1/PC1-like protein can be activators or inhibitors. Modulators further include agonists and antagonists. Preferably, the modulator is an inhibitor of an adhesion GPCR or of a PC1/PC1-like protein.

*Kits*

The present disclosure provides a kit for carrying out a method described herein.

In some cases, a subject kit comprises an expression vector comprising a nucleotide sequence encoding a polypeptide of the present invention.

In some cases, a subject kit comprises a chimeric polypeptide of the present invention.

In some cases, a subject kit comprises a cell that is genetically modified with a nucleic acid comprising a nucleotide sequence encoding a chimeric polypeptide of the present invention. In some cases, the kit comprises a cell that is genetically modified with a recombinant expression vector comprising a nucleotide sequence encoding the polypeptide of the present invention. Kit components can be in the same container, or in separate containers.

Any of the above-described kits can further include one or more additional reagents, where such additional reagents can be selected from: a dilution buffer; a reconstitution solution; a wash buffer; a control reagent; a control expression vector; a negative control polypeptide (e.g., a chimeric polypeptide that lacks the one or more proteolytic cleavage sites); a positive control polypeptide; a reagent for in vitro production of the chimeric polypeptide, and the like.

In addition to above-mentioned components, a subject kit can further include instructions for using the components of the kit to practice the subject methods. The instructions for practicing the subject methods are generally recorded on a suitable recording medium. For example, the instructions may be printed on a substrate, such as paper or plastic. As such, the instructions may be present in the kits as a package insert, in the labeling of the container of the kit or components thereof. In other embodiments, the instructions are present as an electronic file present on a suitable computer readable storage medium. In yet other embodiments, the actual instructions are not present in the kit, but means for obtaining the instructions from a remote source, e.g. via the internet, are provided. An example of this embodiment is a kit that includes a web address where the instructions can be viewed and/or from which the instructions can be downloaded. As with the instructions, this means for obtaining the instructions is recorded on a suitable substrate.

*Sequence listing*

Table 2. Overview of the sequences in the sequence listing.

| SEQ ID NO: | Brief description |
|---|---|
| 1 | GAIN domain of human ADGRA2 |
| 2 | GAIN domain of human ADGRA3 |
| 3 | GAIN domain of human ADGRB1 |
| 4 | GAIN domain of human ADGRB2 |
| 5 | GAIN domain of human ADGRB3 |
| 6 | GAIN domain of human ADGRC1 |
| 7 | GAIN domain of human ADGRC2 |
| 8 | GAIN domain of human ADGRC3 |
| 9 | GAIN domain of human ADGRD1 |
| 10 | GAIN domain of human ADGRD2 |
| 11 | GAIN domain of human ADGRE1 |
| 12 | GAIN domain of human ADGRE2 |
| 13 | GAIN domain of human ADGRE3 |
| 14 | GAIN domain of human ADGRE4P |
| 15 | GAIN domain of human ADGRE5 |
| 16 | GAIN domain of human ADGRF1 |
| 17 | GAIN domain of human ADGRF2 |
| 18 | GAIN domain of human ADGRF3 |
| 19 | GAIN domain of human ADGRF4 |
| 20 | GAIN domain of human ADGRF5 |
| 21 | GAIN domain of human ADGRG1 |
| 22 | GAIN domain of human ADGRG2 |
| 23 | GAIN domain of human ADGRG3 |
| 24 | GAIN domain of human ADGRG4 |
| 25 | GAIN domain of human ADGRG5 |
| 26 | GAIN domain of human ADGRG6 |
| 27 | GAIN domain of human ADGRG7 |
| 28 | GAIN domain of human ADGRL1 |
| 29 | GAIN domain of human ADGRL2 |
| 30 | GAIN domain of human ADGRL3 |
| 31 | GAIN domain of human ADGRL4 |
| 32 | GAIN domain of human ADGRV1 |
| 33 | GAIN domain of hPKD1 |
| 34 | GAIN domain of hPKD1L1 |
| 35 | GAIN domain of hPKD1L2 |
| 36 | GAIN domain of hPKD1L3 |
| 37 | GAIN domain of hPKDREJ |

(continued)

| SEQ ID NO: | Brief description |
|---|---|
| 38 | GPS/putative tethered agonist segment of C. elegans LAT-1/ADGRL |
| 39 | GPS/putative tethered agonist segment of rat ADGRL1 |
| 40 | GPS/putative tethered agonist segment of D. melanogaster CIRL/ADGRL |
| 41 | GPS/putative tethered agonist segment of human ADGRG5 |
| 42 | GPS/putative tethered agonist segment of human ADGRG1 |
| 43 | GPS/putative tethered agonist segment of human ADGRD1 |
| 44 | GPS/putative tethered agonist segment of human ADGRE5 |
| 45 | GPS/putative tethered agonist segment of human ADGRG6 |
| 46 | GPS/putative tethered agonist segment of D. melanogaster FMI/ADGRC |
| 47 | GPS/putative tethered agonist segment of human PC1 |
| 48 | GPS/putative tethered agonist segment of C. elegans LOV-1/PC1 |
| 49 | GPS/putative tethered agonist segment of human PC1L1 |
| 50 | GPS/putative tethered agonist segment of human PC1L2 |
| 51 | GPS/putative tethered agonist segment of human PC1L3 |
| 52 | Sequence comprising human Notch1 iuxta- and transmembrane segment |
| 53 | Sequence comprising human Notch2 iuxta- and transmembrane segment |
| 54 | Sequence comprising human Notch3 iuxta- and transmembrane segment |
| 55 | Sequence comprising human Notch4 iuxta- and transmembrane segment |
| 56 | Sequence comprising drosophila melanogaster Notch iuxta- and transmembrane segment |
| 57 | Drosophila melanogaster Notch iuxta- and transmembrane segment (Fig. 8) |
| 58 | mouse Notch1 iuxta- and transmembrane segment (Fig. 8) |
| 59 | Full-length NTF release sensor for dmCIRL |
| 60 | Full-length NTF release sensor for dmCIRL (GPS cleavage site mutated) |
| 61 | Full-length NTF release sensor for dmCIRL (S3 cleavage site mutated) |
| 62 | NTF release sensor positive control |
| 63 | NTF release sensor negative control |

**EXAMPLES**

**Operating principle of the NRS for adhesion GPCRs and PC1/PC1-like molecules**

We have harnessed the RIP principle employed in Notch receptor activation for the NRS. Notch is the key element in an evolutionarily highly conserved pathway that controls binary developmental decisions and plays essential roles in the determination of cell lineages (reviewed in ref. [24]). Briefly, Notch is activated through one of its ligands Delta or Serrate, which are transmembrane proteins mounted *in cis* and *in trans* to the receptor protein. Ligand endocytosis *in trans* transmits mechanical force onto the Notch ECR (extracellular region) causing the exposure of a iuxtamembrane receptor region[25,26], which during signal inactivity is shielded by the NRR (negative regulatory region). Consequently, the iuxtamembrane S2 site becomes accessible to the matrix metalloprotease Kuzbanian/ADAM (a disintegrin and metalloprotease domain), which cleaves the receptor protein outside the plasma membrane leading to shedding of a large part of the Notch ECR.

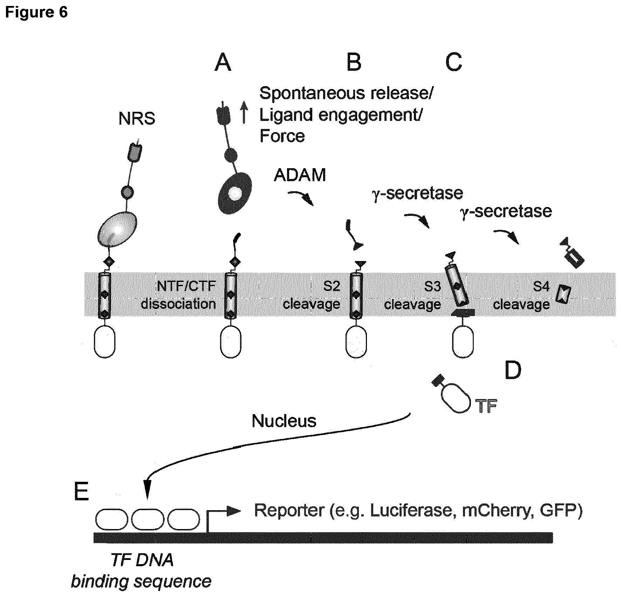

The working principle of the NRS (Figure 6) relies on (*i*) GAIN domain autoproteolysis or proteolysis of other domains in the ECR of aGPCR/PC1/PC1-like protein, and the coupling of (*ii*) NTF-CTF separation at the GPS and subsequent NTF release and exposure of its S2 site, (*iii*) further proteolytic processing of the Notch[ITS] component, to the (iv) intracellular release of a transcription factor moiety, which leads to the transcriptional activation of a genetic reporter element whose expression can be quantified by means of standard biochemical or optical assay systems.

NTF-CTF separation at the GPS and subsequent NTF release off NRS and S2 site exposure of NRS

In the naturally occuring setting for Notch signaling its S2 proteolysis site is protected by multiple EGF domain repeats, which mediate physiological ligand interactions, and the NNR (Notch-1 negative regulatory) region of the receptor's extracellular part of the receptor (reviewed in ref. [27]). EGF repeats and the NRR can be entirely replaced by heterologous domains while still protecting the signal-initiating S2 site from Kuz/ADAM engagement[28] (Figure 6). Accordingly, in case of the NRS activation at the S2 site is suppressed by aGPCR/PC1/PC1-like molecule extracellular regions.

NTF-CTF separation within the NRS is made possible through posttranslational GAIN domain autoproteolysis and subsequent covalent stabilisation of the ensuing NTF-CTF heterodimer or similar autoproteolytic events in other extracellular domains of aGPCRs/PC1/PC1-like proteins. NTF-CTF separation is induced spontaneously, through ligand binding, mechanical stimulation or a combination of the aforementioned processes (Figure 6A). As a result of NTF release, the remaining portion of the NRS consisting of the aGPCR/PC1/PC1-like[TA]-Notch[ITS]-TF chimeric protein is left in the plasma membrane with the S2 site of the Notch[ITS] exposed and accessible for subsequent proteolytic attack.

Proteolytic processing of iuxta- and transmembrane segment of NRS

S2 proteolytic processing of the NRS occurs through ADAM-like matrix metalloproteinases present in the test cell (Figure 6B). This allows for successive proteolytic steps of the remaining Notch[ITS] at S3 and S4 sites, which are located within the NRS' single transmembrane helix, through the $\gamma$-secretase complex (Figure 6C) in a process termed regulated intramembrane proteolysis (RIP) [29-31].

Intracellular release of transcription factor moiety

The Notch intracellular domain (NICD) was replaced with a heterologous transcription factor, (LexA::VP16 cassette), so that NRS activation upon NTF release results in the liberation of the TF from the intracellular face of the membrane (Figure 6D), which in turn and can be read out through the LexA/*lexAop* binary expression system[21] (Figure 6E). This biosynthetic replacement of the NICD has been similarly employed in other biosensors previously[30,32,33].

**Example 1: Demonstration of NRS functionality**

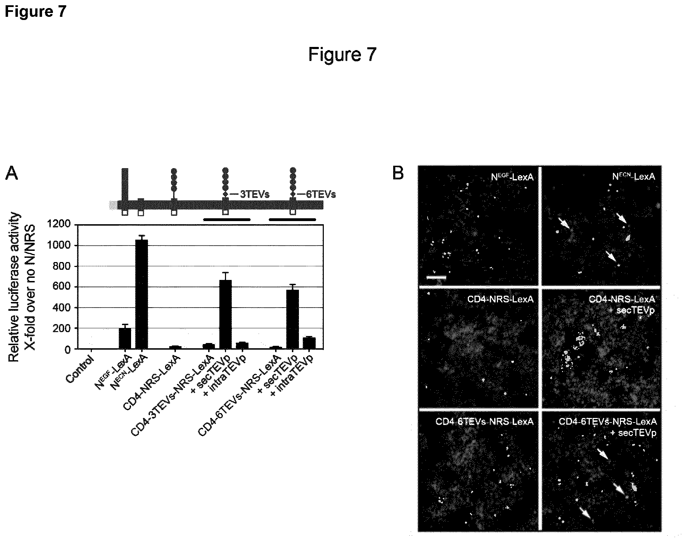

In order to conceptually establish the approach to fuse heterologous ECRs to the Notch[ITS] to suppress its proteolytic activation, a sensor protein containing 4 immunoglobulin (Ig) domains of the human CD4 receptor fused to the Notch[ITS] was engineered [28](Figure 7A). In a luciferase assay in *Drosophila* Schneider 2 (S2) cells addition of the CD4 domains to the NRS-LexA sensor component sufficiently quenched LexA release comparable to a constitutively inactive N[EGF]-LexA control as previously used in *Drosophila melanogaster*[34](Figure 7A).

A tri- or hexarepeat of the highly selective TEV (Tobacco etch virus) protease cleavage site (TEVs) was introduced between CD4 repeats and ITS (CD4-3TEVs-NRS-LexA, CD4-6TEVs-NRS-LexA) in order to conditionally sever artificial ECR and Notch[ITS] of the sensor and expose the extracellular S2 site by TEV protease expression. Upon co-expression of a secreted version of TEV (secTEVp), but not when TEV was expressed intracellularly (intraTEVp) or not at all, strong activation of both CD4-3/6TEVs-NRS-LexA reporters were observed in S2 cells by a commercially available firefly luciferase *lexAop-FLuc* reporter (Fig. 2A), and through expression of a *lexAop-mCherry* reporter (Figure 7A), co-transfected with the sensors in S2 cells, respectively (Figure 7B). As a positive control a constitutively active N[ECN]-LexA receptor[34] with a constantly exposed S2 cleavage site was included (Figure 7A,B).

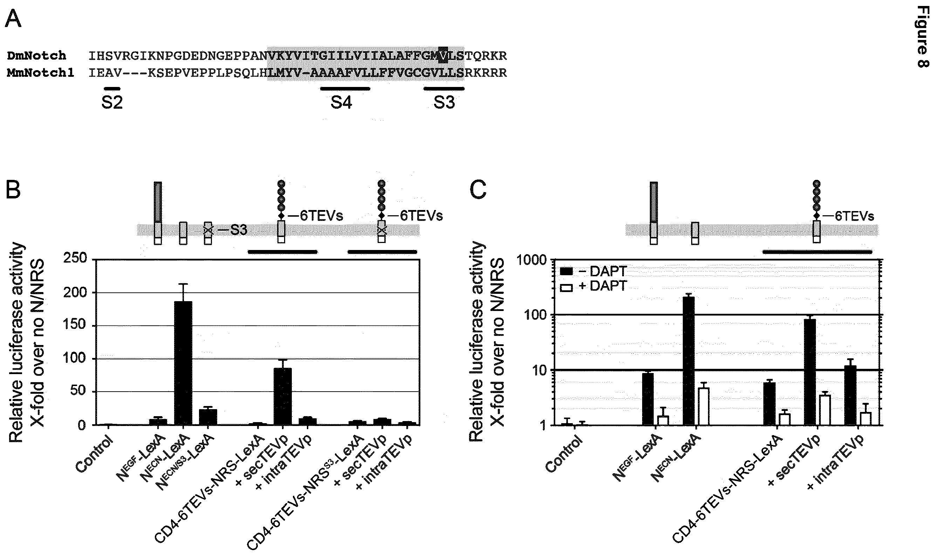

Replacement of a critical Valin residue in the S3 site of Notch[ITS] by Lysine[29] (Figure 8A,B), and pharmacological inhibition of $\gamma$-secretase activity by 10 $\mu$M DAPT abolished reporter activity (Figure 8C), demonstrating that NRS activation is relayed through the canonical Notch proteolytic cascade and that the readout of NRS activation can be pharmacologically modulated.

Next, the extracellular CD4 repeats of the sensor protein were replaced with the ECR of an aGPCR, the Latrophilin/ADGRL-homolog CIRL/CG8639 (calcium independent receptor of $\alpha$-latrotoxin) to enable the NRS to detect the physical dissociation of the NTF-CTF complex within the GAIN domain when appended to the Notch[ITS]. Thus, the entire ECR of CIRL (including the GAIN domain, GPS, tethered agonist segment and predicted linker to the 7TM domain) was N-terminally fused to the NRS core (CIRL-NRS-LexA; Figure 9). In luciferase assays we noticed that CIRL-NRS-LexA readily showed high activity suggesting that in this sensor layout either the S2 site was not sufficiently protected from the proteolytic attack of the metalloprotease by the CIRL-ECR, or that the NTF-CTF heterodimer spontaneously dissociated under the assay conditions rendering the S2 site exposed (Figure 9). In order to distinguish between these two possibilities, we mutated the GPS of the sensor at the -2 position of its catalytic triad and replaced the conserved Histidin by an Alanine (Figure 9) to block GAIN domain self-cleavage and eventual NTF release. Importantly, CIRL[H>A]-NRS-LexA displayed no reporter activity in luciferase assays similar to a CIRL-NRS[S3]-LexA sensor (disabled γ-secretase recognition) and mock transfected cells (Figure 9). This set of experiments establishes that the CIRL-NRS-LexA NRS can be used for monitoring the integrity of the NTF-CTF heterodimer clamped by the GAIN domain of CIRL.

**Example 2: Demonstration of NRS functionality in Drosophila melanogaster**

To test the utility of the NRS sensor principle *in vivo,* an existing genomic engineering platform[13,35] was used to encode NRS sensors in alleles of the adhesion GPCR *cirl/CG8639* of *Drosophila melanogaster.* This places CIRL-NRS expression under the endogenous regulatory control of the *cirl* locus (Figure 10A). The NRS design was adapted to generate minigenes for *cirl-NRS-LexA, cirl[H>A]-NRS-LexA* and *cirl-NRS[S3]-LexA* by fusing the genomic fragment encoding the CIRL-ECR to the NRS-LexA cDNA (Figure 10A,B). The resulting transgenes were inserted in the *cirl* gene using phiC31-assisted transformation generating fly stocks expressing the different sensors variants from the *cirl* locus.

Western blot analysis of fly head homogenates expressing CIRL-NRS-LexA and CIRL-NR[S3]-LexA confirmed that S3 proteolytic processing of CIRL-NRS-LexA is quenched by the mutation (Figure 10C). Further, protein levels of CIRL-NRS-LexA and CIRL[H>A]-NRS-LexA were indistinguishable, and GPS cleavage was entirely blocked by the GPS mutation resulting in a single full-length protein band running at the predicted size of 123 kDa (Figure 10C). Importantly, no further CIRL-NRS-LexA fragments derived from S2/S3/S4 cleavages were observed (Figure 10C). This demonstrates that CIRL-NRS-LexA activity is absolutely dependent on NTF release, and that the chain of molecular events allowing for the detection of NTF release through the NRS is unidirectional, and sequentially unfolds in the intended order: NTF/CTF disruption → S2 cleavage → S3 cleavage → LexA release (Figure 10B).

Next, the functionality of CIRL-NRS was further studied in anatomical expression analyses. Expression of *cirl-NRS-LexA* in thirs instar larvae showed that the CIRL-NRS-LexA is activated only in *Cirl+* neurons in the peripheral nervous system, the ventral nerve cord (VNC) and the brain hemispheres such as previously through a transcriptional *cirlp-Gal4* reporter[35] (data not shown). In adult animals *cirl-NRS-LexA* expression recapitulates endogenous *cirl* expression[35] with strong expression in the eye, proboscis and leg neurons (Figure 11A). Inhibition of S3 cleavage through the V→K mutation completely abrogated CIRL-NRS[S3]-LexA sensor activity apparent by lack of *lexAop-mCherry* expression at all major expression sites (eye, leg joints, proboscis; Figure 11B), indicating that intracellular LexA release is critically dependent on NRS processing through events that precede γ-secretase cleavage *in vivo* too.

Disabling GAIN domain proteolysis and thus potential NTF release of CIRL through replacement of a critical Histidin residue at position -2 to the GPS of the CIRL-NRS-LexA protein (CIRL[H>A]-NRS-LexA) abrogated NRS activation apparent through loss of the mCherry reporter induction in all organs and tissues (Figure 11C).

To control for potential misexpression inherent to the LexA transcriptional reporter, additional *cirl-NRS* transgenes alternatively terminating in *Gal4* or QF2 transcription factor cassettes were generated an analysed. A comparison of expression patterns controlled by *cirl-NRS-LexA* (Figure 12A), *cirl-NRS-Gal4* (Figure 12A) and *cirl-NRS-QF2* (Figure 12A) in adult animals using matching reporter transgenes displayed strong activation of the first two NRS variants (Figure 12A,B). *cirl-NRS-QF2* showed a weaker yet anatomically similar pattern (Figure 12C).

Amino acid sequences:

    SEQ ID NO:59. Full-length NTF release sensor for dmCIRL
    SEQ ID NO:60 Full-length NTF release sensor for dmCIRL (GPS cleavage site mutated)
    SEQ ID NO:61 Full-length NTF release sensor for dmCIRL (S3 cleavage site mutated)
    SEQ ID NO:62 NTF release sensor positive control
    SEQ ID NO:63 NTF release sensor negative control

**Materials and Methods**

S2 cell culture

For transfection, mini- or midi-prepped plasmid DNA (Qiagen) for each construct was adjusted to a stock concentration 100 ng/μl using a nanophotometer (Implen). For individual transfections a total amount of 200 ng DNA per 96-well plate well was used and always contained 30 ng of lexAop>FLuc2 (test construct reporter) and Act5.1Cp>RLuc (transfection control reporter), respectively. Test constructs (X-NRS-LexA) and TEV protease plasmids were added at 1:1 ratio (equimolarly adjusted to lexAop>FLuc2) to the DNA mixes; each DNA mix was supplemented with empty pBSK-SK+ vector (Stratagene) to 200 ng. S2 cells were cultured in Schneider's *Drosophila* medium (Invitrogen, Cat. no. 21720-024). Cultures were maintained in an air incubator at 25 °C. S2 cells were split and plated into individual wells of a 96-well plate at a concentration of $5 \times 10^5$/well on day 0. 24 hours after plating (day 1), cells were transfected with Lipofectamine 2000 (Invitrogen, #11668019) with the suitable 1:1 plasmid/reagent mixture according to manufacturer's instructions and incubated for 48 hours. When induction of the metallothionein-promoter was required, $CuSO_4$ stock medium was added in each well of the experiment to a final concentration of 0.5 mM $CuSO_4$ 24 hrs after transfection (day 2).

Luciferase assays

Cells were lysed on day 3 (48 hrs after transfection) and luciferase measurements with the Dual-Glo luciferase assay system (Promega) were performed according to the manufacturer's protocol. In brief, the supernatant was removed from each well by aspiration and cells were incubated with 200 μl passive lysis buffer on a shaker at room temperature for 30 min. Lysates were analyzed in 96-well plates with a Victor2 plate reader luminometer (PerkinElmer). Firefly and Renilla luciferase luminescence signals were collected for 10 s. Relative luciferase activities were were calculated for each sample individually as principally described in Potter et al. (ref. 23) according to the following formula:

$$RLA_x = (F_x/R_x)/(\overline{F/R})_{Empty} \, ,$$

where

$$(\overline{F/R})_{Empty} = \left(\sum_{i=1}^{n} F_{Empty}^i / R_{Empty}^i\right)/n \, ,$$

where n = number of control samples; F = Firefly luciferase luminescence signal; R = Renilla luciferase luminescence signal; Empty = lexAop>FLuc2 + Ac5.1p>RLuc + BSK-SK+. Each condition was executed at least in triplicate, the average and SEM were determined for each condition, and statistical significance was evaluated using Student's t test. Plasmids used for transfection in S2 cells were: N$^{ECN}$-LexA, N$^{EGF}$-LexA, ChiN-Test, pChiN1, pChiN2, pChiN3, lexAop-FLuc2, Ac5.1-RLuc, secTEVp, intraTEVp, pBSK-SK+.

Immunoblots

Fly heads were collected into 0.5 ml Eppi and immediately frozen in liquid nitrogen. Next, heads were mechanically crushed in 40μl SDS (2%, supplemented with protease Inhibitor Cocktail, Sigma-Aldrich; 1:1000) using a glas stirrer. Samples were icubated on ice for 10 min before the addition of 4 μl Triton-X100 (10%). Next, SDS-based sample buffer (Li-cor) was supplemeted with ß-Mercaptoethanol and was added to final dilution of 1X. Samples were centrifuged for 30min at 14000rpm (4 °C) and supernatant was collected. Centrifugation step was repeated, supernatant collected in fresh tube and subjected to electrophoresis on 6% or 4-12% Tris-Glycin SDS gel (Novex-Wedge-Well; Invitrogen) and blotted onto nitrocellulose membrane (0.2 μm pore size). The membrane was blocked for 1 hour (RT) using Odyssey Blocking buffer (Li-cor) diluted 1:2 with 1x PBS.

Blots were probed with primary antisera at the indicated concentrations overnight at 4°C: rabbit-α-HA (1:1000, RRID: AB_10693385), mouse-α-V5 (1:500; RRID:AB_2556564), mouse-α-tubulinβ(1:5000, RRID:AB_528499), rabbit-α-tubulina (1:500, RRID:AB_25541125). After rinsing twice and 3-10 min washing steps, membranes were incubated with IRDye 680RD goat-α-rabbit (RRID:AB_2721181) and goat-α-mouse (RRID:AB_2651128) as well as 800CW goat-α-mouse (1:15000; RRID:AB_2687825) and goat-α-rabbit (1:15000; RRID:AB_2651127) for 1 hour at RT, and again rinsed twice and washed 3-10 min. Blots were imaged with an OdysseyFc 2800 (Li-cor).

**References**

1. Rask-Andersen, M., Almen, M. S. & Schioth, H. B. Trends in the exploitation of novel drug targets. Nature reviews Drug discovery 10, 579-590 (2011).

2. O'Hayre, M. et al. The emerging mutational landscape of G proteins and G-protein-coupled receptors in cancer. Nat. Rev. Cancer 13, 412-424 (2013).

3. Promel, S., Langenhan, T. & Arac, D. Matching structure with function: the GAIN domain of adhesion-GPCR and PKD1-like proteins. Trends Pharmacol. Sci. 34, 470-478 (2013).

4. Langenhan, T., Aust, G. & Hamann, J. Sticky signaling--adhesion class G protein-coupled receptors take the stage. Science signaling 6, re3 (2013).

5. Scholz, N., Monk, K. R., Kittel, R. J. & Langenhan, T. Adhesion GPCRs as a Putative Class of Metabotropic Mechanosensors. Handb Exp Pharmacol 234, 221-247 (2016).

6. Purcell, R. H. & Hall, R. A. Adhesion G Protein-Coupled Receptors as Drug Targets. Annu. Rev. Pharmacal. Toxicol. 58,429-449 (2018).

7. Langenhan, T. Adhesion G protein-coupled receptors-Candidate metabotropic mechanosensors and novel drug targets. Basic Clin. Pharmacal. Toxicol. 547, 145 (2019).

8. Langenhan, T., Piao, X. & Monk, K. R. Adhesion G protein-coupled receptors in nervous system development and disease. Nat Rev Neurosci 17,550-561 (2016).

9. Arac, D. et al. A novel evolutionarily conserved domain of cell-adhesion GPCRs mediates autoproteolysis. EMBO J 31, 1364-1378 (2012).

10. Lin, H.-H. et al. Autocatalytic cleavage of the EMR2 receptor occurs at a conserved G protein-coupled receptor proteolytic site motif. J Biol Chem 279, 31823-31832 (2004).

11. Krasnoperov, V. G. et al. alpha-Latrotoxin stimulates exocytosis by the interaction with a neuronal G-protein-coupled receptor. Neuron 18, 925-937 (1997).

12. Gray, J. X. et al. CD97 is a processed, seven-transmembrane, heterodimeric receptor associated with inflammation. J Immunol 151, 5438-5447 (1996).

13. Scholz, N. et al. Mechano-dependent signaling by Latrophilin/CIRL quenches cAMP in proprioceptive neurons. elife 6, 1364 (2017).

14. Promel, S. et al. Characterization and functional study of a cluster of four highly conserved orphan adhesion-GPCR in mouse. Dev Dyn 241, 1591-1602 (2012).

15. Promel, S. et al. The GPS Motifls a Molecular Switch for Bimodal Activities of Adhesion Class G Protein-Coupled Receptors. Cell Reports 2, 321-331 (2012).

16. Nieberler, M., Kittel, R. J., Petrenko, A. G., Lin, H.-H. & Langenhan, T. Control of Adhesion GPCR Function Through Proteolytic Processing. Handb Exp Pharmacol 234, 83-109 (2016).

17. Paavola, K. J., Stephenson, J. R., Ritter, S. L., Alter, S. P. & Hall, R. A. The N terminus of the adhesion G protein-coupled receptor GPR56 controls receptor signaling activity. Journal of Biological Chemistry 286,28914-28921 (2011).

18. Liebscher, I. et al. A Tethered Agonist within the Ectodomain Activates the Adhesion G Protein-Coupled Receptors GPR126 and GPRI33. Cell Reports 9, 2018-2026 (2014).

19. Stoveken, H. M., Hajduczok, A. G., Xu, L. & Tall, G. G. Adhesion G protein-coupled receptors are activated by exposure of a cryptic tethered agonist. Proc Natl Acad Sci USA 112, 6194-6199 (2015).

20. Liebscher, I. & Schöneberg, T. Tethered Agonism: A Common Activation Mechanism of Adhesion GPCRs. Handb Exp Pharmacol 234, 111-125 (2016).

21. Lai, S.-L. & Lee, T. Genetic mosaic with dual binary transcription systems in Drosophila. Nat Neurosci 9, 703-709 (2006).

22. Brand, A. H. & Perrimon, N. Targeted gene expression as a means of altering cell fates and generating dominant phenotypes. Development 118,401-415 (1993).

23. Potter, C. J., Tasic, B., Russler, E. V., Liang, L. & Luo, L. The Q system: a repressible binary system for transgene expression, lineage tracing, and mosaic analysis. Cell 141, 536-548 (2010); Riabinina O, Luginbuhl D, Marr E, Liu S, Wu MN, Luo L, Potter CJ. Improved and expanded Q-system reagents for genetic manipulations". Nature Methods. 12 (3): 219-22, 5 p following 222 (2015).

24. Kopan, R. & Ilagan, M. X. G. The canonical Notch signaling pathway: unfolding the activation mechanism. Cell 137, 216-233 (2009).

25. Stephenson, N. L. & Avis, J. M. Direct observation of proteolytic cleavage at the S2 site upon forced unfolding of the Notch negative regulatory region. Proc Natl Acad Sci USA 109, E2757-65 (2012).

26. Meloty-Kapella, L., Shergill, B., Kuon, J., Botvinick, E. & Weinmaster, G. Notch ligand endocytosis generates mechanical pulling force dependent on dynamin, epsins, and actin. Dev Cell 22, 1299-1312 (2012).

27. Gordon, W. R., Arnett, K. L. & Blacklow, S. C. The molecular logic of Notch signaling: a structural and biochemical perspective. J Cell Sci 121, 3109-3119 (2008).

28. Mumm, J. S. et al. A ligand-induced extracellular cleavage regulates gamma-secretase-like proteolytic activation of Notch1. Mol Cell 5, 197-206 (2000).

29. Schroeter, E. H., Kisslinger, J. A. & Kopan, R. Notch-1 signalling requires ligand-induced proteolytic release of intracellular domain. Nature 393,382-386 (1998).

30. Struhl, G. & Adachi, A. Nuclear access and action of notch in vivo. Cell 93, 649-660 (1998).

31. Kopan, R., Schroeter, E. H., Weintraub, H. & Nye, J. S. Signal transduction by activatedmNotch: importance of proteolytic processing and its regulation by the extracellular domain. Proc Natl Acad Sci USA 93, 1683-1688 (1996).

32. Vooijs, M. et al. Mapping the consequence of Notch1 proteolysis in vivo with NIP-CRE. Development 134, 535-544 (2007).

33. Morsut, L. et al. Engineering Customized Cell Sensing and Response Behaviors Using Synthetic Notch Receptors. Cell 164,780-791 (2016).

34. Struhl, G. & Adachi, A. Requirements for presenilin-dependent cleavage of notch and other transmembrane proteins. Mol Cell 6, 625-636 (2000).

35. Scholz, N. et al. The Adhesion GPCR Latrophilin/CIRL Shapes Mechanosensation. Cell Reports 11, 866-874 (2015).

36. Gresko, N. et al. Polycystin 1 is an atypical adhesion GPCR that responds to non-canonical WNT signals and inhibits GSK3$\beta$. The FASEB Journal 33.1_supplement 863-10 (2019).

37. Harmar, A. J. Family-B G-protein-coupled receptors. Genome biology 2(12), reviews 3013-1 (2001).

38. Salzman GS. et al. Structural Basis for Regulation of GPR56/ADGRG1 by Its Alternatively Spliced Extracellular Domains. Neuron 91,1292-1304 (2016).

39. Leon K. et al. Structural basis for adhesion G protein-coupled receptor Gpr126 function. Nature Communications 11, 194 (2020).

**Claims**

1. A polypeptide comprising (i) a first sequence comprising a GPCR autoproteolysis-inducing (GAIN) domain of an adhesion GPCR or of a PC1/PC1-like protein (ii) a second sequence comprising the transmembrane region of a Notch receptor, and (iii) a third sequence comprising a transcription factor moiety.

2. The polypeptide of claim 1, wherein release of an N-terminal portion of the first sequence induces cleavage of the second sequence at the one or more proteolytic cleavage sites, thereby releasing the transcription factor moiety.

3. The polypeptide of claim 2, wherein said release is triggered by proteolysis at the GPCR proteolysis site (GPS) within the GAIN domain.

4. The polypeptide of any one of the preceding claims, wherein the first sequence comprises the N-terminal fragment of said adhesion GPCR or of said PC1/PC1-like protein.

5. The polypeptide of any one of the preceding claims, wherein the second sequence comprises the S2 cleavage site and the S3 cleavage site of the Notch receptor.

6. The polypeptide of claim 5, wherein the second sequence further comprises the S4 cleavage site of the Notch receptor.

7. The polypeptide of any one of the preceding claims, having the amino acid sequence as shown in SEQ ID NO:59, or an amino acid sequence having a sequence identity of at least 90% to SEQ ID NO:59.

8. The polypeptide of any one of the preceding claims, which is a sensor protein suitable for detecting the release of its N-terminal fragment and/or proteolytic cleavage within the GAIN domain.

9. A nucleic acid encoding the polypeptide of any one of the preceding claims.

10. A plasmid or vector comprising the nucleic acid of claim 9.

11. A cell comprising the polypeptide of any one of claims 1 to 8, the nucleic acid of claim 9, or the plasmid or vector of claim 10, optionally further comprising a nucleic acid capable of binding to the transcription factor moiety, operably linked to a nucleic acid encoding a reporter.

12. The cell of claim 11, wherein the transcription factor moiety, upon release from the second sequence, is capable of inducing expression of the reporter.

13. A non-human transgenic animal comprising the polypeptide of any one of claims 1 to 8, the nucleic acid of claim 9, the plasmid or vector of claim 10, or the cell of claim 11 or 12; or a non-human transgenic animal expressing the polypeptide of any one of claims 1 to 8.

14. A screening method comprising the following steps

    (a) contacting a test compound with the cell of claim 11 or 12, or administering the test compound to the transgenic animal of claim 13, and
    (b) determining the level of the reporter or of a detectable signal caused by the reporter.

15. The use of the polypeptide of any one of claims 1 to 8, of the nucleic acid of claim 9, of the plasmid or vector of claim 10, of the cell of claim 11 or 12, or of the transgenic animal of claim 13, for identifying a modulator of an adhesion GPCR or of a PC1/PC1-like protein.

**Figure 1**

**Figure 2**

**Figure 3**

**Figure 4**

Receptor structure
Unstimulated

Receptor structure
Stimulated

⟶

*VTF-CTF heterodimer
associated*

*NTF-CTF heterodimer
dissociated*

*NTF released*

OFF

ON

**Figure 5**

# NTF release sensor (NRS)

aGPCR/
PC1/
PC1-like
ECR

GPS

TA+linker

S2    Extra

Notch[ITS]

(S4)

S3

Intra

TF

**Figure 6**

**Figure 7**

Figure 7

**Figure 8**

**Figure 9**

**Figure 10**

**A**

**B**

GPS
— S2
— S4
— S3

★ — 3×V5 tag

**CIRL-NRS-LexA**
FL: 123 kDa
NTF: 76 kDa
GPS<>LexA: 47 kDa
S2<>LexA: 42 kDa
S3<>LexA: 38 kDa
S4<>LexA: 39 kDa

**C**

**Figure 11**

CIRL-NRS-LexA >
13xlexAop2-6xmCherry-HA

CIRL-NRS$^{S3}$-LexA >
13xlexAop2-6xmCherry-HA

CIRL$^{H>A}$-NRS-LexA >
13xlexAop2-6xmCherry-HA

**Figure 12**

CIRL-NRS-LexA >
13xlexAop2-6xmCherry-HA

CIRL-NRS-Gal4 >
20xUAS-6xmCherry-3xHA

CIRL-NRS-QF2 >
QUAS-mtdTomato-3xHA

**Figure 13**

EP 3 974 535 A1

# EUROPEAN SEARCH REPORT

**Application Number**

EP 20 19 8501

Europäisches Patentamt
European Patent Office
Office européen des brevets

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/099689 A1 (UNIV CALIFORNIA [US]; UNIV COLUMBIA [US]) 23 May 2019 (2019-05-23) | 1-3,8-14 | INV. C12N15/62 C07K14/705 C07K14/47 |
| A | * the whole document * | 4-7,15 | |
| A | AMANDA NICOLE HAYWARD: "Conformational Regulation of Cell Surface Receptor Proteolysis", THESIS UNIVERSITY OF MINNESOTA PH.D, IN PARTIAL FULFILLMENT OF THE REQUIREMENTS FOR THE DEGREE OF DOCTOR OF PHILOSOPHY, UNIVERSITY OF MINNESOTA, US , 1 February 2019 (2019-02-01), pages 1-191, XP009525751, Retrieved from the Internet: URL:https://conservancy.umn.edu/handle/11299/202420 * Chapter 2, p. 40, figure 2.1 Chapter 5, p. 128, last par. * | 1-15 | |
| A | VIZURRAGA ALEXANDER ET AL: "Mechanisms of adhesion G protein-coupled receptor activation", JOURNAL OF BIOLOGICAL CHEMISTRY, vol. 295, no. 41, 6 August 2020 (2020-08-06), pages 14065-14083, XP055777502, ISSN: 0021-9258, DOI: 10.1074/jbc.REV120.007423 Retrieved from the Internet: URL:http://dx.doi.org/10.1074/jbc.REV120.007423> * abstract * * page 14077, left-hand column, paragraph 3 * | 1-15 | TECHNICAL FIELDS SEARCHED (IPC) C07K |

The present search report has been drawn up for all claims

| Place of search | Date of completion of the search | Examiner |
|---|---|---|
| The Hague | 19 February 2021 | Wiame, Ilse |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

107

## ANNEX TO THE EUROPEAN SEARCH REPORT
## ON EUROPEAN PATENT APPLICATION NO.

EP 20 19 8501

This annex lists the patent family members relating to the patent documents cited in the above-mentioned European search report.
The members are as contained in the European Patent Office EDP file on
The European Patent Office is in no way liable for these particulars which are merely given for the purpose of information.

19-02-2021

| Patent document cited in search report | Publication date | Patent family member(s) | Publication date |
|---|---|---|---|
| WO 2019099689 A1 | 23-05-2019 | AU 2018370002 A1<br>CA 3082782 A1<br>EP 3710042 A1<br>US 2020331985 A1<br>WO 2019099689 A1 | 28-05-2020<br>23-05-2019<br>23-09-2020<br>22-10-2020<br>23-05-2019 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **TATUSOVA et al.** *FEMS Microbiol. Lett.,* 1999, vol. 174, 247-250
- **ARAC et al.** *EMBO J,* 2012, vol. 31, 1364-1378
- **SHANER et al.** *Nat. Methods,* 2005, vol. 2, 905-909
- **MATZ et al.** *Nature Biotechnol.,* 1999, vol. 17, 969-973
- **PINKERT.** Transgenic Animal Technology: A Laboratory Handbook
- **RASK-ANDERSEN, M. ; ALMEN, M. S. ; SCHIOTH, H. B.** Trends in the exploitation of novel drug targets. *Nature reviews Drug discovery,* 2011, vol. 10, 579-590
- **O'HAYRE, M. et al.** The emerging mutational landscape of G proteins and G-protein-coupled receptors in cancer. *Nat. Rev. Cancer,* 2013, vol. 13, 412-424
- **PROMEL, S. ; LANGENHAN, T. ; ARAC, D.** Matching structure with function: the GAIN domain of adhesion-GPCR and PKD1-like proteins. *Trends Pharmacol. Sci.,* 2013, vol. 34, 470-478
- **LANGENHAN, T. ; AUST, G. ; HAMANN, J.** Sticky signaling--adhesion class G protein-coupled receptors take the stage. *Science signaling,* 2013, vol. 6, re3
- **SCHOLZ, N. ; MONK, K. R. ; KITTEL, R. J. ; LANGENHAN, T.** Adhesion GPCRs as a Putative Class of Metabotropic Mechanosensors. *Handb Exp Pharmacol,* 2016, vol. 234, 221-247
- **PURCELL, R. H. ; HALL, R. A.** Adhesion G Protein-Coupled Receptors as Drug Targets. *Annu. Rev. Pharmacal. Toxicol.,* 2018, vol. 58, 429-449
- **LANGENHAN, T.** Adhesion G protein-coupled receptors-Candidate metabotropic mechanosensors and novel drug targets. *Basic Clin. Pharmacal. Toxicol.,* 2019, vol. 547, 145
- **LANGENHAN, T. ; PIAO, X. ; MONK, K. R.** Adhesion G protein-coupled receptors in nervous system development and disease. *Nat Rev Neurosci,* 2016, vol. 17, 550-561
- **ARAC, D. et al.** A novel evolutionarily conserved domain of cell-adhesion GPCRs mediates autoproteolysis. *EMBO J,* 2012, vol. 31, 1364-1378
- **LIN, H.-H. et al.** Autocatalytic cleavage of the EMR2 receptor occurs at a conserved G protein-coupled receptor proteolytic site motif. *J Biol Chem,* 2004, vol. 279, 31823-31832

- **KRASNOPEROV, V. G. et al.** alpha-Latrotoxin stimulates exocytosis by the interaction with a neuronal G-protein-coupled receptor. *Neuron,* 1997, vol. 18, 925-937
- **GRAY, J. X. et al.** CD97 is a processed, seven-transmembrane, heterodimeric receptor associated with inflammation. *J Immunol,* 1996, vol. 151, 5438-5447
- **SCHOLZ, N. et al.** Mechano-dependent signaling by Latrophilin/CIRL quenches cAMP in proprioceptive neurons. *elife,* 2017, vol. 6, 1364
- **PROMEL, S. et al.** Characterization and functional study of a cluster of four highly conserved orphan adhesion-GPCR in mouse. *Dev Dyn,* 2012, vol. 241, 1591-1602
- **PROMEL, S. et al.** The GPS Motifls a Molecular Switch for Bimodal Activities of Adhesion Class G Protein-Coupled Receptors. *Cell Reports,* 2012, vol. 2, 321-331
- **NIEBERLER, M. ; KITTEL, R. J. ; PETRENKO, A. G. ; LIN, H.-H. ; LANGENHAN, T.** Control of Adhesion GPCR Function Through Proteolytic Processing. *Handb Exp Pharmacol,* 2016, vol. 234, 83-109
- **PAAVOLA, K. J. ; STEPHENSON, J. R. ; RITTER, S. L. ; ALTER, S. P. ; HALL, R. A.** The N terminus of the adhesion G protein-coupled receptor GPR56 controls receptor signaling activity. *Journal of Biological Chemistry,* 2011, vol. 286, 28914-28921
- **LIEBSCHER, I. et al.** A Tethered Agonist within the Ectodomain Activates the Adhesion G Protein-Coupled Receptors GPR126 and GPRI33. *Cell Reports,* 2014, vol. 9, 2018-2026
- **STOVEKEN, H. M. ; HAJDUCZOK, A. G. ; XU, L. ; TALL, G. G.** Adhesion G protein-coupled receptors are activated by exposure of a cryptic tethered agonist. *Proc Natl Acad Sci USA,* 2015, vol. 112, 6194-6199
- **LIEBSCHER, I. ; SCHÖNEBERG, T.** Tethered Agonism: A Common Activation Mechanism of Adhesion GPCRs. *Handb Exp Pharmacol,* 2016, vol. 234, 111-125
- **LAI, S.-L. ; LEE, T.** Genetic mosaic with dual binary transcriptional systems in Drosophila. *Nat Neurosci,* 2006, vol. 9, 703-709
- **BRAND, A. H. ; PERRIMON, N.** Targeted gene expression as a means of altering cell fates and generating dominant phenotypes. *Development,* 1993, vol. 118, 401-415

- **POTTER, C. J. ; TASIC, B. ; RUSSLER, E. V. ; LIANG, L. ; LUO, L.** The Q system: a repressible binary system for transgene expression, lineage tracing, and mosaic analysis. *Cell,* 2010, vol. 141, 536-548
- **RIABININA O ; LUGINBUHL D ; MARR E ; LIU S ; WU MN ; LUO L ; POTTER CJ.** Improved and expanded Q-system reagents for genetic manipulations. *Nature Methods,* 2015, vol. 12 (3), 219-22, 5
- **KOPAN, R. ; ILAGAN, M. X. G.** The canonical Notch signaling pathway: unfolding the activation mechanism. *Cell,* 2009, vol. 137, 216-233
- **STEPHENSON, N. L. ; AVIS, J. M.** Direct observation of proteolytic cleavage at the S2 site upon forced unfolding of the Notch negative regulatory region. *Proc Natl Acad Sci USA,* 2012, vol. 109, E2757-65
- **MELOTY-KAPELLA, L. ; SHERGILL, B. ; KUON, J. ; BOTVINICK, E. ; WEINMASTER, G.** Notch ligand endocytosis generates mechanical pulling force dependent on dynamin, epsins, and actin. *Dev Cell,* 2012, vol. 22, 1299-1312
- **GORDON, W. R. ; ARNETT, K. L. ; BLACKLOW, S. C.** The molecular logic of Notch signaling: a structural and biochemical perspective. *J Cell Sci,* 2008, vol. 121, 3109-3119
- **MUMM, J. S. et al.** A ligand-induced extracellular cleavage regulates gamma-secretase-like proteolytic activation of Notch1. *Mol Cell,* 2000, vol. 5, 197-206
- **SCHROETER, E. H. ; KISSLINGER, J. A. ; KOPAN, R.** Notch-1 signalling requires ligand-induced proteolytic release of intracellular domain. *Nature,* 1998, vol. 393, 382-386
- **STRUHL, G. ; ADACHI, A.** Nuclear access and action of notch in vivo. *Cell,* 1998, vol. 93, 649-660
- **KOPAN, R. ; SCHROETER, E. H. ; WEINTRAUB, H. ; NYE, J. S.** Signal transduction by activatedm-Notch: importance of proteolytic processing and its regulation by the extracellular domain. *Proc Natl Acad Sci USA,* 1996, vol. 93, 1683-1688
- **VOOIJS, M. et al.** Mapping the consequence of Notch1 proteolysis in vivo with NIP-CRE. *Development,* 2007, vol. 134, 535-544
- **MORSUT, L. et al.** Engineering Customized Cell Sensing and Response Behaviors Using Synthetic Notch Receptors. *Cell,* 2016, vol. 164, 780-791
- **STRUHL, G. ; ADACHI, A.** Requirements for presenilin-dependent cleavage of notch and other transmembrane proteins. *Mol Cell,* 2000, vol. 6, 625-636
- **SCHOLZ, N. et al.** The Adhesion GPCR Latrophilin/CIRL Shapes Mechanosensation. *Cell Reports,* 2015, vol. 11, 866-874
- **GRESKO, N. et al.** Polycystin 1 is an atypical adhesion GPCR that responds to non-canonical WNT signals and inhibits GSK3β. *The FASEB Journal,* 2019, vol. 33.1
- **HARMAR, A. J.** Family-B G-protein-coupled receptors. *Genome biology,* 2001, vol. 2 (12), 3013-1
- **SALZMAN GS et al.** Structural Basis for Regulation of GPR56/ADGRG1 by Its Alternatively Spliced Extracellular Domains. *Neuron,* 2016, vol. 91, 1292-1304
- **LEON K. et al.** Structural basis for adhesion G protein-coupled receptor Gpr126 function. *Nature Communications,* 2020, vol. 11, 194